# EUROPEAN PATENT APPLICATION

(11) **EP 3 909 986 A1**
(43) Date of publication of application: **17.11.2021**
(21) Application number: 20738951.1
(22) Date of filing: 09.01.2020
(51) Int. Cl.: C07K 19/00, C12N 15/62, C12N 15/63, C12P 21/02, A61K 39/395, A61K 51/10, A61P 35/00

(54) **MULTI-TARGET FUSION PROTEIN USED FOR BLOCKING VASCULAR ENDOTHELIAL GROWTH AND ACTIVATING T CELLS, AND PHARMACEUTICAL COMPOSITION COMPRISING SAME**

(30) Priority: 10.01.2019 CN 201910023522
(71) Applicant: Beijing Beyond Biotechnology Co., Ltd., Beijing 100176 (CN); Lunan Pharmaceutical Group Corporation, Linyi, Shandong 276006 (CN)
(72) Inventor: HU, Pinliang, Beijing 100176 (CN); ZOU, Jing, Beijing 100176 (CN); HONG, Weidong, Beijing 100176 (CN); HE, Yun, Beijing 100176 (CN); BAI, Jie, Beijing 100176 (CN); SONG, Lingyun, Beijing 100176 (CN); YANG, Wendi, Beijing 100176 (CN); ZHANG, Guimin, Linyi, Shandong 276006 (CN); ZHAO, Lili, Linyi, Shandong 276006 (CN); LIU, Zhong, Linyi, Shandong 276006 (CN); LI, Zhenyu, Linyi, Shandong 276006 (CN); ZHU, Zhongsong, Linyi, Shandong 276006 (CN)
(74) Representative: Carridge, Andrew Edward
(86) International application number: PCT/CN2020/071213
(87) International publication number: WO 2020/143720

(57) **Abstract**

The present invention provides a multi-targeting fusion protein that blocks the growth of vascular endothelial cells and activates T cells, which comprises (i) a vascular endothelial cell growth inhibitory domain; (ii) an immunoglobulin Fc domain; and (iii) CD80 extracellular domain (ECD). The present invention also provides a polynucleotide encoding the multi-targeting fusion protein, a vector containing the polynucleotide, a host cell comprising the polynucleotide or the vector, and pharmaceutical composition comprising the multi-targeting fusion protein and an anti-PD-1 antibody. The multi-targeting fusion protein and pharmaceutical composition of the present invention can treat or prevent cancerous diseases in individuals.

Sequence Listing

<110> Beijing Beyond Biotechnology Co.,Ltd., Lunan Pharmaceutical Group
Corporation
<120> MULTI-TARGETING FUSION PROTEIN FOR BLOCKING THE GROWTH OF VASCULAR
ENDOTHELIAL CELLS AND ACTIVATING T CELLS AND PHARMACEUTICAL COMPOSITION
COMPRISING THE SAME

<130> P/83185.EP01

<140> EP20738951.1
<241> 2020-01-09

<160> 88
<170> PatentIn version 3.3
<210> 1
<211> 116
<212> PRT
<213> Artificial sequence
<220>
<223> An heavy chain variable region of an anti-VEGF antibody or anti-VEGFR
antibody in fusion protein
<400> 1
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Thr Phe Thr Asn Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro His Tyr Tyr Gly Ser Ser His Trp Tyr Phe Asp Val
            100                 105                 110
Trp Gly Gln Gly
        115
<210> 2
<211> 101
<212> PRT
<213> Artificial sequence
<220>
<223> An light chain variable region of an anti-VEGF antibody or anti-VEGFR
antibody in fusion protein
<400> 2
Asp Ile Gln Met Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly
            100
<210> 3
<211> 116

<212>  PRT
<213>  Artificial sequence
<220>
<223>  An heavy chain variable region of an anti-VEGF antibody or anti-VEGFR
antibody in fusion protein
<400>  3
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
        35                  40                  45
Gly Trp Ile Asn Thr Tyr Thr Gly Glu Pro Thr Tyr Ala Ala Asp Phe
    50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly
        115
<210>  4
<211>  101
<212>  PRT
<213>  Artificial sequence
<220>
<223>  An light chain variable region of an anti-VEGF antibody or anti-VEGFR
antibody in fusion protein
<400>  4
Asp Ile Gln Leu Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
            20                  25                  30
Leu Asn Trp Tyr Gln Gln Lys Pro Gly Lys Ala Pro Lys Val Leu Ile
        35                  40                  45
Tyr Phe Thr Ser Ser Leu His Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
                85                  90                  95
Thr Phe Gly Gln Gly
            100
<210>  5
<211>  109
<212>  PRT
<213>  Artificial sequence
<220>
<223>  An heavy chain variable region of an anti-VEGF antibody or anti-VEGFR
antibody in fusion protein
<400>  5
Glu Val Gln Leu Val Gln Ser Gly Gly Gly Leu Val Lys Pro Gly Gly
1               5                   10                  15
Ser Leu Arg Leu Ser Cys Ala Ala Ser Gly Phe Thr Phe Ser Ser Tyr
            20                  25                  30
Ser Met Asn Trp Val Arg Gln Ala Pro Gly Lys Gly Leu Glu Trp Val
        35                  40                  45
Ser Ser Ile Ser Ser Ser Ser Ser Tyr Ile Tyr Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys

85                  90                  95
Ala Arg Val Thr Asp Ala Phe Asp Ile Trp Gly Gln Gly
            100                 105
<210>  6
<211>  101
<212>  PRT
<213>  Artificial sequence
<220>
<223>  An light chain variable region of an anti-VEGF antibody or anti-VEGFR
antibody in fusion protein
<400>  6
Asp Ile Gln Met Thr Gln Ser Pro Ser Ser Val Ser Ala Ser Ile Gly
1               5                   10                  15
Asp Arg Val Thr Ile Thr Cys Arg Ala Ser Gln Gly Ile Asp Asn Trp
            20                  25                  30
Leu Gly Trp Tyr Gln Gln Lys Pro Gly Lys Ala Pro Lys Leu Leu Ile
        35                  40                  45
Tyr Asp Ala Ser Asn Leu Asp Thr Gly Val Pro Ser Arg Phe Ser Gly
    50                  55                  60
Ser Gly Ser Gly Thr Tyr Phe Thr Leu Thr Ile Ser Ser Leu Gln Ala
65                  70                  75                  80
Glu Asp Phe Ala Val Tyr Phe Cys Gln Gln Ala Lys Ala Phe Pro Pro
                85                  90                  95
Thr Phe Gly Gly Gly
            100
<210>  7
<211>  205
<212>  PRT
<213>  Artificial sequence
<220>
<223>  VEGFR1-D2/VEGFR2-D3
<400>  7
Ser Asp Thr Gly Arg Pro Phe Val Glu Met Tyr Ser Glu Ile Pro Glu
1               5                   10                  15
Ile Ile His Met Thr Glu Gly Arg Glu Leu Val Ile Pro Cys Arg Val
            20                  25                  30
Thr Ser Pro Asn Ile Thr Val Thr Leu Lys Lys Phe Pro Leu Asp Thr
        35                  40                  45
Leu Ile Pro Asp Gly Lys Arg Ile Ile Trp Asp Ser Arg Lys Gly Phe
    50                  55                  60
Ile Ile Ser Asn Ala Thr Tyr Lys Glu Ile Gly Leu Leu Thr Cys Glu
65                  70                  75                  80
Ala Thr Val Asn Gly His Leu Tyr Lys Thr Asn Tyr Leu Thr His Arg
                85                  90                  95
Gln Thr Asn Thr Ile Ile Asp Val Val Leu Ser Pro Ser His Gly Ile
            100                 105                 110
Glu Leu Ser Val Gly Glu Lys Leu Val Leu Asn Cys Thr Ala Arg Thr
        115                 120                 125
Glu Leu Asn Val Gly Ile Asp Phe Asn Trp Glu Tyr Pro Ser Ser Lys
    130                 135                 140
His Gln His Lys Lys Leu Val Asn Arg Asp Leu Lys Thr Gln Ser Gly
145                 150                 155                 160
Ser Glu Met Lys Lys Phe Leu Ser Thr Leu Thr Ile Asp Gly Val Thr
                165                 170                 175
Arg Ser Asp Gln Gly Leu Tyr Thr Cys Ala Ala Ser Ser Gly Leu Met
            180                 185                 190
Thr Lys Lys Asn Ser Thr Phe Val Arg Val His Glu Lys
        195                 200                 205
<210>  8
<211>  303
<212>  PRT
<213>  Artificial sequence
<220>

<223>  VEGFR1-D2/VEGFR2-D3-D4
<400>  8
Ser Asp Thr Gly Arg Pro Phe Val Glu Met Tyr Ser Glu Ile Pro Glu
1               5                   10                  15
Ile Ile His Met Thr Glu Gly Arg Glu Leu Val Ile Pro Cys Arg Val
            20                  25                  30
Thr Ser Pro Asn Ile Thr Val Thr Leu Lys Lys Phe Pro Leu Asp Thr
        35                  40                  45
Leu Ile Pro Asp Gly Lys Arg Ile Ile Trp Asp Ser Arg Lys Gly Phe
    50                  55                  60
Ile Ile Ser Asn Ala Thr Tyr Lys Glu Ile Gly Leu Leu Thr Cys Glu
65                  70                  75                  80
Ala Thr Val Asn Gly His Leu Tyr Lys Thr Asn Tyr Leu Thr His Arg
                85                  90                  95

Gln Thr Asn Thr Ile Ile Asp Val Val Leu Ser Pro Ser His Gly Ile
            100                 105                 110
Glu Leu Ser Val Gly Glu Lys Leu Val Leu Asn Cys Thr Ala Arg Thr
        115                 120                 125
Glu Leu Asn Val Gly Ile Asp Phe Asn Trp Glu Tyr Pro Ser Ser Lys
    130                 135                 140
His Gln His Lys Lys Leu Val Asn Arg Asp Leu Lys Thr Gln Ser Gly
145                 150                 155                 160
Ser Glu Met Lys Lys Phe Leu Ser Thr Leu Thr Ile Asp Gly Val Thr
                165                 170                 175
Arg Ser Asp Gln Gly Leu Tyr Thr Cys Ala Ala Ser Ser Gly Leu Met
            180                 185                 190
Thr Lys Lys Asn Ser Thr Phe Val Arg Val His Glu Lys Pro Phe Val
        195                 200                 205
Ala Phe Gly Ser Gly Met Glu Ser Leu Val Glu Ala Thr Val Gly Glu
    210                 215                 220
Arg Val Arg Ile Pro Ala Lys Tyr Leu Gly Tyr Pro Pro Pro Glu Ile
225                 230                 235                 240
Lys Trp Tyr Lys Asn Gly Ile Pro Leu Glu Ser Asn His Thr Ile Lys
                245                 250                 255
Ala Gly His Val Leu Thr Ile Met Glu Val Ser Glu Arg Asp Thr Gly
            260                 265                 270
Asn Tyr Thr Val Ile Leu Thr Asn Pro Ile Ser Lys Glu Lys Gln Ser
        275                 280                 285
His Val Val Ser Leu Val Val Tyr Val Pro Pro Gly Pro Gly Asp
    290                 295                 300
<210>  9
<211>  100
<212>  PRT
<213>  Artificial sequence
<220>
<223>  VEGFR1-D2
<400>  9
Ser Asp Thr Gly Arg Pro Phe Val Glu Met Tyr Ser Glu Ile Pro Glu
1               5                   10                  15
Ile Ile His Met Thr Glu Gly Arg Glu Leu Val Ile Pro Cys Arg Val
            20                  25                  30
Thr Ser Pro Asn Ile Thr Val Thr Leu Lys Lys Phe Pro Leu Asp Thr
        35                  40                  45
Leu Ile Pro Asp Gly Lys Arg Ile Ile Trp Asp Ser Arg Lys Gly Phe
    50                  55                  60
Ile Ile Ser Asn Ala Thr Tyr Lys Glu Ile Gly Leu Leu Thr Cys Glu
65                  70                  75                  80
Ala Thr Val Asn Gly His Leu Tyr Lys Thr Asn Tyr Leu Thr His Arg
                85                  90                  95
Gln Thr Asn Thr
            100
<210>  10

<211>  227
<212>  PRT
<213>  Artificial sequence
<220>
<223>  An amino acid sequence of IgG1 Fc in the fusion protein
<400>  10
Asp Lys Thr His Thr Cys Pro Pro Cys Pro Ala Pro Glu Leu Leu Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Asn Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Tyr Thr Leu Pro Pro Ser Arg Glu Glu Met Thr Lys Asn Gln Val Ser
    130                 135                 140
Leu Thr Cys Leu Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Tyr Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly Lys
225
<210>  11
<211>  232
<212>  PRT
<213>  Artificial sequence
<220>
<223>  An amino acid sequence of IgG2 Fc in fusion protein
<400>  11
Asp Lys Thr Val Glu Arg Lys Cys Cys Val Glu Cys Pro Pro Cys Pro
1               5                   10                  15
Ala Pro Pro Val Ala Gly Pro Ser Val Phe Leu Phe Pro Pro Lys Pro
            20                  25                  30
Lys Asp Thr Leu Met Ile Ser Arg Thr Pro Glu Val Thr Cys Val Val
        35                  40                  45
Val Asp Val Ser His Glu Asp Pro Glu Val Gln Phe Asn Trp Tyr Val
    50                  55                  60
Asp Gly Val Glu Val His Asn Ala Lys Thr Lys Pro Arg Glu Glu Gln
65                  70                  75                  80
Phe Asn Ser Thr Phe Arg Val Val Ser Val Leu Thr Val Val His Gln
                85                  90                  95
Asp Trp Leu Asn Gly Lys Glu Tyr Lys Cys Lys Val Ser Asn Lys Gly
            100                 105                 110
Leu Pro Ala Pro Ile Glu Lys Thr Ile Ser Lys Thr Lys Gly Gln Pro
        115                 120                 125
Arg Glu Pro Gln Val Tyr Thr Leu Pro Pro Ser Arg Glu Glu Met Thr
    130                 135                 140
Lys Asn Gln Val Ser Leu Thr Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175
Lys Thr Thr Pro Pro Met Leu Asp Ser Asp Gly Ser Phe Phe Leu Tyr
            180                 185                 190
Ser Lys Leu Thr Val Asp Lys Ser Arg Trp Gln Gln Gly Asn Val Phe
        195                 200                 205
Ser Cys Ser Val Met His Glu Ala Leu His Asn His Tyr Thr Gln Lys
    210                 215                 220
Ser Leu Ser Leu Ser Pro Gly Lys
225                 230
<210>  12
<211>  233
<212>  PRT
<213>  Artificial sequence
<220>
<223>   An amino acid sequence of IgG4 Fc in the fusion protein
<400>  12
Asp Lys Arg Val Glu Ser Lys Tyr Gly Pro Pro Cys Pro Pro Cys Pro
1               5                   10                  15
Ala Pro Glu Phe Leu Gly Gly Pro Ser Val Phe Leu Phe Pro Pro Lys
            20                  25                  30
Pro Lys Asp Thr Leu Met Ile Ser Arg Thr Pro Glu Val Thr Cys Val
        35                  40                  45
Val Val Asp Val Ser Gln Glu Asp Pro Glu Val Gln Phe Asn Trp Tyr
    50                  55                  60
Val Asp Gly Val Glu Val His Asn Ala Lys Thr Lys Pro Arg Glu Glu
65                  70                  75                  80
Gln Phe Asn Ser Thr Tyr Arg Val Val Ser Val Leu Thr Val Leu His
                85                  90                  95
Gln Asp Trp Leu Asn Gly Lys Glu Tyr Lys Cys Lys Val Ser Asn Lys
            100                 105                 110
Gly Leu Pro Ser Ser Ile Glu Lys Thr Ile Ser Lys Ala Lys Gly Gln
        115                 120                 125
Pro Arg Glu Pro Gln Val Tyr Thr Leu Pro Pro Ser Gln Glu Glu Met
    130                 135                 140
Thr Lys Asn Gln Val Ser Leu Thr Cys Leu Val Lys Gly Phe Tyr Pro
145                 150                 155                 160
Ser Asp Ile Ala Val Glu Trp Glu Ser Asn Gly Gln Pro Glu Asn Asn
                165                 170                 175
Tyr Lys Thr Thr Pro Pro Val Leu Asp Ser Asp Gly Ser Phe Phe Leu
            180                 185                 190
Tyr Ser Arg Leu Thr Val Asp Lys Ser Arg Trp Gln Glu Gly Asn Val
        195                 200                 205
Phe Ser Cys Ser Val Met His Glu Ala Leu His Asn His Tyr Thr Gln
    210                 215                 220
Lys Ser Leu Ser Leu Ser Leu Gly Lys
225                 230
<210>  13
<211>  112
<212>  PRT
<213>  Artificial sequence
<220>
<223>  CD80-IgV
<400>  13
Val Ile His Val Thr Lys Glu Val Lys Glu Val Ala Thr Leu Ser Cys
1               5                   10                  15
Gly His Asn Val Ser Val Glu Glu Leu Ala Gln Thr Arg Ile Tyr Trp
            20                  25                  30
Gln Lys Glu Lys Lys Met Val Leu Thr Met Met Ser Gly Asp Met Asn
        35                  40                  45
Ile Trp Pro Glu Tyr Lys Asn Arg Thr Ile Phe Asp Ile Thr Asn Asn
    50                  55                  60
Leu Ser Ile Val Ile Leu Ala Leu Arg Pro Ser Asp Glu Gly Thr Tyr

65                  70                  75                  80
Glu Cys Val Val Leu Lys Tyr Glu Lys Asp Ala Phe Lys Arg Glu His
                85                  90                  95
Leu Ala Glu Val Thr Leu Ser Val Lys Ala Asp Phe Pro Thr Pro Ser
            100                 105                 110
<210>  14
<211>  208
<212>  PRT
<213>  Artificial sequence
<220>
<223>  CD80-IgVIgC
<400>  14
<210>  15
<211>  113
<212>  PRT
<213>  Artificial sequence
<220>
<223>  A light chain constant region sequence of an anti-VEGF antibody or
anti-VEGFR antibody in the fusion protein, type ??
<400>  15
Thr Lys Val Glu Ile Lys Arg Thr Val Ala Ala Pro Ser Val Phe Ile
1               5                   10                  15
Phe Pro Pro Ser Asp Glu Gln Leu Lys Ser Gly Thr Ala Ser Val Val
            20                  25                  30
Cys Leu Leu Asn Asn Phe Tyr Pro Arg Glu Ala Lys Val Gln Trp Lys
        35                  40                  45
Val Asp Asn Ala Leu Gln Ser Gly Asn Ser Gln Glu Ser Val Thr Glu
    50                  55                  60
Gln Asp Ser Lys Asp Ser Thr Tyr Ser Leu Ser Ser Thr Leu Thr Leu
65                  70                  75                  80
Ser Lys Ala Asp Tyr Glu Lys His Lys Val Tyr Ala Cys Glu Val Thr
                85                  90                  95
His Gln Gly Leu Ser Ser Pro Val Thr Lys Ser Phe Asn Arg Gly Glu
            100                 105                 110
Cys

<210>  16

<211>  112
<212>  PRT
<213>  Artificial sequence
<220>
<223> A light chain constant region sequence of an anti-VEGF antibody or
anti-VEGFR antibody in the fusion protein, ?? type
<400>  16
Thr Lys Leu Thr Val Leu Gly Gln Pro Lys Ala Ala Pro Ser Val Thr
1               5                   10                  15
Leu Phe Pro Pro Ser Ser Glu Glu Leu Gln Ala Asn Lys Ala Thr Leu
            20                  25                  30
Val Cys Leu Ile Ser Asp Phe Tyr Pro Gly Ala Val Thr Val Ala Trp
        35                  40                  45
Lys Ala Asp Ser Ser Pro Val Lys Ala Gly Val Glu Thr Thr Thr Pro
    50                  55                  60
Ser Lys Gln Ser Asn Asn Lys Tyr Ala Ala Ser Ser Tyr Leu Ser Leu
65                  70                  75                  80
Thr Pro Glu Gln Trp Lys Ser His Arg Ser Tyr Ser Cys Gln Val Thr
                85                  90                  95
His Glu Gly Ser Thr Val Glu Lys Thr Val Ala Pro Thr Glu Cys Ser
            100                 105                 110
<210>  17
<211>  337
<212>  PRT
<213>  Artificial sequence
<220>
<223>  A heavy chain constant region sequence of an antibody in the fusion
protein, IgG1 type
<400>  17
Thr Leu Val Thr Val Ser Ser Ala Ser Thr Lys Gly Pro Ser Val Phe
1               5                   10                  15
Pro Leu Ala Pro Ser Ser Lys Ser Thr Ser Gly Gly Thr Ala Ala Leu
            20                  25                  30
Gly Cys Leu Val Lys Asp Tyr Phe Pro Glu Pro Val Thr Val Ser Trp
        35                  40                  45
Asn Ser Gly Ala Leu Thr Ser Gly Val His Thr Phe Pro Ala Val Leu
    50                  55                  60
Gln Ser Ser Gly Leu Tyr Ser Leu Ser Ser Val Val Thr Val Pro Ser
65                  70                  75                  80
Ser Ser Leu Gly Thr Gln Thr Tyr Ile Cys Asn Val Asn His Lys Pro
                85                  90                  95
Ser Asn Thr Lys Val Asp Lys Lys Val Glu Pro Lys Ser Cys Asp Lys
            100                 105                 110
Thr His Thr Cys Pro Pro Cys Pro Ala Pro Glu Leu Leu Gly Gly Pro
        115                 120                 125
Ser Val Phe Leu Phe Pro Pro Lys Pro Lys Asp Thr Leu Met Ile Ser
    130                 135                 140
Arg Thr Pro Glu Val Thr Cys Val Val Val Asp Val Ser His Glu Asp
145                 150                 155                 160
Pro Glu Val Lys Phe Asn Trp Tyr Val Asp Gly Val Glu Val His Asn
                165                 170                 175
Ala Lys Thr Lys Pro Arg Glu Glu Gln Tyr Asn Ser Thr Tyr Arg Val
            180                 185                 190
Val Ser Val Leu Thr Val Leu His Gln Asp Trp Leu Asn Gly Lys Glu
        195                 200                 205
Tyr Lys Cys Lys Val Ser Asn Lys Ala Leu Pro Ala Pro Ile Glu Lys
    210                 215                 220
Thr Ile Ser Lys Ala Lys Gly Gln Pro Arg Glu Pro Gln Val Tyr Thr
225                 230                 235                 240
Leu Pro Pro Ser Arg Glu Glu Met Thr Lys Asn Gln Val Ser Leu Thr
                245                 250                 255
Cys Leu Val Lys Gly Phe Tyr Pro Ser Asp Ile Ala Val Glu Trp Glu
            260                 265                 270

Ser Asn Gly Gln Pro Glu Asn Asn Tyr Lys Thr Thr Pro Pro Val Leu
        275                 280                 285
Asp Ser Asp Gly Ser Phe Phe Leu Tyr Ser Lys Leu Thr Val Asp Lys
    290                 295                 300
Ser Arg Trp Gln Gln Gly Asn Val Phe Ser Cys Ser Val Met His Glu
305                 310                 315                 320
Ala Leu His Asn His Tyr Thr Gln Lys Ser Leu Ser Leu Ser Pro Gly
                325                 330                 335
Lys

<210>  18
<211>  333
<212>  PRT
<213>  Artificial sequence
<220>
<223>  A heavy chain constant region sequence of an antibody in the fusion
protein, IgG2 type
<400>  18
Thr Thr Val Thr Val Ser Thr Ala Ser Thr Lys Gly Pro Ser Val Phe
1               5                   10                  15
Pro Leu Ala Pro Cys Ser Arg Ser Thr Ser Glu Ser Thr Ala Ala Leu
            20                  25                  30
Gly Cys Leu Val Lys Asp Tyr Phe Pro Glu Pro Val Thr Val Ser Trp
        35                  40                  45
Asn Ser Gly Ala Leu Thr Ser Gly Val His Thr Phe Pro Ala Val Leu
    50                  55                  60
Gln Ser Ser Gly Leu Tyr Ser Leu Ser Ser Val Val Thr Val Pro Ser
65                  70                  75                  80
Ser Asn Phe Gly Thr Gln Thr Tyr Thr Cys Asn Val Asp His Lys Pro
                85                  90                  95
Ser Asn Thr Lys Val Asp Lys Thr Val Glu Arg Lys Cys Cys Val Glu
            100                 105                 110
Cys Pro Pro Cys Pro Ala Pro Pro Val Ala Gly Pro Ser Val Phe Leu
        115                 120                 125
Phe Pro Pro Lys Pro Lys Asp Thr Leu Met Ile Ser Arg Thr Pro Glu
    130                 135                 140
Val Thr Cys Val Val Val Asp Val Ser His Glu Asp Pro Glu Val Gln
145                 150                 155                 160
Phe Asn Trp Tyr Val Asp Gly Val Glu Val His Asn Ala Lys Thr Lys
                165                 170                 175
Pro Arg Glu Glu Gln Phe Asn Ser Thr Phe Arg Val Val Ser Val Leu
            180                 185                 190
Thr Val Val His Gln Asp Trp Leu Asn Gly Lys Glu Tyr Lys Cys Lys
        195                 200                 205
Val Ser Asn Lys Gly Leu Pro Ala Pro Ile Glu Lys Thr Ile Ser Lys
    210                 215                 220
Thr Lys Gly Gln Pro Arg Glu Pro Gln Val Tyr Thr Leu Pro Pro Ser
225                 230                 235                 240
Arg Glu Glu Met Thr Lys Asn Gln Val Ser Leu Thr Cys Leu Val Lys
                245                 250                 255
Gly Phe Tyr Pro Ser Asp Ile Ala Val Glu Trp Glu Ser Asn Gly Gln
            260                 265                 270
Pro Glu Asn Asn Tyr Lys Thr Thr Pro Pro Met Leu Asp Ser Asp Gly
        275                 280                 285
Ser Phe Phe Leu Tyr Ser Lys Leu Thr Val Asp Lys Ser Arg Trp Gln
    290                 295                 300
Gln Gly Asn Val Phe Ser Cys Ser Val Met His Glu Ala Leu His Asn
305                 310                 315                 320
His Tyr Thr Gln Lys Ser Leu Ser Leu Ser Pro Gly Lys
                325                 330
<210>  19
<211>  334
<212>  PRT

<213>  Artificial sequence
<220>
<223>  A heavy chain constant region sequence of an antibody in the fusion
protein, IgG4 type
<400>  19
Thr Leu Val Thr Val Ser Ser Ala Ser Thr Lys Gly Pro Ser Val Phe
1               5                   10                  15
Pro Leu Ala Pro Cys Ser Arg Ser Thr Ser Glu Ser Thr Ala Ala Leu
            20                  25                  30
Gly Cys Leu Val Lys Asp Tyr Phe Pro Glu Pro Val Thr Val Ser Trp
        35                  40                  45
Asn Ser Gly Ala Leu Thr Ser Gly Val His Thr Phe Pro Ala Val Leu
    50                  55                  60
Gln Ser Ser Gly Leu Tyr Ser Leu Ser Ser Val Val Thr Val Pro Ser
65                  70                  75                  80
Ser Ser Leu Gly Thr Lys Thr Tyr Thr Cys Asn Val Asp His Lys Pro
                85                  90                  95
Ser Asn Thr Lys Val Asp Lys Arg Val Glu Ser Lys Tyr Gly Pro Pro
            100                 105                 110
Cys Pro Pro Cys Pro Ala Pro Glu Phe Leu Gly Gly Pro Ser Val Phe
        115                 120                 125
Leu Phe Pro Pro Lys Pro Lys Asp Thr Leu Met Ile Ser Arg Thr Pro
    130                 135                 140
Glu Val Thr Cys Val Val Val Asp Val Ser Gln Glu Asp Pro Glu Val
145                 150                 155                 160
Gln Phe Asn Trp Tyr Val Asp Gly Val Glu Val His Asn Ala Lys Thr
                165                 170                 175
Lys Pro Arg Glu Glu Gln Phe Asn Ser Thr Tyr Arg Val Val Ser Val
            180                 185                 190
Leu Thr Val Leu His Gln Asp Trp Leu Asn Gly Lys Glu Tyr Lys Cys
        195                 200                 205
Lys Val Ser Asn Lys Gly Leu Pro Ser Ser Ile Glu Lys Thr Ile Ser
    210                 215                 220
Lys Ala Lys Gly Gln Pro Arg Glu Pro Gln Val Tyr Thr Leu Pro Pro
225                 230                 235                 240
Ser Gln Glu Glu Met Thr Lys Asn Gln Val Ser Leu Thr Cys Leu Val
                245                 250                 255
Lys Gly Phe Tyr Pro Ser Asp Ile Ala Val Glu Trp Glu Ser Asn Gly
            260                 265                 270
Gln Pro Glu Asn Asn Tyr Lys Thr Thr Pro Pro Val Leu Asp Ser Asp
        275                 280                 285
Gly Ser Phe Phe Leu Tyr Ser Arg Leu Thr Val Asp Lys Ser Arg Trp
    290                 295                 300
Gln Glu Gly Asn Val Phe Ser Cys Ser Val Met His Glu Ala Leu His
305                 310                 315                 320
Asn His Tyr Thr Gln Lys Ser Leu Ser Leu Ser Leu Gly Lys
                325                 330
<210>  20
<211>  16
<212>  PRT
<213>  Artificial sequence
<220>
<223>  A peptide linker in the fusion protein
<400>  20
Ala Lys Thr Thr Pro Lys Leu Glu Glu Gly Glu Phe Ser Glu Ala Arg
1               5                   10                  15
<210>  21
<211>  17
<212>  PRT
<213>  Artificial sequence
<220>
<223>  A peptide linker in the fusion protein
<400>  21

Ala Lys Thr Thr Pro Lys Leu Glu Glu Gly Glu Phe Ser Glu Ala Arg
1               5                   10                  15
Val

<210>  22
<211>  9
<212>  PRT
<213>  Artificial sequence
<220>
<223>  A peptide linker in the fusion protein
<400>  22
Ala Lys Thr Thr Pro Lys Leu Gly Gly
1               5
<210>  23
<211>  10
<212>  PRT
<213>  Artificial sequence
<220>
<223>  A peptide linker in the fusion protein
<400>  23
Ser Ala Lys Thr Thr Pro Lys Leu Gly Gly
1               5                   10
<210>  24
<211>  6
<212>  PRT
<213>  Artificial sequence
<220>
<223>  A peptide linker in the fusion protein
<400>  24
Ser Ala Lys Thr Thr Pro
1               5
<210>  25
<211>  6
<212>  PRT
<213>  Artificial sequence
<220>
<223>  A peptide linker in the fusion protein
<400>  25
Arg Ala Asp Ala Ala Pro
1               5
<210>  26
<211>  9
<212>  PRT
<213>  Artificial sequence
<220>
<223>  A peptide linker in the fusion protein
<400>  26
Arg Ala Asp Ala Ala Pro Thr Val Ser
1               5
<210>  27
<211>  12
<212>  PRT
<213>  Artificial sequence
<220>
<223>  A peptide linker in the fusion protein
<400>  27
Arg Ala Asp Ala Ala Ala Ala Gly Gly Pro Gly Ser
1               5                   10
<210>  28
<211>  7
<212>  PRT
<213>  Artificial sequence
<220>

<223>  A peptide linker in the fusion protein
<400>  28
Arg Ala Asp Ala Ala Ala Ala
1               5
<210>  29
<211>  18
<212>  PRT
<213>  Artificial sequence
<220>
<223>  A peptide linker in the fusion protein
<400>  29
Ser Ala Lys Thr Thr Pro Lys Leu Glu Glu Gly Glu Phe Ser Glu Ala
1               5                   10                  15
Arg Val

<210>  30
<211>  5
<212>  PRT
<213>  Artificial sequence
<220>
<223>  A peptide linker in the fusion protein
<400>  30
Ala Asp Ala Ala Pro
1               5
<210>  31
<211>  11
<212>  PRT
<213>  Artificial sequence
<220>
<223>  A peptide linker in the fusion protein
<400>  31
Asp Ala Ala Pro Thr Val Ser Ile Phe Pro Pro
1               5                   10
<210>  32
<211>  5
<212>  PRT
<213>  Artificial sequence
<220>
<223>  A peptide linker in the fusion protein
<400>  32
Thr Val Ala Ala Pro
1               5
<210>  33
<211>  12
<212>  PRT
<213>  Artificial sequence
<220>
<223>  A peptide linker in the fusion protein
<400>  33
Thr Val Ala Ala Pro Ser Val Phe Ile Phe Pro Pro
1               5                   10
<210>  34
<211>  6
<212>  PRT
<213>  Artificial sequence
<220>
<223>  A peptide linker in the fusion protein
<400>  34
Gln Pro Lys Ala Ala Pro
1               5
<210>  35
<211>  13
<212>  PRT

<213>  Artificial sequence
<220>
<223>  A peptide linker in the fusion protein
<400>  35
Gln Pro Lys Ala Ala Pro Ser Val Thr Leu Phe Pro Pro
1               5                   10
<210>  36
<211>  6
<212>  PRT
<213>  Artificial sequence
<220>
<223>  A peptide linker in the fusion protein
<400>  36
Ala Lys Thr Thr Pro Pro
1               5
<210>  37
<211>  13
<212>  PRT
<213>  Artificial sequence
<220>
<223>  A peptide linker in the fusion protein
<400>  37
Ala Lys Thr Thr Pro Pro Ser Val Thr Pro Leu Ala Pro
1               5                   10
<210>  38
<211>  6
<212>  PRT
<213>  Artificial sequence
<220>
<223>  A peptide linker in the fusion protein
<400>  38
Ala Lys Thr Thr Ala Pro
1               5
<210>  39
<211>  13
<212>  PRT
<213>  Artificial sequence
<220>
<223>  A peptide linker in the fusion protein
<400>  39
Ala Lys Thr Thr Ala Pro Ser Val Tyr Pro Leu Ala Pro
1               5                   10
<210>  40
<211>  6
<212>  PRT
<213>  Artificial sequence
<220>
<223>  A peptide linker in the fusion protein
<400>  40
Ala Ser Thr Lys Gly Pro
1               5
<210>  41
<211>  13
<212>  PRT
<213>  Artificial sequence
<220>
<223>  A peptide linker in the fusion protein
<400>  41
Ala Ser Thr Lys Gly Pro Ser Val Phe Pro Leu Ala Pro
1               5                   10
<210>  42
<211>  15
<212>  PRT

<213>  Artificial sequence
<220>
<223>  A peptide linker in the fusion protein
<400>  42
Gly Gly Gly Gly Ser Gly Gly Gly Gly Ser Gly Gly Gly Gly Ser
1               5                   10                  15
<210>  43
<211>  15
<212>  PRT
<213>  Artificial sequence
<220>
<223>  A peptide linker in the fusion protein
<400>  43
Gly Glu Asn Lys Val Glu Tyr Ala Pro Ala Leu Met Ala Leu Ser
1               5                   10                  15
<210>  44
<211>  15
<212>  PRT
<213>  Artificial sequence
<220>
<223>  A peptide linker in the fusion protein
<400>  44
Gly Pro Ala Lys Glu Leu Thr Pro Leu Lys Glu Ala Lys Val Ser
1               5                   10                  15
<210>  45
<211>  15
<212>  PRT
<213>  Artificial sequence
<220>
<223>  A peptide linker in the fusion protein
<400>  45
Gly His Glu Ala Ala Ala Val Met Gln Val Gln Tyr Pro Ala Ser
1               5                   10                  15
<210>  46
<211>  16
<212>  PRT
<213>  Artificial sequence
<220>
<223>  A peptide linker in the fusion protein
<400>  46
Gly Gly Gly Gly Ser Gly Gly Gly Gly Ser Gly Gly Gly Gly Ser Ala
1               5                   10                  15
<210>  47
<211>  106
<212>  PRT
<213>  Artificial sequence
<220>
<223>  A heavy chain variable region sequence of an anti-PD-1 antibody in
combination therapy
<400>  47
Gln Val Gln Leu Val Glu Ser Gly Gly Gly Val Val Gln Pro Gly Arg
1               5                   10                  15
Ser Leu Arg Leu Asp Cys Lys Ala Ser Gly Ile Thr Phe Ser Asn Ser
            20                  25                  30
Gly Met His Trp Val Arg Gln Ala Pro Gly Lys Gly Leu Glu Trp Val
        35                  40                  45
Ala Val Ile Trp Tyr Asp Gly Ser Lys Arg Tyr Tyr Ala Asp Ser Val
    50                  55                  60
Lys Gly Arg Phe Thr Ile Ser Arg Asp Asn Ser Lys Asn Thr Leu Phe
65                  70                  75                  80
Leu Gln Met Asn Ser Leu Arg Ala Glu Asp Thr Ala Val Tyr Tyr Cys
                85                  90                  95
Ala Thr Asn Asp Asp Tyr Trp Gly Gln Gly

                100                 105
<210>  48
<211>  101
<212>  PRT
<213>  Artificial sequence
<220>
<223>  A light chain variable region sequence of an anti-PD-1 antibody in
combination therapy
<400>  48
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Asp Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Ser Ser Asn Trp Pro Arg
                85                  90                  95
Thr Phe Gly Gln Gly
            100
<210>  49
<211>  113
<212>  PRT
<213>  Artificial sequence
<220>
<223>  A heavy chain variable region sequence of an anti-PD-1 antibody in
combination therapy
<400>  49
Gln Val Gln Leu Val Gln Ser Gly Val Glu Val Lys Lys Pro Gly Ala
1               5                   10                  15
Ser Val Lys Val Ser Cys Lys Ala Ser Gly Tyr Thr Phe Thr Asn Tyr
            20                  25                  30
Tyr Met Tyr Trp Val Arg Gln Ala Pro Gly Gln Gly Leu Glu Trp Met
        35                  40                  45
Gly Gly Ile Asn Pro Ser Asn Gly Gly Thr Asn Phe Asn Glu Lys Phe
    50                  55                  60
Lys Asn Arg Val Thr Leu Thr Thr Asp Ser Ser Thr Thr Thr Ala Tyr
65                  70                  75                  80
Met Glu Leu Lys Ser Leu Gln Phe Asp Asp Thr Ala Val Tyr Tyr Cys
                85                  90                  95
Ala Arg Arg Asp Tyr Arg Phe Asp Met Gly Phe Asp Tyr Trp Gly Gln
            100                 105                 110
Gly

<210>  50
<211>  105
<212>  PRT
<213>  Artificial sequence
<220>
<223>  A light chain variable region sequence of an anti-PD-1 antibody in
combination therapy
<400>  50
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Lys Gly Val Ser Thr Ser
            20                  25                  30
Gly Tyr Ser Tyr Leu His Trp Tyr Gln Gln Lys Pro Gly Gln Ala Pro
        35                  40                  45
Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80
Ser Leu Glu Pro Glu Asp Phe Ala Val Tyr Tyr Cys Gln His Ser Arg
                85                  90                  95
Asp Leu Pro Leu Thr Phe Gly Gly Gly
            100                 105
<210>  51
<211>  110
<212>  PRT
<213>  Artificial sequence
<220>
<223>  A heavy chain variable region sequence of an anti-PD-1 antibody in
combination therapy
<400>  51
Gln Val Gln Leu Val Gln Ser Gly Ser Glu Leu Lys Lys Pro Gly Ala
1               5                   10                  15
Ser Val Lys Ile Ser Cys Lys Ala Ser Gly Tyr Thr Phe Thr Asn Tyr
            20                  25                  30
Gly Met Asn Trp Val Arg Gln Ala Pro Gly Gln Gly Leu Gln Trp Met
        35                  40                  45
Gly Trp Ile Asn Thr Asp Ser Gly Glu Ser Thr Tyr Ala Glu Glu Phe
    50                  55                  60
Lys Gly Arg Phe Val Phe Ser Leu Asp Thr Ser Val Asn Thr Ala Tyr
65                  70                  75                  80
Leu Gln Ile Thr Ser Leu Thr Ala Glu Asp Thr Gly Met Tyr Phe Cys
                85                  90                  95
Val Arg Val Gly Tyr Asp Ala Leu Asp Tyr Trp Gly Gln Gly
            100                 105                 110
<210>  52
<211>  100
<212>  PRT
<213>  Artificial sequence
<220>
<223>  A light chain variable region sequence of an anti-PD-1 antibody in
combination therapy
<400>  52
Glu Ile Val Leu Thr Gln Ser Pro Ser Ser Leu Ser Ala Ser Val Gly
1               5                   10                  15
Asp Arg Val Thr Ile Thr Cys Ser Ala Arg Ser Ser Val Ser Tyr Met
            20                  25                  30
His Trp Phe Gln Gln Lys Pro Gly Lys Ala Pro Lys Leu Trp Ile Tyr
        35                  40                  45
Arg Thr Ser Asn Leu Ala Ser Gly Val Pro Ser Arg Phe Ser Gly Ser
    50                  55                  60
Gly Ser Gly Thr Ser Tyr Cys Leu Thr Ile Asn Ser Leu Gln Pro Glu
65                  70                  75                  80
Asp Phe Ala Thr Tyr Tyr Cys Gln Gln Arg Ser Ser Phe Pro Leu Thr
                85                  90                  95
Phe Gly Gly Gly
            100
<210>  53
<211>  109
<212>  PRT
<213>  Artificial sequence
<220>
<223>  A heavy chain variable region sequence of an anti-PD-1 antibody in
combination therapy
<400>  53
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Met Met Ser Trp Val Arg Gln Ala Pro Gly Lys Gly Leu Glu Trp Val

35                  40                  45
Ala Thr Ile Ser Gly Gly Gly Ala Asn Thr Tyr Tyr Pro Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Gln Leu Tyr Tyr Phe Asp Tyr Trp Gly Gln Gly
            100                 105
<210>  54
<211>  101
<212>  PRT
<213>  Artificial sequence
<220>
<223>  A light chain variable region sequence of an anti-PD-1 antibody in
combination therapy
<400>  54
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Thr Ile Gly Thr Trp
            20                  25                  30
Leu Thr Trp Tyr Gln Gln Lys Pro Gly Lys Ala Pro Lys Leu Leu Ile
        35                  40                  45
Tyr Thr Ala Thr Ser Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Val Tyr Ser Ile Pro Trp
                85                  90                  95
Thr Phe Gly Gly Gly
            100
<210>  55
<211>  114
<212>  PRT
<213>  Artificial sequence
<220>
<223>  A heavy chain variable region sequence of an anti-PD-1 antibody in
combination therapy
<400>  55
Gln Val Thr Leu Lys Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Thr Cys Val Ser Trp Ile Arg Gln Pro Pro Gly Lys Ala Leu Glu
        35                  40                  45
Trp Leu Ala Thr Ile Cys Trp Glu Asp Ser Lys Gly Tyr Asn Pro Ser
    50                  55                  60
Leu Lys Ser Arg Leu Thr Ile Ser Lys Asp Thr Ser Lys Asn Gln Ala
65                  70                  75                  80
Val Leu Thr Met Thr Asn Met Asp Pro Val Asp Thr Ala Thr Tyr Tyr
                85                  90                  95
Cys Ala Arg Arg Glu Asp Ser Gly Tyr Phe Trp Phe Pro Tyr Trp Gly
            100                 105                 110
Gln Gly

<210>  56
<211>  100
<212>  PRT
<213>  Artificial sequence
<220>
<223>  A light chain variable region sequence of an anti-PD-1 antibody in
combination therapy
<400>  56

Asn Ile Gln Met Thr Gln Ser Pro Ser Ser Leu Ser Ala Ser Val Gly
1               5                   10                  15
Asp Arg Val Thr Ile Thr Cys Lys Ala Gly Gln Asn Val Asn Asn Tyr
            20                  25                  30
Leu Ala Trp Tyr Gln Gln Lys Pro Gly Lys Ala Pro Lys Val Leu Ile
        35                  40                  45
Phe Asn Ala Asn Ser Leu Gln Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Asn Ser Trp Thr Thr
                85                  90                  95
Phe Gly Gly Gly
            100
<210>  57
<211>  111
<212>  PRT
<213>  Artificial sequence
<220>
<223>  A heavy chain variable region sequence of an anti-PD-1 antibody in
combination therapy
<400>  57
Gln Val Gln Leu Gln Glu Ser Gly Pro Gly Leu Val Lys Pro Ser Glu
1               5                   10                  15
Thr Leu Ser Leu Thr Cys Thr Val Ser Gly Phe Ser Leu Thr Ser Tyr
            20                  25                  30
Gly Val His Trp Ile Arg Gln Pro Pro Gly Lys Gly Leu Glu Trp Leu
        35                  40                  45
Gly Val Ile Trp Ala Gly Gly Ser Thr Asn Tyr Asn Pro Ser Leu Lys
    50                  55                  60
Ser Arg Leu Thr Ile Ser Lys Asp Asn Ser Lys Ser Gln Val Ser Leu
65                  70                  75                  80
Lys Met Ser Ser Val Thr Ala Ala Asp Thr Ala Val Tyr Tyr Cys Ala
                85                  90                  95
Arg Ala Tyr Gly Asn Tyr Trp Tyr Ile Asp Val Trp Gly Gln Gly
            100                 105                 110
<210>  58
<211>  101
<212>  PRT
<213>  Artificial sequence
<220>
<223>  A light chain variable region sequence of an anti-PD-1 antibody in
combination therapy
<400>  58
Asp Ile Val Met Thr Gln Ser Pro Asp Ser Leu Ala Val Ser Leu Gly
1               5                   10                  15
Glu Arg Ala Thr Ile Asn Cys Lys Ala Ser Gln Ser Val Ser Asn Asp
            20                  25                  30
Val Ala Trp Tyr Gln Gln Lys Pro Gly Gln Pro Pro Lys Leu Leu Ile
        35                  40                  45
Asn Tyr Ala Phe His Arg Phe Thr Gly Val Pro Asp Arg Phe Ser Gly
    50                  55                  60
Ser Gly Tyr Gly Thr Asp Phe Thr Leu Thr Ile Ser Ser Leu Gln Ala
65                  70                  75                  80
Glu Asp Val Ala Val Tyr Tyr Cys His Gln Ala Tyr Ser Ser Pro Tyr
                85                  90                  95
Thr Phe Gly Gly Gly
            100
<210>  59
<211>  105
<212>  PRT
<213>  Artificial sequence
<220>

<223>  A heavy chain variable region sequence of an anti-PD-1 antibody in
combination therapy
<400>  59
Val Gln Leu Val Glu Ser Gly Gly Gly Val Val Gln Pro Gly Arg Ser
1               5                   10                  15
Leu Arg Leu Asp Cys Lys Ala Ser Gly Ile Thr Phe Ser Asn Tyr Gly
            20                  25                  30
Met His Trp Val Arg Gln Ala Pro Gly Lys Gly Leu Glu Trp Val Ala
        35                  40                  45
Val Ile Trp Tyr Asp Ser Ser Arg Lys Tyr Tyr Ala Asp Ser Val Lys
    50                  55                  60
Gly Arg Phe Thr Ile Ser Arg Asp Asn Ser Lys Asn Thr Leu Phe Leu
65                  70                  75                  80
Gln Met Asn Ser Leu Arg Ala Glu Asp Thr Ala Val Tyr Tyr Cys Ala
                85                  90                  95
Thr Asn Asn Asp Tyr Trp Gly Gln Gly
            100                 105
<210>  60
<211>  101
<212>  PRT
<213>  Artificial sequence
<220>
<223>  A light chain variable region sequence of an anti-PD-1 antibody in
combination therapy
<400>  60
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Ser Val Ser Asn Tyr
            20                  25                  30
Leu Asp Trp Tyr Gln Gln Lys Pro Gly Lys Ala Pro Lys Leu Leu Ile
        35                  40                  45
Tyr Asp Ala Ser Thr Arg Ala Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Asn Met Gln Leu Pro Leu
                85                  90                  95
Thr Phe Gly Gln Gly
            100
<210>  61
<211>  113
<212>  PRT
<213>  Artificial sequence
<220>
<223>  A heavy chain variable region sequence of an anti-PD-1 antibody in
combination therapy
<400>  61
Asp Leu Val Gln Ser Gly Ala Glu Val Lys Lys Pro Gly Ala Ser Val
1               5                   10                  15
Lys Val Ser Cys Lys Ala Ser Gly Tyr Thr Phe Thr Ser Tyr Gly Ile
            20                  25                  30
Ser Trp Val Arg Gln Ala Pro Gly Gln Gly Leu Glu Trp Met Gly Trp
        35                  40                  45
Ile Ser Ala Tyr Asn Gly Asn Thr Asn Tyr Ala Gln Lys Leu Gln Gly
    50                  55                  60
Arg Val Thr Met Thr Thr Asp Thr Ser Thr Ser Thr Ala Tyr Met Glu
65                  70                  75                  80
Leu Arg Ser Leu Arg Ser Asp Asp Thr Ala Val Tyr Tyr Cys Ala Arg
                85                  90                  95
Gly Arg Gly Tyr Ser Tyr Gly Ile Asp Ala Phe Asp Ile Trp Gly Gln
            100                 105                 110
Gly

<210>  62
<211>  103
<212>  PRT
<213>  Artificial sequence
<220>
<223>  A light chain variable region sequence of an anti-PD-1 antibody in
combination therapy
<400>  62
Leu Ser Tyr Val Leu Thr Gln Pro Pro Ser Val Ser Val Ser Pro Gly
1               5                   10                  15
Gln Thr Ala Arg Ile Thr Cys Ser Gly Asp Ala Leu Pro Lys Gln Tyr
            20                  25                  30
Ala Tyr Trp Tyr Gln Gln Lys Pro Gly Gln Ala Pro Val Leu Val Ile
        35                  40                  45
Tyr Lys Asp Ser Glu Arg Pro Ser Gly Ile Pro Glu Arg Phe Ser Gly
    50                  55                  60
Ser Ser Ser Gly Thr Thr Val Thr Leu Thr Ile Ser Gly Val Gln Ala
65                  70                  75                  80
Glu Asp Glu Ala Asp Tyr Tyr Cys Gln Ser Ala Asp Ser Ser Gly Thr
                85                  90                  95
Tyr Val Val Phe Gly Gly Gly
            100
<210>  63
<211>  118
<212>  PRT
<213>  Artificial sequence
<220>
<223>  A heavy chain variable region sequence of an anti-PD-1 antibody in
combination therapy
<400>  63
Gln Gly Gln Leu Val Gln Ser Gly Ala Glu Val Lys Lys Pro Gly Ala
1               5                   10                  15
Ser Val Lys Val Ser Cys Lys Ala Ser Gly Tyr Thr Phe Thr Asp Tyr
            20                  25                  30
Glu Met His Trp Val Arg Gln Ala Pro Gly Gln Gly Leu Glu Trp Met
        35                  40                  45
Gly Val Ile Glu Ser Glu Thr Gly Gly Thr Ala Tyr Asn Gln Lys Phe
    50                  55                  60
Gln Gly Arg Val Thr Leu Thr Ala Asp Lys Ser Ser Ser Thr Ala Tyr
65                  70                  75                  80
Met Glu Leu Ser Ser Leu Arg Ser Glu Asp Thr Ala Val Tyr Tyr Cys
                85                  90                  95
Thr Arg Glu Gly Ile Thr Thr Val Ala Thr Thr Tyr Tyr Trp Tyr Phe
            100                 105                 110
Asp Val Trp Gly Gln Gly
        115
<210>  64
<211>  106
<212>  PRT
<213>  Artificial sequence
<220>
<223>  A light chain variable region sequence of an anti-PD-1 antibody in
combination therapy
<400>  64
Asp Val Val Met Thr Gln Ser Pro Leu Ser Leu Pro Val Thr Leu Gly
1               5                   10                  15
Gln Pro Ala Ser Ile Ser Cys Arg Ser Ser Gln Ser Ile Val His Ser
            20                  25                  30
Asn Gly Asn Thr Tyr Leu Glu Trp Tyr Leu Gln Lys Pro Gly Gln Ser
        35                  40                  45
Pro Gln Leu Leu Ile Tyr Lys Val Ser Asn Arg Phe Ser Gly Val Pro
    50                  55                  60
Asp Arg Phe Ser Gly Ser Gly Ser Gly Thr Asp Phe Thr Leu Lys Ile

65                  70                  75                  80
Ser Arg Val Glu Ala Glu Asp Val Gly Val Tyr Tyr Cys Phe Gln Gly
                85                  90                  95
Ser His Val Pro Leu Thr Phe Gly Gln Gly
            100                 105
<210>  65
<211>  111
<212>  PRT
<213>  Artificial sequence
<220>
<223>  A heavy chain variable region sequence of an anti-PD-1 antibody in
combination therapy
<400>  65
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Gly Met Ser Trp Val Arg Gln Ala Pro Gly Lys Gly Leu Glu Trp Val
        35                  40                  45
Ala Thr Ile Ser Gly Gly Gly Ser Asp Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Gln Leu Asn Tyr Ala Trp Phe Ala Tyr Trp Gly Gln Gly
            100                 105                 110
<210>  66
<211>  105
<212>  PRT
<213>  Artificial sequence
<220>
<223>  A light chain variable region sequence of an anti-PD-1 antibody in
combination therapy
<400>  66
Asp Ile Val Leu Thr Gln Ser Pro Ala Ser Leu Ala Val Ser Pro Gly
1               5                   10                  15
Gln Arg Ala Thr Ile Thr Cys Arg Ala Ser Glu Ser Val Asp Asn Tyr
            20                  25                  30
Gly Ile Ser Phe Met Asn Trp Tyr Gln Gln Lys Pro Gly Gln Pro Pro
        35                  40                  45
Lys Leu Leu Ile Tyr Thr Ser Ser Asn Lys Asp Thr Gly Val Pro Ala
    50                  55                  60
Arg Phe Ser Gly Ser Gly Ser Gly Thr Asp Phe Thr Leu Thr Ile Asn
65                  70                  75                  80
Pro Met Glu Ala Glu Asp Thr Ala Val Tyr Tyr Cys Gln Gln Ser Lys
                85                  90                  95
Glu Val Pro Trp Thr Phe Gly Gly Gly
            100                 105
<210>  67
<211>  113
<212>  PRT
<213>  Artificial sequence
<220>
<223>  A heavy chain variable region sequence of an anti-PD-1 antibody in
combination therapy
<400>  67
Gln Val Gln Leu Val Gln Ser Gly Ala Glu Val Lys Lys Pro Gly Ser
1               5                   10                  15
Ser Val Lys Val Ser Cys Lys Ala Ser Gly Gly Thr Phe Ser Ser Tyr
            20                  25                  30
Ala Ile Ser Trp Val Arg Gln Ala Pro Gly Gln Gly Leu Glu Trp Met
        35                  40                  45

Gly Leu Ile Ile Pro Met Phe Asp Thr Ala Gly Tyr Ala Gln Lys Phe
    50                  55                  60
Gln Gly Arg Val Ala Ile Thr Val Asp Glu Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Ala Glu His Ser Ser Thr Gly Thr Phe Asp Tyr Trp Gly Gln
            100                 105                 110
Gly

<210>  68
<211>  101
<212>  PRT
<213>  Artificial sequence
<220>
<223>  A light chain variable region sequence of an anti-PD-1 antibody in
combination therapy
<400>  68
Asp Ile Gln Met Thr Gln Ser Pro Ser Ser Val Ser Ala Ser Val Gly
1               5                   10                  15
Asp Arg Val Thr Ile Thr Cys Arg Ala Ser Gln Gly Ile Ser Ser Trp
            20                  25                  30
Leu Ala Trp Tyr Gln Gln Lys Pro Gly Lys Ala Pro Lys Leu Leu Ile
        35                  40                  45
Ser Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ala Asn His Leu Pro Phe
                85                  90                  95
Thr Phe Gly Gly Gly
            100
<210>  69
<211>  116
<212>  PRT
<213>  Artificial sequence
<220>
<223>  A heavy chain variable region sequence of an anti-PD-1 antibody in
combination therapy
<400>  69
Gln Leu Gln Leu Gln Glu Ser Gly Pro Gly Leu Val Lys Pro Ser Glu
1               5                   10                  15
Thr Leu Thr Leu Thr Cys Thr Val Ser Ala Asp Ser Ile Ser Ser Thr
            20                  25                  30
Thr Tyr Tyr Trp Val Trp Ile Arg Gln Pro Pro Gly Lys Gly Leu Glu
        35                  40                  45
Trp Ile Gly Ser Ile Ser Tyr Ser Gly Ser Thr Tyr Tyr Asn Pro Ser
    50                  55                  60
Leu Lys Ser Arg Val Thr Val Ser Val Asp Thr Ser Lys Asn Gln Phe
65                  70                  75                  80
Ser Leu Lys Leu Asn Ser Val Ala Ala Thr Asp Thr Ala Leu Tyr Tyr
                85                  90                  95
Cys Ala Arg His Leu Gly Tyr Asn Gly Arg Tyr Leu Pro Phe Asp Tyr
            100                 105                 110
Trp Gly Gln Gly
        115
<210>  70
<211>  104
<212>  PRT
<213>  Artificial sequence
<220>
<223>  A light chain variable region sequence of an anti-PD-1 antibody in
combination therapy

<400>  70
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Phe Tyr
            20                  25                  30
Asn Tyr Val Ser Trp Tyr Gln Gln His Pro Gly Lys Ala Pro Glu Leu
        35                  40                  45
Met Ile Tyr Asp Val Ser Asn Arg Pro Ser Gly Val Ser Asp Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Asn Ile
                85                  90                  95
Ser Thr Trp Val Phe Gly Gly Gly
            100
<210>  71
<211>  106
<212>  PRT
<213>  Artificial sequence
<220>
<223>  A heavy chain variable region sequence of an anti-PD-1 antibody in
combination therapy
<400>  71
Gln Val Gln Leu Val Glu Ser Gly Gly Gly Val Val Gln Pro Gly Arg
1               5                   10                  15
Ser Leu Arg Leu Thr Cys Lys Ala Ser Gly Leu Thr Phe Ser Ser Ser
            20                  25                  30
Gly Met His Trp Val Arg Gln Ala Pro Gly Lys Gly Leu Glu Trp Val
        35                  40                  45
Ala Val Ile Trp Tyr Asp Gly Ser Lys Arg Tyr Tyr Ala Asp Ser Val
    50                  55                  60
Lys Gly Arg Phe Thr Ile Ser Arg Asp Asn Ser Lys Asn Thr Leu Phe
65                  70                  75                  80
Leu Gln Met Asn Ser Leu Arg Ala Glu Asp Thr Ala Val Tyr Tyr Cys
                85                  90                  95
Ala Thr Asn Asn Asp Tyr Trp Gly Gln Gly
            100                 105
<210>  72
<211>  101
<212>  PRT
<213>  Artificial sequence
<220>
<223>  A light chain variable region sequence of an anti-PD-1 antibody in
combination therapy
<400>  72
Glu Ile Val Leu Thr Gln Ser Pro Ala Thr Leu Ser Leu Ser Pro Gly
1               5                   10                  15
Glu Arg Ala Thr Leu Ser Cys Arg Ala Ser Gln Ser Val Ser Ser Tyr
            20                  25                  30
Leu Ala Trp Tyr Gln Gln Lys Pro Gly Gln Ala Pro Arg Leu Leu Ile
        35                  40                  45
Tyr Thr Ala Ser Asn Arg Ala Thr Gly Ile Pro Ala Arg Phe Ser Gly
    50                  55                  60
Ser Gly Ser Gly Thr Asp Phe Thr Leu Thr Ile Ser Ser Leu Glu Pro
65                  70                  75                  80
Glu Asp Phe Ala Val Tyr Tyr Cys Gln Gln Tyr Ser Asn Trp Pro Arg
                85                  90                  95
Thr Phe Gly Gln Gly
            100
<210>  73
<211>  723
<212>  DNA
<213>  Artificial sequence

<220>
<223>  A nucleotide sequence of the light chain (BY18.1L) of the anti-PD1
antibody BY18.1
<400>  73
ctcgaggcca ccatggagac cgacacactc ctcctgtggg tgctgctgct gtgggtgcct      60
ggctccactg gcgagattgt gctgacacag tcccccgcta ctctgagcct gagccctggc     120
gagagggcta cactgtcttg cagagcttct cagtccgtgt cttcttacct cgcttggtat     180
cagcagaagc ccggccaggc tccaagactg ctgatctatg acgcttctaa ccgcgctaca     240
ggcattcctg ctaggttcag cggcagcggc tctggcaccg acttcacact cacaattagc     300
tctcttgaac ctgaggactt cgccgtgtac tactgccagc agtctagcaa ctggcctaga     360
acattcggcc agggcactaa ggtggagatt aagagaaccg tggccgcccc cagcgtgttc     420
atcttccctc ccagcgacga gcagctgaag tctggcaccg ccagcgtggt gtgcctgctg     480
aacaacttct acccccgcga ggccaaggtg cagtggaagg tggacaacgc cctgcagagc     540
ggcaacagcc aggagagcgt gaccgagcag gactccaagg acagcaccta cagcctgagc     600
agcaccctga ccctgagcaa ggccgactac gagaagcaca aggtgtacgc ctgcgaggtg     660
acccaccagg gactgtctag ccccgtgacc aagagcttca accggggcga gtgctaagaa     720
ttc                                                                   723
<210>  74
<211>  234
<212>  PRT
<213>  Artificial sequence
<220>
<223>  An amino acid sequence of light chain (BY18.1L) of anti-PD1 antibody
BY18.1
<400>  74
Met Glu Thr Asp Thr Leu Leu Leu Trp Val Leu Leu Leu Trp Val Pro
1               5                   10                  15
Gly Ser Thr Gly Glu Ile Val Leu Thr Gln Ser Pro Ala Thr Leu Ser
            20                  25                  30
Leu Ser Pro Gly Glu Arg Ala Thr Leu Ser Cys Arg Ala Ser Gln Ser
        35                  40                  45
Val Ser Ser Tyr Leu Ala Trp Tyr Gln Gln Lys Pro Gly Gln Ala Pro
    50                  55                  60

Arg Leu Leu Ile Tyr Asp Ala Ser Asn Arg Ala Thr Gly Ile Pro Ala
65                  70                  75                  80
Arg Phe Ser Gly Ser Gly Ser Gly Thr Asp Phe Thr Leu Thr Ile Ser
                85                  90                  95
Ser Leu Glu Pro Glu Asp Phe Ala Val Tyr Tyr Cys Gln Gln Ser Ser
            100                 105                 110
Asn Trp Pro Arg Thr Phe Gly Gln Gly Thr Lys Val Glu Ile Lys Arg
        115                 120                 125
Thr Val Ala Ala Pro Ser Val Phe Ile Phe Pro Pro Ser Asp Glu Gln
    130                 135                 140
Leu Lys Ser Gly Thr Ala Ser Val Val Cys Leu Leu Asn Asn Phe Tyr
145                 150                 155                 160
Pro Arg Glu Ala Lys Val Gln Trp Lys Val Asp Asn Ala Leu Gln Ser
                165                 170                 175
Gly Asn Ser Gln Glu Ser Val Thr Glu Gln Asp Ser Lys Asp Ser Thr
            180                 185                 190
Tyr Ser Leu Ser Ser Thr Leu Thr Leu Ser Lys Ala Asp Tyr Glu Lys
        195                 200                 205
His Lys Val Tyr Ala Cys Glu Val Thr His Gln Gly Leu Ser Ser Pro
    210                 215                 220
Val Thr Lys Ser Phe Asn Arg Gly Glu Cys
225                 230
<210>  75
<211>  1401
<212>  DNA
<213>  Artificial sequence
<220>
<223>  A nucleotide sequence of heavy chain (BY18.1H) of anti-PD1 antibody
BY18.1

<400>  75
tctagagcca ccatggagac cgacaccctg ctgctgtggg tgctgctcct gtgggtgcct      60
ggctccacag gccaggtgca gctcgtggag tccggcggcg gcgtggtgca gcccggcaga     120
tccctcagac tggactgcaa ggcatccggc attacattct ctaactctgg aatgcactgg     180
gtgagacagg ctcctggcaa gggcctggaa tgggtggccg tgatttggta cgacggctct     240
aagagatact acgctgactc cgtgaagggc cggttcacaa ttagcagaga caactccaag     300
aacactctgt tcctccagat gaacagcctg agagccgagg acaccgctgt gtactactgc     360
gccaccaacg acgactactg gggccagggc accctcgtga cagtgtcttc cgcctccacc     420
aagggccctt ccgtgttccc tctggcccct tgctcccgct ccacctccga gtccaccgcc     480
gccctgggct gcctggtgaa ggactacttc cctgagcctg tgaccgtgtc ctggaactcc     540
ggcgccctga cctccggcgt gcacaccttc cctgccgtgc tgcagtcctc cggcctgtac     600
tccctgtcct ccgtggtgac cgtgccttcc tcctccctgg gcaccaagac ctacacctgc     660
aacgtggacc acaagccttc caacaccaag gtggacaagc gcgtggagtc caagtacggc     720
cctccttgcc ctccttgccc tgcccctgag ttcctgggcg gcccttccgt gttcctgttc     780
cctcctaagc ctaaggacac cctgatgatc tcccgcaccc ctgaggtgac ctgcgtggtg     840
gtggacgtgt cccaggagga ccctgaggtg cagttcaact ggtacgtgga cggcgtggag     900
gtgcacaacg ccaagaccaa gcctcgcgag gagcagttca actccaccta ccgcgtggtg     960
tccgtgctga ccgtgctgca ccaggactgg ctgaacggca aggagtacaa gtgcaaggtg    1020
tccaacaagg gcctgccttc ctccatcgag aagaccatct ccaaggccaa gggccagcct    1080
cgcgagcctc aggtgtacac cctgcctcct tcccaggagg agatgaccaa gaaccaggtg    1140
tccctgacct gcctggtgaa gggcttctac ccttccgaca tcgccgtgga gtgggagtcc    1200
aacggccagc ctgagaacaa ctacaagacc acccctcctg tgctggactc cgacggctcc    1260
ttcttcctgt actcccgcct gaccgtggac aagtcccgct ggcaggaggg caacgtgttc    1320
tcctgctccg tgatgcacga ggccctgcac aaccactaca cccagaagtc cctgtccctg    1380
tccctgggca agtaagtcga c                                              1401
<210>  76
<211>  460
<212>  PRT
<213>  Artificial sequence
<220>
<223>  An amino acid sequence of heavy chain (BY18.1H) of anti-PD1 antibody
BY18.1
<400>  76
Met Glu Thr Asp Thr Leu Leu Leu Trp Val Leu Leu Leu Trp Val Pro
1               5                   10                  15
Gly Ser Thr Gly Gln Val Gln Leu Val Glu Ser Gly Gly Gly Val Val
            20                  25                  30
Gln Pro Gly Arg Ser Leu Arg Leu Asp Cys Lys Ala Ser Gly Ile Thr
        35                  40                  45
Phe Ser Asn Ser Gly Met His Trp Val Arg Gln Ala Pro Gly Lys Gly
    50                  55                  60
Leu Glu Trp Val Ala Val Ile Trp Tyr Asp Gly Ser Lys Arg Tyr Tyr
65                  70                  75                  80
Ala Asp Ser Val Lys Gly Arg Phe Thr Ile Ser Arg Asp Asn Ser Lys
                85                  90                  95
Asn Thr Leu Phe Leu Gln Met Asn Ser Leu Arg Ala Glu Asp Thr Ala
            100                 105                 110
Val Tyr Tyr Cys Ala Thr Asn Asp Asp Tyr Trp Gly Gln Gly Thr Leu
        115                 120                 125
Val Thr Val Ser Ser Ala Ser Thr Lys Gly Pro Ser Val Phe Pro Leu
    130                 135                 140
Ala Pro Cys Ser Arg Ser Thr Ser Glu Ser Thr Ala Ala Leu Gly Cys
145                 150                 155                 160
Leu Val Lys Asp Tyr Phe Pro Glu Pro Val Thr Val Ser Trp Asn Ser
                165                 170                 175
Gly Ala Leu Thr Ser Gly Val His Thr Phe Pro Ala Val Leu Gln Ser
            180                 185                 190
Ser Gly Leu Tyr Ser Leu Ser Ser Val Val Thr Val Pro Ser Ser Ser
        195                 200                 205
Leu Gly Thr Lys Thr Tyr Thr Cys Asn Val Asp His Lys Pro Ser Asn
    210                 215                 220
Thr Lys Val Asp Lys Arg Val Glu Ser Lys Tyr Gly Pro Pro Cys Pro
225                 230                 235                 240

Pro Cys Pro Ala Pro Glu Phe Leu Gly Gly Pro Ser Val Phe Leu Phe
                245                 250                 255
Pro Pro Lys Pro Lys Asp Thr Leu Met Ile Ser Arg Thr Pro Glu Val
            260                 265                 270
Thr Cys Val Val Val Asp Val Ser Gln Glu Asp Pro Glu Val Gln Phe
        275                 280                 285
Asn Trp Tyr Val Asp Gly Val Glu Val His Asn Ala Lys Thr Lys Pro
    290                 295                 300
Arg Glu Glu Gln Phe Asn Ser Thr Tyr Arg Val Val Ser Val Leu Thr
305                 310                 315                 320
Val Leu His Gln Asp Trp Leu Asn Gly Lys Glu Tyr Lys Cys Lys Val
                325                 330                 335
Ser Asn Lys Gly Leu Pro Ser Ser Ile Glu Lys Thr Ile Ser Lys Ala
            340                 345                 350
Lys Gly Gln Pro Arg Glu Pro Gln Val Tyr Thr Leu Pro Pro Ser Gln
        355                 360                 365
Glu Glu Met Thr Lys Asn Gln Val Ser Leu Thr Cys Leu Val Lys Gly
    370                 375                 380
Phe Tyr Pro Ser Asp Ile Ala Val Glu Trp Glu Ser Asn Gly Gln Pro
385                 390                 395                 400
Glu Asn Asn Tyr Lys Thr Thr Pro Pro Val Leu Asp Ser Asp Gly Ser
                405                 410                 415
Phe Phe Leu Tyr Ser Arg Leu Thr Val Asp Lys Ser Arg Trp Gln Glu
            420                 425                 430
Gly Asn Val Phe Ser Cys Ser Val Met His Glu Ala Leu His Asn His
        435                 440                 445
Tyr Thr Gln Lys Ser Leu Ser Leu Ser Leu Gly Lys
    450                 455                 460
<210>  77
<211>  1113
<212>  DNA
<213>  Artificial sequence
<220>
<223>  A nucleotide sequence of fusion protein BY31.19 (CD80-Fc, IgG4)
<400>  77
ctcgaggcca ccatggagac cgacacactc ctcctgtggg tgctgctgct gtgggtgcct      60
ggctccactg gcgtcatcca tgtgaccaaa gaggtcaaag aagtcgcaac tctgtcttgc     120
ggacacaacg tctccgtcga agaactcgcc cagactagaa tatattggca gaaggaaaag     180
aagatggtgc tcaccatgat gtccggcgat atgaacattt ggcctgagta caagaaccgg     240
acaatcttcg atattactaa taacctgagt atcgtgattc tcgctctgcg ccctagcgac     300
gagggcacat acgagtgtgt ggtgctgaag tacgagaagg atgcattcaa gcgggagcac     360
ctcgcagagg tgacactctc cgtgaaggcc gacttcccaa ccccatctga caagcgcgtg     420
gagtccaagt acggccctcc ttgccctcct tgccctgccc ctgagttcct gggcggccct     480
tccgtgttcc tgttccctcc taagcctaag gacaccctga tgatctcccg cacccctgag     540
gtgacctgcg tggtggtgga cgtgtcccag gaggaccctg aggtgcagtt caactggtac     600
gtggacggcg tggaggtgca caacgccaag accaagcctc gcgaggagca gttcaactcc     660
acctaccgcg tggtgtccgt gctgaccgtg ctgcaccagg actggctgaa cggcaaggag     720
tacaagtgca aggtgtccaa caagggcctg ccttcctcca tcgagaagac catctccaag     780
gccaagggcc agcctcgcga gcctcaggtg tacaccctgc ctccttccca ggaggagatg     840
accaagaacc aggtgtccct gacctgcctg gtgaagggct tctacccttc cgacatcgcc     900
gtggagtggg agtccaacgg ccagcctgag aacaactaca agaccacccc tcctgtgctg     960
gactccgacg gctccttctt cctgtactcc cgcctgaccg tggacaagtc ccgctggcag    1020
gagggcaacg tgttctcctg ctccgtgatg cacgaggccc tgcacaacca ctacacccag    1080
aagtccctgt ccctgtccct gggctaagaa ttc                                 1113
<210>  78
<211>  364
<212>  PRT
<213>  Artificial sequence
<220>
<223>  An amino acid sequence of the fusion protein BY31.19 (CD80-Fc, IgG4)
<400>  78
Met Glu Thr Asp Thr Leu Leu Leu Trp Val Leu Leu Leu Trp Val Pro
1               5                   10                  15

Gly Ser Thr Gly Val Ile His Val Thr Lys Glu Val Lys Glu Val Ala
            20                  25                  30
Thr Leu Ser Cys Gly His Asn Val Ser Val Glu Glu Leu Ala Gln Thr
        35                  40                  45
Arg Ile Tyr Trp Gln Lys Glu Lys Lys Met Val Leu Thr Met Met Ser
    50                  55                  60
Gly Asp Met Asn Ile Trp Pro Glu Tyr Lys Asn Arg Thr Ile Phe Asp
65                  70                  75                  80
Ile Thr Asn Asn Leu Ser Ile Val Ile Leu Ala Leu Arg Pro Ser Asp
                85                  90                  95
Glu Gly Thr Tyr Glu Cys Val Val Leu Lys Tyr Glu Lys Asp Ala Phe
            100                 105                 110
Lys Arg Glu His Leu Ala Glu Val Thr Leu Ser Val Lys Ala Asp Phe
        115                 120                 125
Pro Thr Pro Ser Asp Lys Arg Val Glu Ser Lys Tyr Gly Pro Pro Cys
    130                 135                 140
Pro Pro Cys Pro Ala Pro Glu Phe Leu Gly Gly Pro Ser Val Phe Leu
145                 150                 155                 160
Phe Pro Pro Lys Pro Lys Asp Thr Leu Met Ile Ser Arg Thr Pro Glu
                165                 170                 175
Val Thr Cys Val Val Val Asp Val Ser Gln Glu Asp Pro Glu Val Gln
            180                 185                 190
Phe Asn Trp Tyr Val Asp Gly Val Glu Val His Asn Ala Lys Thr Lys
        195                 200                 205
Pro Arg Glu Glu Gln Phe Asn Ser Thr Tyr Arg Val Val Ser Val Leu
    210                 215                 220
Thr Val Leu His Gln Asp Trp Leu Asn Gly Lys Glu Tyr Lys Cys Lys
225                 230                 235                 240
Val Ser Asn Lys Gly Leu Pro Ser Ser Ile Glu Lys Thr Ile Ser Lys
                245                 250                 255
Ala Lys Gly Gln Pro Arg Glu Pro Gln Val Tyr Thr Leu Pro Pro Ser
            260                 265                 270
Gln Glu Glu Met Thr Lys Asn Gln Val Ser Leu Thr Cys Leu Val Lys
        275                 280                 285
Gly Phe Tyr Pro Ser Asp Ile Ala Val Glu Trp Glu Ser Asn Gly Gln
    290                 295                 300
Pro Glu Asn Asn Tyr Lys Thr Thr Pro Pro Val Leu Asp Ser Asp Gly
305                 310                 315                 320
Ser Phe Phe Leu Tyr Ser Arg Leu Thr Val Asp Lys Ser Arg Trp Gln
                325                 330                 335
Glu Gly Asn Val Phe Ser Cys Ser Val Met His Glu Ala Leu His Asn
            340                 345                 350
His Tyr Thr Gln Lys Ser Leu Ser Leu Ser Leu Gly
        355                 360
<210> 79
<211> 723
<212> DNA
<213> Artificial sequence
<220>
<223> A nucleotide sequence of the first subunit (BY24.4L) of the fusion
protein BY24.4 (??, IgG4)
<400> 79
ctcgaggcca ccatggagac agatactctg ctgctctggg tcctgctgct gtgggtgcca 60
ggcagcacag gcgacatcca gatgacccag tccccttcct ccctgtctgc ctccgtgggc 120
gacagggtga ccatcacctg ttccgcatcc caggatatct ccaactacct gaactggtat 180
cagcagaagc caggaaaggc ccccaaggtg ctgatctact ttacttcttc cctgcattcc 240
ggcgtgccct ccaggttctc tggctccggg tctggaaccg actttacact gacaatctcc 300
tctctgcagc cagaagactt tgccacctat tactgccagc agtactccac tgtcccctgg 360
accttcggcc aaggaaccaa agtggaaatc aagaggaccg tggccgcccc cagcgtgttc 420
atcttccctc ccagcgacga gcagctgaag tctggcaccg ccagcgtggt gtgcctgctg 480
aacaacttct acccccgcga ggccaaggtg cagtggaagg tggacaacgc cctgcagagc 540
ggcaacagcc aggagagcgt gaccgagcag gactccaagg acagcaccta cagcctgagc 600
agcaccctga ccctgagcaa ggccgactac gagaagcaca aggtgtacgc ctgcgaggtg 660

acccaccagg gactgtctag ccccgtgacc aagagcttca accggggcga gtgctaagaa 720
ttc 723
<210> 80
<211> 234
<212> PRT
<213> Artificial sequence
<220>
<223> An amino acid sequence of the first subunit (BY24.4L) of the fusion
protein BY24.4 (??, IgG4)
<400> 80
Met Glu Thr Asp Thr Leu Leu Leu Trp Val Leu Leu Leu Trp Val Pro
1               5                   10                  15
Gly Ser Thr Gly Asp Ile Gln Met Thr Gln Ser Pro Ser Ser Leu Ser
            20                  25                  30
Ala Ser Val Gly Asp Arg Val Thr Ile Thr Cys Ser Ala Ser Gln Asp
        35                  40                  45
Ile Ser Asn Tyr Leu Asn Trp Tyr Gln Gln Lys Pro Gly Lys Ala Pro
    50                  55                  60
Lys Val Leu Ile Tyr Phe Thr Ser Ser Leu His Ser Gly Val Pro Ser
65                  70                  75                  80
Arg Phe Ser Gly Ser Gly Ser Gly Thr Asp Phe Thr Leu Thr Ile Ser
                85                  90                  95
Ser Leu Gln Pro Glu Asp Phe Ala Thr Tyr Tyr Cys Gln Gln Tyr Ser
            100                 105                 110
Thr Val Pro Trp Thr Phe Gly Gln Gly Thr Lys Val Glu Ile Lys Arg
        115                 120                 125
Thr Val Ala Ala Pro Ser Val Phe Ile Phe Pro Pro Ser Asp Glu Gln
    130                 135                 140
Leu Lys Ser Gly Thr Ala Ser Val Val Cys Leu Leu Asn Asn Phe Tyr
145                 150                 155                 160
Pro Arg Glu Ala Lys Val Gln Trp Lys Val Asp Asn Ala Leu Gln Ser
                165                 170                 175
Gly Asn Ser Gln Glu Ser Val Thr Glu Gln Asp Ser Lys Asp Ser Thr
            180                 185                 190
Tyr Ser Leu Ser Ser Thr Leu Thr Leu Ser Lys Ala Asp Tyr Glu Lys
        195                 200                 205
His Lys Val Tyr Ala Cys Glu Val Thr His Gln Gly Leu Ser Ser Pro
    210                 215                 220
Val Thr Lys Ser Phe Asn Arg Gly Glu Cys
225                 230
<210> 81
<211> 1806
<212> DNA
<213> Artificial sequence
<220>
<223> A nucleotide sequence of the second subunit (BY24.4H) of the fusion
protein BY24.4 (??, IgG4)
<400> 81
tctagagcca ccatggaaac cgacacactg ctgctgtggg tcctgctgct gtgggtgcca 60
ggctctacag gcgaagtgca gctggtcgaa tctggcggcg gactggtgca accagggggc 120
agcctgaggc tgtcctgcgc cgcttctgga tacaccttta ccaattacgg catgaattgg 180
gtgaggcagg ctcctggcaa gggcctggag tgggtgggat ggatcaacac ctacaccggc 240
gagcccacct acgccgctga ctttaagagg aggttcacct tctctctgga cacctccaag 300
tccaccgctt acctgcaaat gaactctctg agggctgaag acaccgccgt gtactactgc 360
gccaagtacc cccattacta cgggtcctcc cactggtact tcgacgtgtg gggccagggc 420
accctcgtga cagtgtcttc cgcctccacc aagggccctt ccgtgttccc tctggcccct 480
tgctcccgct ccacctccga gtccaccgcc gccctgggct gcctggtgaa ggactacttc 540
cctgagcctg tgaccgtgtc ctggaactcc ggcgccctga cctccggcgt gcacaccttc 600
cctgccgtgc tgcagtcctc cggcctgtac tccctgtcct ccgtggtgac cgtgccttcc 660
tcctccctgg gcaccaagac ctacacctgc aacgtggacc acaagccttc caacaccaag 720
gtggacaagc gcgtggagtc caagtacggc cctccttgcc ctccttgccc tgcccctgag 780
ttcctgggcg gcccttccgt gttcctgttc cctcctaagc ctaaggacac cctgatgatc 840
tcccgcaccc ctgaggtgac ctgcgtggtg gtggacgtgt cccaggagga ccctgaggtg 900

cagttcaact ggtacgtgga cggcgtggag gtgcacaacg ccaagaccaa gcctcgcgag 960
gagcagttca actccaccta ccgcgtggtg tccgtgctga ccgtgctgca ccaggactgg 1020
ctgaacggca aggagtacaa gtgcaaggtg tccaacaagg gcctgccttc ctccatcgag 1080
aagaccatct ccaaggccaa gggccagcct cgcgagcctc aggtgtacac cctgcctcct 1140
tcccaggagg agatgaccaa gaaccaggtg tccctgacct gcctggtgaa gggcttctac 1200
ccttccgaca tcgccgtgga gtgggagtcc aacggccagc ctgagaacaa ctacaagacc 1260
acccctcctg tgctggactc cgacggctcc ttcttcctgt actcccgcct gaccgtggac 1320
aagtcccgct ggcaggaggg caacgtgttc tcctgctccg tgatgcacga ggccctgcac 1380
aaccactaca cccagaagtc cctgtccctg tccctgggcg gcggaggatc tggcggcgga 1440
ggcagtggag gcggcggaag tgtgatccat gtaacaaaag aagtgaagga agtggctaca 1500
ctctcttgcg gccacaacgt gtccgtggag gaactagctc agacccggat ctattggcag 1560
aaagaaaaga agatggtgct gaccatgatg tccggcgaca tgaacatttg gccagagtac 1620
aagaaccgca caattttcga cattacaaac aacctctcta ttgtgattct ggctctcagg 1680
cctagcgacg agggcacata cgagtgcgtg gtgctcaagt acgagaagga cgctttcaag 1740
cgggagcacc tcgctgaggt gaccctgtcc gtgaaggccg acttccctac tccatcttaa 1800
gtcgac 1806
<210> 82
<211> 595
<212> PRT
<213> Artificial sequence
<220>
<223> An amino acid sequence of the second subunit (BY24.4H) of the fusion
protein BY24.4 (??, IgG4)
<400> 82
Met Glu Thr Asp Thr Leu Leu Leu Trp Val Leu Leu Leu Trp Val Pro
1               5                   10                  15
Gly Ser Thr Gly Glu Val Gln Leu Val Glu Ser Gly Gly Gly Leu Val
            20                  25                  30
Gln Pro Gly Gly Ser Leu Arg Leu Ser Cys Ala Ala Ser Gly Tyr Thr
        35                  40                  45
Phe Thr Asn Tyr Gly Met Asn Trp Val Arg Gln Ala Pro Gly Lys Gly
    50                  55                  60
Leu Glu Trp Val Gly Trp Ile Asn Thr Tyr Thr Gly Glu Pro Thr Tyr
65                  70                  75                  80
Ala Ala Asp Phe Lys Arg Arg Phe Thr Phe Ser Leu Asp Thr Ser Lys
                85                  90                  95
Ser Thr Ala Tyr Leu Gln Met Asn Ser Leu Arg Ala Glu Asp Thr Ala
            100                 105                 110
Val Tyr Tyr Cys Ala Lys Tyr Pro His Tyr Tyr Gly Ser Ser His Trp
        115                 120                 125
Tyr Phe Asp Val Trp Gly Gln Gly Thr Leu Val Thr Val Ser Ser Ala
    130                 135                 140
Ser Thr Lys Gly Pro Ser Val Phe Pro Leu Ala Pro Cys Ser Arg Ser
145                 150                 155                 160
Thr Ser Glu Ser Thr Ala Ala Leu Gly Cys Leu Val Lys Asp Tyr Phe
                165                 170                 175
Pro Glu Pro Val Thr Val Ser Trp Asn Ser Gly Ala Leu Thr Ser Gly
            180                 185                 190
Val His Thr Phe Pro Ala Val Leu Gln Ser Ser Gly Leu Tyr Ser Leu
        195                 200                 205
Ser Ser Val Val Thr Val Pro Ser Ser Ser Leu Gly Thr Lys Thr Tyr
    210                 215                 220
Thr Cys Asn Val Asp His Lys Pro Ser Asn Thr Lys Val Asp Lys Arg
225                 230                 235                 240
Val Glu Ser Lys Tyr Gly Pro Pro Cys Pro Pro Cys Pro Ala Pro Glu
                245                 250                 255
Phe Leu Gly Gly Pro Ser Val Phe Leu Phe Pro Pro Lys Pro Lys Asp
            260                 265                 270
Thr Leu Met Ile Ser Arg Thr Pro Glu Val Thr Cys Val Val Val Asp
        275                 280                 285
Val Ser Gln Glu Asp Pro Glu Val Gln Phe Asn Trp Tyr Val Asp Gly
    290                 295                 300
Val Glu Val His Asn Ala Lys Thr Lys Pro Arg Glu Glu Gln Phe Asn

305                 310                 315                 320
Ser Thr Tyr Arg Val Val Ser Val Leu Thr Val Leu His Gln Asp Trp
                325                 330                 335
Leu Asn Gly Lys Glu Tyr Lys Cys Lys Val Ser Asn Lys Gly Leu Pro
            340                 345                 350
Ser Ser Ile Glu Lys Thr Ile Ser Lys Ala Lys Gly Gln Pro Arg Glu
        355                 360                 365
Pro Gln Val Tyr Thr Leu Pro Pro Ser Gln Glu Glu Met Thr Lys Asn
    370                 375                 380
Gln Val Ser Leu Thr Cys Leu Val Lys Gly Phe Tyr Pro Ser Asp Ile
385                 390                 395                 400
Ala Val Glu Trp Glu Ser Asn Gly Gln Pro Glu Asn Asn Tyr Lys Thr
                405                 410                 415
Thr Pro Pro Val Leu Asp Ser Asp Gly Ser Phe Phe Leu Tyr Ser Arg
            420                 425                 430
Leu Thr Val Asp Lys Ser Arg Trp Gln Glu Gly Asn Val Phe Ser Cys
        435                 440                 445
Ser Val Met His Glu Ala Leu His Asn His Tyr Thr Gln Lys Ser Leu
    450                 455                 460
Ser Leu Ser Leu Gly Gly Gly Gly Ser Gly Gly Gly Gly Ser Gly Gly
465                 470                 475                 480
Gly Gly Ser Val Ile His Val Thr Lys Glu Val Lys Glu Val Ala Thr
                485                 490                 495
Leu Ser Cys Gly His Asn Val Ser Val Glu Glu Leu Ala Gln Thr Arg
            500                 505                 510
Ile Tyr Trp Gln Lys Glu Lys Lys Met Val Leu Thr Met Met Ser Gly
        515                 520                 525
Asp Met Asn Ile Trp Pro Glu Tyr Lys Asn Arg Thr Ile Phe Asp Ile
    530                 535                 540
Thr Asn Asn Leu Ser Ile Val Ile Leu Ala Leu Arg Pro Ser Asp Glu
545                 550                 555                 560
Gly Thr Tyr Glu Cys Val Val Leu Lys Tyr Glu Lys Asp Ala Phe Lys
                565                 570                 575
Arg Glu His Leu Ala Glu Val Thr Leu Ser Val Lys Ala Asp Phe Pro
            580                 585                 590
Thr Pro Ser
        595
<210>  83
<211>  723
<212>  DNA
<213>  Artificial sequence
<220>
<223>  A nucleotide sequence of the first subunit (BY24.5L) of the fusion
protein BY24.5 (??, IgG4)
<400>  83
ctcgaggcca ccatggagac agatactctg ctgctctggg tcctgctgct gtgggtgcca      60
ggcagcacag gcgacatcca gatgacccag tctcctagct ccgtgagcgc ctctatcggc     120
gacagagtga ccatcacatg cagagcttct cagggcatcg acaactggct cggctggtac     180
cagcagaagc ctggcaaggc ccccaagctg ctgatctacg acgcctctaa cctggacacc     240
ggcgtgccat cccggttcag cggctctggc tccggcacat acttcacact cacaatctct     300
tccctgcagg ccgaggactt cgctgtgtac ttctgccagc aggctaaggc tttcccacct     360
acattcggcg gcggaaccaa agtggaaatc aagaggaccg tggccgcccc cagcgtgttc     420
atcttccctc ccagcgacga gcagctgaag tctggcaccg ccagcgtggt gtgcctgctg     480
aacaacttct acccccgcga ggccaaggtg cagtggaagg tggacaacgc cctgcagagc     540
ggcaacagcc aggagagcgt gaccgagcag gactccaagg acagcaccta cagcctgagc     600
agcaccctga ccctgagcaa ggccgactac gagaagcaca aggtgtacgc ctgcgaggtg     660
acccaccagg gactgtctag ccccgtgacc aagagcttca accggggcga gtgctaagaa     720
ttc                                                                   723
<210>  84
<211>  234
<212>  PRT
<213>  Artificial sequence
<220>

<223>  An amino acid sequence of the first subunit (BY24.5L) of the fusion
protein BY24.5 (??, IgG4)
<400>  84
Met Glu Thr Asp Thr Leu Leu Leu Trp Val Leu Leu Leu Trp Val Pro
1               5                   10                  15
Gly Ser Thr Gly Asp Ile Gln Met Thr Gln Ser Pro Ser Ser Val Ser
            20                  25                  30
Ala Ser Ile Gly Asp Arg Val Thr Ile Thr Cys Arg Ala Ser Gln Gly
        35                  40                  45
Ile Asp Asn Trp Leu Gly Trp Tyr Gln Gln Lys Pro Gly Lys Ala Pro
    50                  55                  60
Lys Leu Leu Ile Tyr Asp Ala Ser Asn Leu Asp Thr Gly Val Pro Ser
65                  70                  75                  80
Arg Phe Ser Gly Ser Gly Ser Gly Thr Tyr Phe Thr Leu Thr Ile Ser
                85                  90                  95
Ser Leu Gln Ala Glu Asp Phe Ala Val Tyr Phe Cys Gln Gln Ala Lys
            100                 105                 110
Ala Phe Pro Pro Thr Phe Gly Gly Gly Thr Lys Val Glu Ile Lys Arg
        115                 120                 125
Thr Val Ala Ala Pro Ser Val Phe Ile Phe Pro Pro Ser Asp Glu Gln
    130                 135                 140
Leu Lys Ser Gly Thr Ala Ser Val Val Cys Leu Leu Asn Asn Phe Tyr
145                 150                 155                 160
Pro Arg Glu Ala Lys Val Gln Trp Lys Val Asp Asn Ala Leu Gln Ser
                165                 170                 175
Gly Asn Ser Gln Glu Ser Val Thr Glu Gln Asp Ser Lys Asp Ser Thr
            180                 185                 190
Tyr Ser Leu Ser Ser Thr Leu Thr Leu Ser Lys Ala Asp Tyr Glu Lys
        195                 200                 205
His Lys Val Tyr Ala Cys Glu Val Thr His Gln Gly Leu Ser Ser Pro
    210                 215                 220
Val Thr Lys Ser Phe Asn Arg Gly Glu Cys
225                 230
<210>  85
<211>  1785
<212>  DNA
<213>  Artificial sequence
<220>
<223>  A nucleotide sequence of the second subunit (BY24.5H) of the fusion
protein BY24.5 (??, IgG4)
<400>  85
tctagagcca ccatggaaac cgacacactg ctgctgtggg tcctgctgct gtgggtgcca      60
ggctctacag gcgaggtgca gctcgtgcag tctggcggcg gcctcgtgaa gccaggcggc     120
tccctcagac tgtcttgcgc tgctagcggc ttcacattct cttcctactc tatgaactgg     180
gtgagacagg ctcccggcaa gggcctggag tgggtgtcca gcatctcttc ctctagctct     240
tacatctact acgccgactc cgtgaagggc agattcacaa tcagccggga caacgccaag     300
aactccctgt acctgcagat gaacagcctg agagccgagg acaccgctgt gtactactgc     360
gctagagtga ccgacgcttt cgacatctgg ggccagggca ctctcgtgac agtgtcttcc     420
gcctccacca agggcccttc cgtgttccct ctggcccctt gctcccgctc cacctccgag     480
tccaccgccg ccctgggctg cctggtgaag gactacttcc ctgagcctgt gaccgtgtcc     540
tggaactccg gcgccctgac ctccggcgtg cacaccttcc ctgccgtgct gcagtcctcc     600
ggcctgtact ccctgtcctc cgtggtgacc gtgccttcct cctccctggg caccaagacc     660
tacacctgca acgtggacca caagccttcc aacaccaagg tggacaagcg cgtggagtcc     720
aagtacggcc ctccttgccc tccttgccct gcccctgagt tcctgggcgg cccttccgtg     780
ttcctgttcc ctcctaagcc taaggacacc ctgatgatct cccgcacccc tgaggtgacc     840
tgcgtggtgg tggacgtgtc ccaggaggac cctgaggtgc agttcaactg gtacgtggac     900
ggcgtggagg tgcacaacgc caagaccaag cctcgcgagg agcagttcaa ctccacctac     960
cgcgtggtgt ccgtgctgac cgtgctgcac caggactggc tgaacggcaa ggagtacaag    1020
tgcaaggtgt ccaacaaggg cctgccttcc tccatcgaga agaccatctc caaggccaag    1080
ggccagcctc gcgagcctca ggtgtacacc ctgcctcctt cccaggagga gatgaccaag    1140
aaccaggtgt ccctgacctg cctggtgaag ggcttctacc cttccgacat cgccgtggag    1200
tgggagtcca acggccagcc tgagaacaac tacaagacca cccctcctgt gctggactcc    1260
gacggctcct tcttcctgta ctcccgcctg accgtggaca agtcccgctg gcaggagggc    1320

aacgtgttct cctgctccgt gatgcacgag gccctgcaca accactacac ccagaagtcc 1380
ctgtccctgt ccctgggcgg cggaggatct ggcggcggag gcagtggagg cggcggaagt 1440
gtgatccatg taacaaaaga agtgaaggaa gtggctacac tctcttgcgg ccacaacgtg 1500
tccgtggagg aactagctca gacccggatc tattggcaga aagaaaagaa gatggtgctg 1560
accatgatgt ccggcgacat gaacatttgg ccagagtaca agaaccgcac aattttcgac 1620
attacaaaca acctctctat tgtgattctg gctctcaggc ctagcgacga gggcacatac 1680
gagtgcgtgg tgctcaagta cgagaaggac gctttcaagc gggagcacct cgctgaggtg 1740
accctgtccg tgaaggccga cttccctact ccatcttaag tcgac 1785
<210> 86
<211> 588
<212> PRT
<213> Artificial sequence
<220>
<223> An amino acid sequence of the second subunit (BY24.5H) of the fusion
protein BY24.5 (??, IgG4)
<400> 86
Met Glu Thr Asp Thr Leu Leu Leu Trp Val Leu Leu Leu Trp Val Pro
1               5                   10                  15
Gly Ser Thr Gly Glu Val Gln Leu Val Gln Ser Gly Gly Gly Leu Val
            20                  25                  30
Lys Pro Gly Gly Ser Leu Arg Leu Ser Cys Ala Ala Ser Gly Phe Thr
        35                  40                  45
Phe Ser Ser Tyr Ser Met Asn Trp Val Arg Gln Ala Pro Gly Lys Gly
    50                  55                  60
Leu Glu Trp Val Ser Ser Ile Ser Ser Ser Ser Ser Tyr Ile Tyr Tyr
65                  70                  75                  80
Ala Asp Ser Val Lys Gly Arg Phe Thr Ile Ser Arg Asp Asn Ala Lys
                85                  90                  95
Asn Ser Leu Tyr Leu Gln Met Asn Ser Leu Arg Ala Glu Asp Thr Ala
            100                 105                 110
Val Tyr Tyr Cys Ala Arg Val Thr Asp Ala Phe Asp Ile Trp Gly Gln
        115                 120                 125
Gly Thr Leu Val Thr Val Ser Ser Ala Ser Thr Lys Gly Pro Ser Val
    130                 135                 140
Phe Pro Leu Ala Pro Cys Ser Arg Ser Thr Ser Glu Ser Thr Ala Ala
145                 150                 155                 160
Leu Gly Cys Leu Val Lys Asp Tyr Phe Pro Glu Pro Val Thr Val Ser
                165                 170                 175
Trp Asn Ser Gly Ala Leu Thr Ser Gly Val His Thr Phe Pro Ala Val
            180                 185                 190
Leu Gln Ser Ser Gly Leu Tyr Ser Leu Ser Ser Val Val Thr Val Pro
        195                 200                 205
Ser Ser Ser Leu Gly Thr Lys Thr Tyr Thr Cys Asn Val Asp His Lys
    210                 215                 220
Pro Ser Asn Thr Lys Val Asp Lys Arg Val Glu Ser Lys Tyr Gly Pro
225                 230                 235                 240
Pro Cys Pro Pro Cys Pro Ala Pro Glu Phe Leu Gly Gly Pro Ser Val
                245                 250                 255
Phe Leu Phe Pro Pro Lys Pro Lys Asp Thr Leu Met Ile Ser Arg Thr
            260                 265                 270
Pro Glu Val Thr Cys Val Val Val Asp Val Ser Gln Glu Asp Pro Glu
        275                 280                 285
Val Gln Phe Asn Trp Tyr Val Asp Gly Val Glu Val His Asn Ala Lys
    290                 295                 300
Thr Lys Pro Arg Glu Glu Gln Phe Asn Ser Thr Tyr Arg Val Val Ser
305                 310                 315                 320
Val Leu Thr Val Leu His Gln Asp Trp Leu Asn Gly Lys Glu Tyr Lys
                325                 330                 335
Cys Lys Val Ser Asn Lys Gly Leu Pro Ser Ser Ile Glu Lys Thr Ile
            340                 345                 350
Ser Lys Ala Lys Gly Gln Pro Arg Glu Pro Gln Val Tyr Thr Leu Pro
        355                 360                 365
Pro Ser Gln Glu Glu Met Thr Lys Asn Gln Val Ser Leu Thr Cys Leu

370                 375                 380
Val Lys Gly Phe Tyr Pro Ser Asp Ile Ala Val Glu Trp Glu Ser Asn
385                 390                 395                 400
Gly Gln Pro Glu Asn Asn Tyr Lys Thr Thr Pro Pro Val Leu Asp Ser
                405                 410                 415
Asp Gly Ser Phe Phe Leu Tyr Ser Arg Leu Thr Val Asp Lys Ser Arg
            420                 425                 430
Trp Gln Glu Gly Asn Val Phe Ser Cys Ser Val Met His Glu Ala Leu
        435                 440                 445
His Asn His Tyr Thr Gln Lys Ser Leu Ser Leu Ser Leu Gly Gly Gly
    450                 455                 460
Gly Ser Gly Gly Gly Gly Ser Gly Gly Gly Gly Ser Val Ile His Val
465                 470                 475                 480
Thr Lys Glu Val Lys Glu Val Ala Thr Leu Ser Cys Gly His Asn Val
                485                 490                 495
Ser Val Glu Glu Leu Ala Gln Thr Arg Ile Tyr Trp Gln Lys Glu Lys
            500                 505                 510
Lys Met Val Leu Thr Met Met Ser Gly Asp Met Asn Ile Trp Pro Glu
        515                 520                 525
Tyr Lys Asn Arg Thr Ile Phe Asp Ile Thr Asn Asn Leu Ser Ile Val
    530                 535                 540
Ile Leu Ala Leu Arg Pro Ser Asp Glu Gly Thr Tyr Glu Cys Val Val
545                 550                 555                 560
Leu Lys Tyr Glu Lys Asp Ala Phe Lys Arg Glu His Leu Ala Glu Val
                565                 570                 575
Thr Leu Ser Val Lys Ala Asp Phe Pro Thr Pro Ser
            580                 585
<210>  87
<211>  1776
<212>  DNA
<213>  Artificial sequence
<220>
<223>  A nucleotide sequence of the fusion protein BY24.12 (CD80-Fc-VEGFR)
<400>  87
ctcgaggcca ccgccaccat ggagaccgac acactcctcc tgtgggtgct gctgctgtgg      60
gtgcctggct ccactggcgt catccatgtg accaaagagg tcaaagaagt cgcaactctg     120
tcttgcggac acaacgtctc cgtcgaagaa ctcgcccaga ctagaatata ttggcagaag     180
gaaaagaaga tggtgctcac catgatgtcc ggcgatatga acatttggcc tgagtacaag     240
aaccggacaa tcttcgatat tactaataac ctgagtatcg tgattctcgc tctgcgccct     300
agcgacgagg gcacatacga gtgtgtggtg ctgaagtacg agaaggatgc attcaagcgg     360
gagcacctcg cagaggtgac actctccgtg aaggccgact tcccaacccc atctgacaag     420
cgcgtggagt ccaagtacgg ccctccttgc cctccttgcc ctgcccctga gttcctgggc     480
ggcccttccg tgttcctgtt ccctcctaag cctaaggaca ccctgatgat ctcccgcacc     540
cctgaggtga cctgcgtggt ggtggacgtg tcccaggagg accctgaggt gcagttcaac     600
tggtacgtgg acggcgtgga ggtgcacaac gccaagacca agcctcgcga ggagcagttc     660
aactccacct accgcgtggt gtccgtgctg accgtgctgc accaggactg gctgaacggc     720
aaggagtaca agtgcaaggt gtccaacaag ggcctgcctt cctccatcga gaagaccatc     780
tccaaggcca agggccagcc tcgcgagcct caggtgtaca ccctgcctcc ttcccaggag     840
gagatgacca agaaccaggt gtccctgacc tgcctggtga agggcttcta cccttccgac     900
atcgccgtgg agtgggagtc caacggccag cctgagaaca actacaagac cacccctcct     960
gtgctggact ccgacggctc cttcttcctg tactcccgcc tgaccgtgga caagtcccgc    1020
tggcaggagg gcaacgtgtt ctcctgctcc gtgatgcacg aggccctgca caaccactac    1080
acccagaagt ccctgtccct gtccctgggc ggcggaggat ctggcggcgg aggcagtgga    1140
ggcggcggaa gtgcttccga caccggccgc cctttcgtgg agatgtactc cgagatccct    1200
gagatcatcc acatgaccga gggccgcgag ctggtgatcc cttgccgcgt gacctcccct    1260
aacatcaccg tgaccctgaa gaagttccct ctggacaccc tgatccctga cggcaagcgc    1320
atcatctggg actcccgcaa gggcttcatc atctccaacg ccacctacaa ggagatcggc    1380
ctgctgacct gcgaggccac cgtgaacggc cacctgtaca agaccaacta cctgacccac    1440
cgccagacca acaccatcat cgacgtggtg ctgtcccctt cccacggcat cgagctgtcc    1500
gtgggcgaga agctggtgct gaactgcacc gcccgcaccg agctgaacgt gggcatcgac    1560
ttcaactggg agtacccttc ctccaagcac cagcacaaga agctggtgaa ccgcgacctg    1620
aagacccagt ccggctccga gatgaagaag ttcctgtcca ccctgaccat cgacggcgtg    1680
acccgctccg accagggcct gtacacctgc gccgcctcct ccggcctgat gaccaagaag    1740

aactccacct tcgtgcgcgt gcacgagtaa gaattc                                1776
<210>  88
<211>  583
<212>  PRT
<213>  Artificial sequence
<220>
<223>  An amino acid sequence of the fusion protein BY24.12 (CD80-Fc-VEGFR)
<400>  88
Met Glu Thr Asp Thr Leu Leu Leu Trp Val Leu Leu Leu Trp Val Pro
1               5                   10                  15
Gly Ser Thr Gly Val Ile His Val Thr Lys Glu Val Lys Glu Val Ala
            20                  25                  30
Thr Leu Ser Cys Gly His Asn Val Ser Val Glu Glu Leu Ala Gln Thr
        35                  40                  45
Arg Ile Tyr Trp Gln Lys Glu Lys Lys Met Val Leu Thr Met Met Ser
    50                  55                  60
Gly Asp Met Asn Ile Trp Pro Glu Tyr Lys Asn Arg Thr Ile Phe Asp
65                  70                  75                  80
Ile Thr Asn Asn Leu Ser Ile Val Ile Leu Ala Leu Arg Pro Ser Asp
                85                  90                  95
Glu Gly Thr Tyr Glu Cys Val Val Leu Lys Tyr Glu Lys Asp Ala Phe
            100                 105                 110
Lys Arg Glu His Leu Ala Glu Val Thr Leu Ser Val Lys Ala Asp Phe
        115                 120                 125
Pro Thr Pro Ser Asp Lys Arg Val Glu Ser Lys Tyr Gly Pro Pro Cys
    130                 135                 140
Pro Pro Cys Pro Ala Pro Glu Phe Leu Gly Gly Pro Ser Val Phe Leu
145                 150                 155                 160
Phe Pro Pro Lys Pro Lys Asp Thr Leu Met Ile Ser Arg Thr Pro Glu
                165                 170                 175
Val Thr Cys Val Val Val Asp Val Ser Gln Glu Asp Pro Glu Val Gln
            180                 185                 190
Phe Asn Trp Tyr Val Asp Gly Val Glu Val His Asn Ala Lys Thr Lys
        195                 200                 205
Pro Arg Glu Glu Gln Phe Asn Ser Thr Tyr Arg Val Val Ser Val Leu
    210                 215                 220
Thr Val Leu His Gln Asp Trp Leu Asn Gly Lys Glu Tyr Lys Cys Lys
225                 230                 235                 240
Val Ser Asn Lys Gly Leu Pro Ser Ser Ile Glu Lys Thr Ile Ser Lys
                245                 250                 255
Ala Lys Gly Gln Pro Arg Glu Pro Gln Val Tyr Thr Leu Pro Pro Ser
            260                 265                 270
Gln Glu Glu Met Thr Lys Asn Gln Val Ser Leu Thr Cys Leu Val Lys
        275                 280                 285
Gly Phe Tyr Pro Ser Asp Ile Ala Val Glu Trp Glu Ser Asn Gly Gln
    290                 295                 300
Pro Glu Asn Asn Tyr Lys Thr Thr Pro Pro Val Leu Asp Ser Asp Gly
305                 310                 315                 320
Ser Phe Phe Leu Tyr Ser Arg Leu Thr Val Asp Lys Ser Arg Trp Gln
                325                 330                 335
Glu Gly Asn Val Phe Ser Cys Ser Val Met His Glu Ala Leu His Asn
            340                 345                 350
His Tyr Thr Gln Lys Ser Leu Ser Leu Ser Leu Gly Gly Gly Gly Ser
        355                 360                 365
Gly Gly Gly Gly Ser Gly Gly Gly Gly Ser Ala Ser Asp Thr Gly Arg
    370                 375                 380
Pro Phe Val Glu Met Tyr Ser Glu Ile Pro Glu Ile Ile His Met Thr
385                 390                 395                 400
Glu Gly Arg Glu Leu Val Ile Pro Cys Arg Val Thr Ser Pro Asn Ile
                405                 410                 415
Thr Val Thr Leu Lys Lys Phe Pro Leu Asp Thr Leu Ile Pro Asp Gly
            420                 425                 430
Lys Arg Ile Ile Trp Asp Ser Arg Lys Gly Phe Ile Ile Ser Asn Ala

        435                 440                 445
Thr Tyr Lys Glu Ile Gly Leu Leu Thr Cys Glu Ala Thr Val Asn Gly
    450                 455                 460
His Leu Tyr Lys Thr Asn Tyr Leu Thr His Arg Gln Thr Asn Thr Ile
465                 470                 475                 480
Ile Asp Val Val Leu Ser Pro Ser His Gly Ile Glu Leu Ser Val Gly
                485                 490                 495
Glu Lys Leu Val Leu Asn Cys Thr Ala Arg Thr Glu Leu Asn Val Gly
            500                 505                 510
Ile Asp Phe Asn Trp Glu Tyr Pro Ser Ser Lys His Gln His Lys Lys
        515                 520                 525
Leu Val Asn Arg Asp Leu Lys Thr Gln Ser Gly Ser Glu Met Lys Lys
    530                 535                 540
Phe Leu Ser Thr Leu Thr Ile Asp Gly Val Thr Arg Ser Asp Gln Gly
545                 550                 555                 560
Leu Tyr Thr Cys Ala Ala Ser Ser Gly Leu Met Thr Lys Lys Asn Ser
                565                 570                 575
Thr Phe Val Arg Val His Glu
            580

## Description

### FIELD OF TECHNOLOGY

The present invention generally relates to the field of medical biotechnology. Specifically, the present invention relates to a multi-targeting fusion protein comprising (i) a vascular endothelial cell growth inhibitor domain; (ii) an immunoglobulin Fc domain; and (iii) a multi-targeting fusion protein of CD80 extracellular domain (ECD), a polynucleotide encoding the multi-targeting fusion protein, a vector containing the polynucleotide, a host cell comprising the polynucleotide or the vector, and pharmaceutical composition comprising the multi-targeting fusion protein and an anti-PD-1 antibody. The multi-targeting fusion protein and pharmaceutical composition of the present invention can treat or prevent cancerous diseases in individuals.

### BACKGROUND

Tumor microenvironment is a complex environment for tumor cells to survive and develop and it is composed of cellular components and non-cellular components, wherein cellular components include tumor cells, immune cells, endothelial cells, etc.; non-cellular components include cytokines, chemokines, and the like. With the researchon tumors, people have realizedthat the generation, growth and metastasis of tumors are regulated by the tumor microenvironment. The tumor microenvironment determines whether tumor cells can grow dominantly

Inhibition of T lymphocyte activity usually exists in the tumormicroenvironment, resulting in the T lymphocytes unable to effectively exert a killing effect on tumors (Yao S, Zhu Y and Chen L., Advances in targeting cell surface signaling molecules for immune modulation., Nat Rev Drug Discov, 2013, 12(2): 130-146), for example, tumor cells inhibit the activity of T lymphocytes by expressing PD-L1, thereby using the inhibitory signaling pathway of immune checkpoints (ie, PD-1/PD-L1 inhibitory signal pathway), wherein the PD-L1 is not expressed in normal human tissues.

It was previously thought that inhibition of T lymphocyte activation is achieved by inhibition of the T cell receptor (TCR)/major histocompatibility complex (MHC) after binding of PD-1 to its ligand PD-L1, but recent studies by Hui et al.have shown that PD-1 and CD 28 co-localize to the T lymphocyte membrane, targets of PD-1 mediated immunosuppression are mainly CD 28 rather than TCR. Specifically, Shp2 phospholipase is rapidly recruited after PD-1 binds to PD-L1 and Shp2 phospholipase preferentially dephosphorylates CD28, which is stronger than dephosphorylation of TCR, thereby inhibiting T cell function by inactivating the signal transduction of CD28. (Hui E.https://www.ncbi.nlm.nih.gov/pubmed/?term=Hui%20E%5bAuthor%5d&cauthor=true&cauthor_uid=2828024 7et al., T cell costimulatory receptor CD28 is a primary target for PD-1-mediated inhibition, Science,https://www.ncbi.nlm.nih.gov/pubmed/?term=Enfu+Hui+Jeanne+Cheung 2017, 355(6332):1428-1433).

Kamphorst AOet al. confirmed that the activation of CD28 costimulatory signals is one of the important conditions for T cell "reactivation" (KamphorstAO et al., Rescue of exhausted CD8 T cells by PD-1-targeted therapies is CD28-dependent,Science,2017, 355(6332): 1423-1427). The activation of the costimulatory pathway of CD28/B7 (that is, the costimulatory molecule CD28 on the surface of T lymphocytes combined with CD86 (also known as B7-2) or CD80 (also known as B7-1)) is critical to tumor-bearing mice and the therapeutic effect of anti-PD-1 antibody during chronic viral infection. If the binding of B7 molecules to CD28 is blocked with anti-B7 antibodies, the inhibition of the anti-PD-1 antibody against the tumor is significantly reduced. Other studies have shown that soluble CD80 has therapeutic effects in vivo in mouse tumor systems (Horn LAet al., Soluble CD80 Protein Delays Tumor Growth and Promotes Tumor-Infiltratmg Lymphocytes,Cancer Immunol Res.2018; 6(1): 59-68). The low or no expression of CD80 and CD86 in the tumor microenvironment is also one of the important mechanisms that cause tumor immune evasion.

In addition, CTLA-4 expressed on the surface of T cells and the costimulatory molecule CD28 on the surface of T cells have a high degree of homology, and they have the same ligand CD86 (B7-2) or CD80 (B7-1). The combination of CTLA-4 and B7 molecules usually inhibits the activation of T cells, thus blocking the immune checkpoint B7/CTLA-4 pathway may enhance tumor-specific T cell activation.

On the other hand, tumor cells also release pro-angiogenic factors in the tumor microenvironment, such asVascular Endothelial Cell Growth Factor (VEGF), which leads to a surge in the number ofVEGF, VEGF mediates the proliferation and migration of vascular endothelial cells, improves vascular permeability, inhibits tumor cell apoptosis and provides a good microenvironment for tumor growth and metastasis by binding to its cell surface receptor VEGFR.

Currently, single-target tumor therapeutic drugs for blocking the PD-1/PD-L1 inhibitory signaling pathway, blocking the B7/CTLA-4 pathway, or blocking the VEGF/VEGFR pathway have been approved for marketing. They are, for example, anti-PD-1 antibody drugs, such as Nivolumab from Bristol-Myers Squibb Company (BMS) and Pembrolizumabfrom Merck Company(Merck); CTLA-4 antibody Ipilimumab (Ipilimumab, trade name Yervoy) from Bristol-Myers Squibb Company (BMS); Human-mouse chimeric anti-VEGF antibody Bevacizumab (trade name Avastin) from Genentech Inc.; Aflibercept Developed as a VEGF-Trap by Sanofi-aventis Company and Regeneron Company and so on. However, a considerable number of cancer patients do not respond to single-targeting tumor therapy or develop drug resistance. For example, the current average treatment efficiency of anti-PD-1 and PD-L1 antibody drugs is only about 20%, and the five-year survival rate of lung cancer is only 16%. Therefore, in many cases, multi-targeting therapy needs to be employed to avoid the problem that patients have no response to asingle targeted tumor or generate drug resistance.

Since multi-targeting fusion proteins are capable of simultaneously specifically targeting multiple Signal transduction pathways involved in tumor generation and development, there is a need to develop multi-targeting fusion proteins capable of improving tumor microenvironment by blocking vascular endothelial cell growth and activating T cells, and the need to combine the multi-targeting fusion protein with other anti-cancer drugs.

### SUMMARY OF THE INVENTION

The present inventors have developed a set of multi-targeting fusion proteins that block growth of vascular endothelial cells and activate T cells by the inventor (s), which comprises (i) a vascular endothelial cell growth inhibitor domain; (ii) an immunoglobulin FC domain; and (iii) a CD80 extracellular domain (ECD). The multi-targeting fusion protein can improve the tumor microenvironment and the effect of tumor immunotherapy from two aspects, wherein the improvement on the tumor microenvironment on one hand is achieved by the specific binding of the CD80 extracellular domain (ECD) of the multi-targeting fusion protein to CD28, PD-L1 and CTLA -4, specifically PD-1/PD-L1 inhibitory signal pathway is relieved to "Brake release" the immune system by binding PD-L1 this immune checkpoint to the CD80 extracellular domain; the same function of inhibiting regulatory T cells (Treg) as the anti-CTLA-4 antibody ipilimumab is exerted by binding the CD80 extracellular domain to CTLA-4;the CD28/B7 costimulatory pathway is activated through binding the CD80 extracellular domain to CD28 and T lymphocytes are activated to "throttle up the engine" for the activation of lymphocytes; on the other hand, the improvement of the tumor microenvironment is achieved by blocking the VEGF/VEGFR pathway through the vascular endothelial cell growth inhibitor domain of the multi-targeting fusion protein to inhibit the formation of tumor angiogenesis and to normalize tumor tissue blood vessels, so that more lymphocytes invade the tumor tissue, and relieve the inhibitory effect of VEGFs on immune cells.

In one embodiment, the multi-targeting fusion protein of the present invention comprises (i) derived from an antigen-binding fragment of an anti-VEGF antibody and/or an anti-VEGFR antibody and/or a VEGFR extracellular receptor functional domain; (ii) an immunoglobulin Fc domain; and (iii) a CD80 extracellular domain (ECD).

The antigen-binding fragment derived from the anti-VEGF antibody contained in the multi-targeting fusion protein can be derived from an antigen-binding fragment of any anti-VEGF antibody, as long as it is an antibody that can bind to VEGF and thereby block or inhibit the binding of VEGF to its receptor VEGFR. The anti-VEGF antibodies include anti-VEGF antibodies known in the prior art and anti-VEGF antibodies developed in the future. In one embodiment, preferably, the antigen-binding fragment of the anti-VEGF antibody is the Fab, Fab', F(ab')₂, Fv, single-chain Fv of the anti-VEGF antibody; preferably, the antigen-binding fragment of the anti-VEGF antibody comprises all six heavy chain CDRs and light chain CDRs that are contained in paired heavy chain variable region sequence/light chain variable region sequence selected from the group consisting of SEQ ID NO: 1/2, 3/4, and 5/6, or a sequence having one, two, three, four, or five amino acid changes (e.g., amino acid replacement or deletion) from one or more CDR(s) in all the six heavy chain CDRs and light chain CDRs; more preferably, the antigen-binding fragment of the anti-VEGF antibody comprises the paired heavy chain variable region sequence/light chain variable region sequence selected from the group consisting of SEQ ID NO: 1/2, 3/4 and 5/6, or a sequence having at least 90%,91%, 92%, 93%, 94%, 95%, 96%, 97%, 98%, 99% or moresequence identity to the paired heavy chain variable region sequence/light chain variable region sequence; most preferably, the antigen-binding fragment of the anti-VEGF antibody is the Fab of Bevacizumab, Ranibizumab or Ramucirumab.

The antigen-binding fragment derived from the anti-VEGFR antibody contained in the multi-targeting fusion protein can be derived from an antigen-binding fragment of any anti-VEGFR antibody, as long as it is an antibody that can bind to VEGFR and thereby block or inhibit VEGF binding to its receptor VEGFR. The anti-VEGFR antibodies include anti-VEGFR antibodies known in the art and anti-VEGFR antibodies developed in the future. In one embodiment, preferably, the antigen-binding fragment of the anti-VEGFR antibody is the Fab, Fab', F(ab')₂, Fv, single-chain Fv of the anti-VEGFR antibody and/or the anti-VEGFR antibody; preferably, the antigen-binding fragment of the anti-VEGFR antibody comprises all six heavy chain CDRs and light chain CDRs that are contained in paired heavy chain variable region sequence/light chain variable region sequence selected from the group consisting of SEQ ID NO: 1/2, 3/4, and 5/6, or a sequence having one, two, three, four, or five amino acid changes (e.g., amino acid replacement or deletion) from one or more CDR(s) in all the six heavy chain CDRs and light chain CDRs; more preferably, the antigen-binding fragment of the anti-VEGFR antibody comprises the paired heavy chain variable region sequence/light chain variable region sequence selected from the group consisting of SEQ ID NO: 1/2, 3/4 and 5/6, or a sequence having at least 90%,91%, 92%, 93%, 94%, 95%, 96%, 97%, 98%, 99% or moresequence identity to the paired heavy chain variable region sequence/light chain variable region sequence; most preferably, the antigen-binding fragment of the anti-VEGFR antibody is the Fab of Bevacizumab, Ranibizumab or Ramucirumab;

The VEGFR extracellular receptor functional domain contained in the multi-targeting fusion protein can be any VEGFR extracellular receptor functional domain, provided that it is an VEGFR extracellular receptor functional domain that can bind to VEGF and thereby block or inhibit the binding of VEGF to its receptor VEGFR. Preferably, the VEGFR extracellular receptor functional domain comprises the immunoglobulin-like domain 2 of VEGFR1 and the immunoglobulin-like domain 3 of VEGFR2; or theVEGFR extracellular receptor functional domain comprises the immunoglobulin-like domain 2 of VEGFR1 and theimmunoglobulin-like domain 3 of VEGFR2 and the immunoglobulin-like domain 4 of VEGFR2; or the VEGFR extracellular receptor functional domain comprises the immunoglobulin-like domain 2 of VEGFR1; more preferably,the VEGFR extracellular receptor functional domain has any amino acid sequence selected fromSEQ ID NO: 7-9 or an amino acid sequence having at least 90%, 91%, 92%, 93%, 94%, 95%, 96%, 97%, 98%, 99% or more sequence identity to the amino acid sequence shown in SEQ ID NO: 7-9.

(ii) The immunoglobulin Fc domain contained in the multi-targeting fusion protein can be any immunoglobulin Fc domain, in particular, the (ii) is a human immunoglobulin Fc domain. In one embodiment, the immunoglobulin Fc domain is an Fc domain of an IgG class antibody, in particular an Fc domain of IgG₁ subclass, IgG₂ subclass, IgG₄ subclass antibody. In a preferred embodiment, the immunoglobulin Fc domain contained in the multi-targeting fusion protein of the present invention is an Fc domain of an IgG1 subclass antibody, in particular an Fc domain of a human IgG1 subclass antibody. In a preferred embodiment, the immunoglobulin Fc domain contained in the multi-targeting fusion protein of the present invention is an Fc domain of an IgG4 subclass antibody, in particular an Fc domain of a human IgG4 subclass antibody. In some embodiments, (ii) the immunoglobulin Fc domain of the multi-targeting fusion protein of the present invention comprises an Fc domain of the amino acid sequence shown in SEQ ID NO: 10, 11, or 12, or an Fc domain comprising at least 90%,91%, 92%, 93%, 94%, 95%, 96%, 97%, 98%, 99%, or moresequence identity to the amino acid sequence shown in SEQ ID NO: 10, 11, or 12.

(III) The CD80 ECD included in the multi-targeting fusion protein is part of the extracellular domain of CD80. In one embodiment, theCD80 ECD comprises a CD80 immunoglobulin V (IgV) domain (CD80-IgV). In one embodiment, theCD80 ECD comprises a CD80 immunoglobulin V domain and a C domain (CD80-IgVIgC). In one embodiment, theCD80 ECD is a human CD80 ECD, preferably the CD80 ECD comprises a human CD80 IgV. In one embodiment, theCD80-IgV has an amino acid sequence as shown in SEQ ID NO: 13, or an amino acid sequence having at least 90%, 91%, 92%, 93%, 94%, 95%, 96%, 97%, 98%, 99% or more identity to the amino acid sequence of SEQ ID NO: 13. In one embodiment, theCD80-IgVIgC has an amino acid sequence as shown in SEQ ID NO: 14, or an amino acid sequence having at least 90%, 91%, 92%, 93%, 94%, 95%, 96%, 97%, 98%, 99% or more identity to the amino acid sequence of SEQ ID NO: 14.

In one embodiment, the multi-targeting fusion protein further comprises a peptide linker between the (i), (ii), and/or (iii); preferably, the peptide linker comprises one or more amino acids, more preferably at least 5 amino acids, and most preferably comprises a peptide linker selected from the group consisting of SEQ ID NOs: 20-46.

In one embodiment, the multi-targeting fusion protein is effectively linked from the n-terminal to the c-terminal in the order of (i), (ii), and (iii); in the order of (iii), (i) and (ii); or in the order of (iii), (ii), (i).

In some embodiments, the multi-targeting fusion protein of the present invention comprises
(a) A full-length anti-VEGF antibody, a full-length anti-VEGFR antibody, or a full-length anti-VEGF and VEGFR bispecific antibody; and one CD80 ECD effectively linked to the C-terminal of each of the two heavy chains of the antibody;
(b) A full-length anti-VEGF antibody, a full-length anti-VEGFR antibody, or a full-length anti-VEGF and VEGFR bispecific antibody; and one CD80 ECD effectively linked to the N-terminal of each of the two heavy chains of the antibody; and one CD80 ECD effectively connected to the N-terminal of each of the two light chains of the antibody;
(c) A CD80 ECD; an immunoglobulin Fc domain in dimer form effectively linked at the C-terminal of a CD80 ECD; and an antigen-binding fragment derived from an anti-VEGF antibody and/or an anti-VEGFR antibody effectively connected at the C-terminal of an immunoglobulin Fc domain in dimer form;
   or
(d) A CD80 ECD; an immunoglobulin Fc domain in dimer form effectively linked at the C-terminal of a CD80 ECD; and a VEGFR extracellular receptor functional domain effectively connected to the C-terminal of the immunoglobulin Fc domain in dimer form;

Preferably, the antibody is an IgG class antibody, particularly an IgG subclass, an IgG₂ subclass, an IgG₄ subclass antibody; and more particularly an IgG4 subclass antibody; also preferably, the IgG₄ subclass antibody contains amino acid replacement at the S228 position of the Fc domain, more preferably amino acid replacement S228P; further preferably, the light chain type of the antibody is κ type or λ type, preferably κ type;

Preferably, the full-length anti-VEGF antibody is Bevacizumab, and the full-length anti-VEGFR antibody is Ramucirumab.

In some embodiments, the multi-targeting fusion protein of the present invention is selected from
(1) A fusion protein comprising the first subunit of the fusion protein of SEQ ID NO: 80 and the second subunit of the fusion protein of SEQ ID NO: 82;
(2) A fusion protein comprising the first subunit of the fusion protein of SEQ ID NO: 84 and the second subunit of the fusion protein of SEQ ID NO: 86;
(3) A fusion protein comprising the fusion protein subunit of SEQ ID NO:88.

The present invention also provides polynucleotides encoding multi-targeting fusion proteins of the present invention, vectors comprising polynucleotides encoding multi-targeting fusion proteins of the present invention, preferably expression vectors, and most preferably glutamine synthetase expression vectors with double expression cassettes. In another aspect, the present invention provides a host cell comprising a polynucleotide or vector of the present invention. In one embodiment, the host cell is CHO, HEK293, or NSO cell. The present invention also provides a method for producing a multi-targeting fusion protein of the present invention, which comprises step (i) culturing host cell of the present invention under conditions suitable for expressing the multi-targeting fusion protein of the present invention, and step (ii) recovering the multi-targeting fusion protein of the present invention.

In another aspect, the invention also provides a pharmaceutical composition of the multi-targeting fusion protein of the present invention in combination with an anti-PD-1 antibody. The multi-targeting fusion protein of the present invention exhibits better synergy when used in combination with an anti-PD-1 antibody, thereby it is possible to achieve the purpose of better inhibition of tumor growth.

When used in combination with the multi-targeting fusion protein of the present invention, the anti-PD-1 antibody can be any anti-PD-1 antibody, as long as it is an antibody capable of inhibiting or reducing the binding of PD-1 to its ligand, including anti-PD-1 antibodies known in the art and anti-PD-1 antibodies developed in the future. In some embodiments, the anti-PD-1 antibody comprises all six heavy chain CDRs and light chain CDRs contained in paired heavy chain variable region sequences/light chain variable region sequence selected from the group consisting of SEQ ID NOS: 47/48, 49/50, 51/52, 53/54, 55/56, 57/58, 59/60, 61/62, 63/64, 65/66, 67/68, 69/70, and 71/72, or a sequence having one, two, three, four, or five amino acid changes (e.g., amino acid replacement or deletion) from one or more CDR(s) of the six heavy chain CDRs and light chain CDRs) sequence; further preferably, the anti-PD-1 antibody comprises paired heavy chain variable region sequence/light chain variable region sequence selected from SEQ ID NO: 47/ 48, 49/50, 51/52, 53/54, 55/56, 57/58, 59/60, 61/62, 63/64, 65/66, 67/68, 69/70 and 71/72 , or a sequence having at least 90%,91%, 92%, 93%, 94%, 95%, 96%, 97%, 98%, 99% or moresequence identity to the paired heavy chain variable region sequence/light chain variable region sequence; most preferably, the anti-PD-1 antibody is selected from Nivolumab, Pidilizumab and Pembrolizumab.

In yet another aspect, the present invention provides a use of multi-targeting fusion proteins or a use of pharmaceutical compositions of the present invention for manufacturing a medicament for the treatment or prevention of a cancerous disease(e.g., a solid tumor or a soft tissue tumor) in an individual, preferably the cancerous disease is melanoma, breast cancer, colon cancer, esophageal cancer, gastrointestinal stromal tumor (GIST), kidney cancer (e.g., renal cell carcinoma), liver cancer, non-small cell lung cancer (NSCLC), ovarian cancer, pancreatic cancer, prostate cancer, head and neck tumors, gastric cancer, or a hematological malignancy (e.g., lymphoma); in particular, the disease is colon cancer or triple negative breast cancer; preferably, wherein the individual is a mammal, more preferably a human.

Unless otherwise defined, all technical and scientific terms used herein have the same meaning as commonly understood by those ordinary skilled in the art to which the present invention belongs. All publications, patent applications, patents, and other references mentioned herein are hereby incorporated by reference in their entirety. Moreover, the materials, methods, and examples described herein are illustrative only and are not intended to be limiting. Other features, objects, and advantages of the invention will be apparent from the description and drawings, and from the appended claims.

### BRIEF DESCRIPTION OF THE DRAWINGS

The following detailed description of the preferred embodiments of the invention will be better understood when read in conjunction with the following drawings. For the purpose of illustrating the invention, embodiments are shown in the drawings which are presently preferred. However, it is to be understood that the invention is not limited to the precise arrangements and instrumentalities of the embodiments shown in the figures.

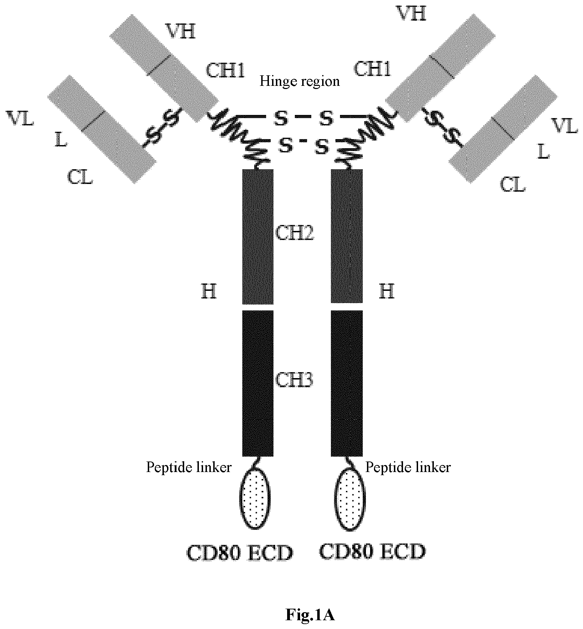

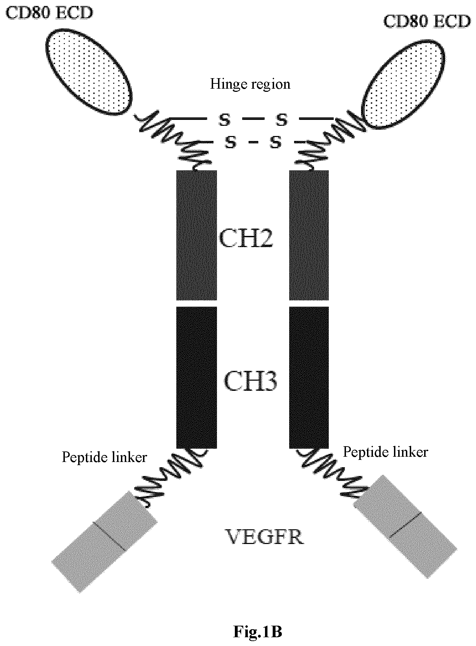

FIGS. 1A and B provide a schematic diagram of a multi-targeting fusion protein of the present invention, wherein FIG. 1A illustrates a structural schematic of a multi-targeting fusion protein comprising an antigen-binding fragment of an antibody, an immunoglobulin Fc domain, and a CD80 ECD from an N-terminal to a C-terminus; FIG. 1B illustrates a structural schematic of a fusion protein comprising CD80 ECD, immunoglobulin Fc domain, and VEGFR extracellular receptor functional region from N-terminus to C-terminus.

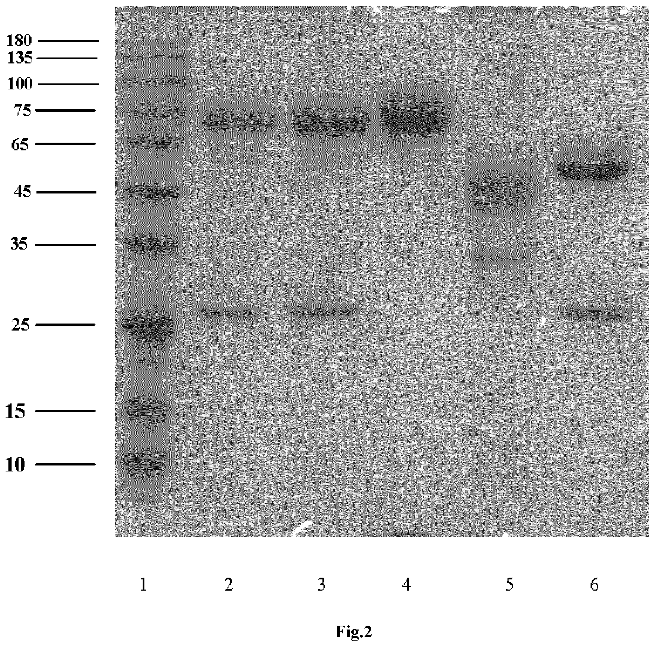

FIG. 2 shows the result that each prepared and purified protein of interest in the present invention is electrophoresed by the SDS-PAGE and then is stained with Coomassie Blue in the presence of a reducing agent (5 mM 1,4-dithiothreitol) in example 2. Lane 1: Protein Molecular Weight Standard Marker; Lane 2: Fusion Protein BY 24.4; Lane 3: Fusion Protein BY 24.5; Lane 4: Fusion Protein BY 24.12; Lane 5: Fusion Protein BY 31.19; Lane 6: Antibody BY 18.1

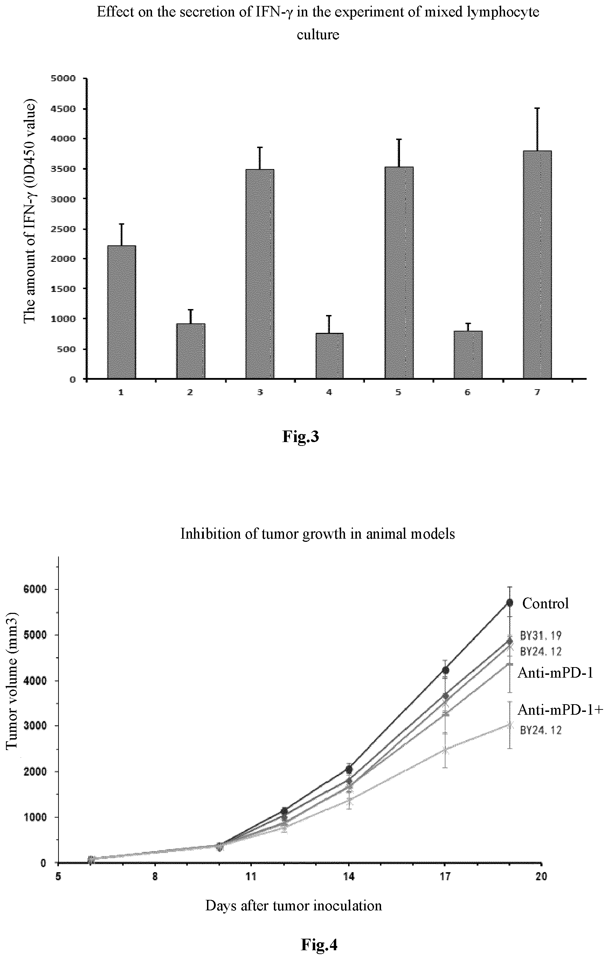

FIG. 3 shows that the multi-targeting fusion protein of the present invention and its combination with anti-PD-1 antibody have effect on the secretion of IFN-γ in the mixed lymphocyte reaction (MLR) in example 5. Column 1: BY18.1 group; column 2: BY24.4 group; column 3: BY18.1 + BY24.4 group; column 4: BY24.5 group; column 5: BY18.1 + BY24.5 group; column 6: BY24.12 group; column 7: BY18.1 + BY24.12 group.
Figure 4 shows that the multi-targeting fusion protein of the present invention and its combination with anti-PD-1 antibody have anti-tumor growth inhibitory effect on the animal model in Example 6.

### DETAILED DESCRIPTION OF THE INVENTION

The present invention provides a multi-targeting fusion protein that blocks vascular endothelial cell growth and activates T cells and a pharmaceutical composition comprising the multi-targeting fusion protein. The present invention also provides methods for producing the multi-targeted fusion protein, and the use of the multi-targeting fusion protein or pharmaceutical composition in the treatment or prevention of cancerous disease in individuals.

Unless otherwise defined herein, the terms in this specification are used as commonly used in the art.

### I. DEFINITION

The term" about", when used in conjunction with a numerical value, is intended to encompass numerical values having a lower limit of 5% less than the specified numerical value and an upper limit of 5% greater than the specified numerical value.

As used herein, the terms "comprises" or "comprising" is intended to include the stated elements, integers, or steps, but not to exclude any other elements, integers, or steps.

The "PD-1/PD-L1 inhibitory signaling pathway", "PD-1/PD-L1 signal pathway", "PD-1/PD-L1 signal transduction pathway", "PD-1/PD-L1 pathway" may be used interchangeably herein to refer to any intracellular signaling pathway initiated by PD-1 binding to PD-L1.

As used herein, "mitigating", "interfering", "inhibiting" or "blocking" PD-1/PD-L1 inhibitory signaling pathway may be used interchangeably, meaning (i) interfering with the interaction between PD-1 and PD-L1; and/or (ii) inhibition of at least one biological function leading to a PD-1/PD-L1 signaling pathway. The "mitigating", "interfering", "inhibiting" or "blocking" PD-1/PD-L1 signaling pathways resulting from the specific binding of the multi-targeting fusion protein and PD-L1 of the present invention need not be complete mitigating, interfering, inhibiting, or blocking.

As used herein, "CD28/B7 signaling pathway", "CD28/B7 costimulatory pathway", "CD28/B7 pathway" may be used interchangeably to refer to (i) asignaling pathwaythat stimulates cell activation by binding CD28 to CD80; and/or (ii) a signaling pathway that stimulates cell activation by binding CD28 to CD86.

Both "CD80" and "CD86" are transmembrane glycoprotein, which belong to an immunoglobulin superfamily member of a highly similar structure, also referred to as B7 molecules. The CD80 and CD86 extracellular regions are comprised of an immunoglobulin V (IgV) domain and an immunoglobulin C (IgC) domain. The mature CD80 molecule consists of 254 amino acids, wherein the extracellular domain consists of 208 amino acids, 25 amino acids across the membrane domain, and 21 amino acids in the intracellular domain. Similarly, the mature CD86 molecule is composed of 303 amino acids, wherein the extracellular domain is composed of 222 amino acids, 20 amino acids across the transmembrane domain, and 61 amino acids in the intracellular domain. CD80, also known as B7.1, expressed in the surface of T cells, B cells, dendritic cells, and monocytes, binding to CD28, PD-L1 and CTLA -4 at a lower affinity by its immunoglobulin V (IgV) domain, wherein the binding affinity of CD80 to CD28 is 4 µm; binding affinity of CD80 to PD-L1 is∼1.7 µm; binding affinity of CD80 to CTLA -4 is 0.2 µm (Butte, J et al.Programmed Death -1 Ligand1 Interaction Specifications with the B7-1 Costimulatory Molecule to Gbit T Cell Responses, Immunity, July 2007; 27 (1): 111-122). CD 86 binds to CD28 and CTLA -4, but does not bind to PD-L1.

Soluble CD80 (eg, CD80-Fc) can produce continuously activate T lymphocytes by a CD28/B7 costimulatory pathway and stimulate production of interferon. Experiments show that CD80-Fc maintains T lymphocytes in vitro to produce interferon, even more efficient than anti-PD-1 antibodies or anti-PD-Ll antibodies. In terms of inhibiting tumor growth in vivo, soluble CD80 (for example, CD80-Fc) is more effective than anti-PD-Ll antibody (Ostrnd-Rosenberg S et al., Novel Strategies for Inhibiting PD-1 Pathway-Mediated Immune Suppression while Simultaneous Delivering Activity Signals to Tumor-Reactive T Cells,Cancer Immunol Immunother. October 2015; 64 (10): 1287-93). The CD80-Fc can inhibit the immunosuppression mediated by PD-1/PD-L1 pathway by binding PD-L1, and deliver a costimulatory signal to T cells activated by the CD28/B7 costimulatory pathway, thereby enhancing the activation of T lymphocyte. In summary, CD80-Fc can mitigate immunosuppression of PD-1/PD-L1 pathways while activating tumor immunoreactive T cells. Although soluble CD86 (eg, CD86-Fc) can also activate CD28, even a 3-5 times activation of CD80-Fc can be produced, but since CD86 does not bind to PD-L1, finally, CD80-Fc has stronger activation effect on T lymphocytes than CD86-Fc (Haile St et al. Soluable CD80 Restorres T Cell Activation and Overcomes Tumor Cell Programmed Death Ligand 1-Mediated Immune Suppression). J Immunol.Sep. 1, 2013; 191 (5): 2829-36). In accordance with existing studies, CD80-Fc has the following effects: (i) when CD80-Fc is used alone, the effect of inhibiting the tumor is better than that of PD-L1 antibody (AACR ANNUAL MEETING, April 14-18, 2018, USA, Ill. Chicago); (ii) CD80-Fcpromotes lymphocyte invasion of tumor tissue, and is better than PD-L1 antibody (Horn Lala et al.), Soluble CD80 Protein DelayS Tumor Growth and Promoter Tumor-Infiltrazol,CancerImmunol Res. , January 2018; 6 (1): 59-68); (iii) when CD80-Fc is used alone, the effect of inhibiting the tumor is better than that of the PD-1/PD-L1 pathway and has a synergistic effect when combined with PD-1 antibody. Five-Prime Corporation even considers CD80-Fc superior to T cell agonists such as GITRL, OX40L, and 4-1 BBL. Since the CD80-Fc good immunotherapy effect was seen, the CD80-Fc Project FPT 155 Plan of Five-Prime Therapeutics, Inc. is about to start clinical trials recently.

As used herein, "B7/CTLA-4 pathway", "B7/CTLA-4 signaling pathway" may be used interchangeably to refer to (i) signaling pathwaysresulting from binding CD80 to CTLA-4; and/or (ii)signaling pathways resulting from binding CD86 to CTLA-4.

As used herein, "VEGF/VEGFR pathway", "VEGF/VEGFR signaling pathway" may be used interchangeably to refer to signaling pathways through binding one or more of the VEGF families to one or more of the cell surface receptor VEGFR families. The VEGF family contains six closely related polypeptides, which are highly conserved homodimer glycoprotein, with six subtypes: VEGF-A,-B,-C,-D,-E, and placental growth factor (PLGF), with molecular weights ranging from 35 to 44 kDa. The expression of VEGF-A (including splices thereof such as VEGF 165) is related to the microvascular density of some solid tumors, and the concentration of VEGF-A in the tissue is related to the prognosis of solid tumors such as breast cancer, lung cancer, prostate cancer and colon cancer. The biological activity of each VEGF family member is mediated by one or more of the cell surface VEGF receptor(VEGFR) families, including VEGFR1 (also referred to as FLT -1), VEGFR2 (also referred to as KDR, FLK -1), VEGFR3 (also referred to as FLT -4), etc. where VEGFR1, VEGFR2 is closely related to the generation of blood vessels, the VEGF-C/D/VEGFR3 is closely related to the lymphatic vessel generation. The main biological function of the VEGF family comprises: (1) selectively promoting mitosis of vascular endothelial cells, stimulating endothelial cells proliferation and promoting angiogenesis; (2) increasing the permeability of the blood vessel, particularly the microvascular, so that plasma macromolecules are extravasated and deposited in a matrix outside the blood vessels, and make plasma macromolecules extravasate and deposit in the matrix outside the blood vessels to provide nutrients for the growth of tumor cells and the establishment of new capillary networks;(3) promoting the proliferation and metastasis of tumors, the proliferation and metastasis of the tumor depend on the VEGF family to make vascular endothelial cells secrete collagenase and plasminogen to degrade the vascular base membrane, while the newly formed microvascular base membrane inside the tumor tissue is not perfect, which properties allow the tumor to easily enter the blood circulation; and (4) VEGF can be used as an immunosuppressive molecule to inhibit the immune response of the organism, and promote the infiltration and metastasis of malignant tumors (Lasperre-Prost Aet al. Immunomodulatory Activity of VEGF in Cancer,Int. Rev. Cell Mol. Biol., 2017; 330: 295-342); (5) Other effects: VEGF family can induce gaps and window openings in epithelial cells, and activate the cytoplasmic vesicles and organelles of epithelial cells; the VEGF family directly stimulates endothelial cells to release proteolytic enzymes, degrade the matrix, release more VEGF family molecules, and accelerate the development of tumors, extracellular proteases can also activate associativity of extracellular matrix and release of VEGF family; VEGF family releases plasma proteins (including fibrinogen) and cellulose network is formed by increasing vascular permeability, which provides a good matrix for tumor growth, development and metastasis; VEGF family promotes the formation of abnormal blood vessels and prevents immune cell invasion and so on.

Clinical studies show the use of anti-VEGF monoclonal antibodies, anti-VEGFR monoclonal antibodies, or soluble VEGFR capable of blocking binding of VEGF families with their receptors and impeding conduction of VEGF family signaling pathways. Bevacizumab (trade name Avastin) developed by Genentech is a recombinant human-mouse chimeric anti-VEGF antibody that can block the binding of VEGF-A and VEGFR, so that VEGFR cannot be activated. This plays an anti-angiogenic effect. Bevacizumab is currently used in the treatment of metastatic colorectal cancer, lung cancer, breast cancer, pancreatic cancer, kidney cancer, etc. Aflibercept developed by Sanofi-aventis and Regeneron is a VEGF-Trap, which is a kind of fusion protein obtained by fusing the second extracellular domain of VEGFR1 and the third extracellular domain of VEGFR2 with the constant region of human IgG1, can exert anti-tumor effect on some tumor patients by inhibiting angiogenesis.

As used herein, the term "specific binding" means that binding to an antigen or target molecule with selectivity and can be distinguished from unwanted or non-specific interaction. The specific binding may be measured by enzyme-linked immunosorbent assay (ELISA) or other techniques familiar to those skilled in the art, such as surface plasmon resonance (SPR) techniques (analyzed on Biacore, et al. Analysis of Agcto-Ciglin Rheumami Using Surface Plasmons Resonance, Glyco. J. 2000, 17, 323-329).

The term "vascular endothelial growth inhibitor domain" refers to a portion of a vascular endothelial growth inhibitor that blocks VEGF/VEGFR signaling conduction, said portion is an domain playing a role in inhibiting vascular endothelial growth. The vascular endothelial growth inhibitor domains may be provided by, for example, a variable domain (also referred to as an antibody variable region) of one or more anti-VEGF antibodies, a variable domain of one or more anti-VEGFR antibodies, or a VEGFR extracellular receptor function.

The term "regulatory T cell" represents a particular T lymphocyte subgroup that is critical to maintaining self-tolerance. The Treg cells with the inhibitor function can be separated from other T lymphocyte by intracellular expression of transcription factor FOXP3 and other cell markers such as CD127 ^{low}, CTLA -4 +, LAP, CD39 +, PD - 1 +, GRP, etc.

"Affinity" or "binding affinity" refers to the inherent binding affinity that reflects interactions between members in binding pair. The affinity of molecule X to its partner Y may generally be represented by a dissociation constant (KD), which is a ratio of dissociation rate constant and associated rate constant (K OFF and K ON, respectively). The affinity may be measured by a common method known in the art. One specific method for measuring affinity is surface plasmon resonance (SPR).

The term "antibody" is used herein in its broadest sense and includes, but is not limited to, monoclonal antibodies, polyclonal antibodies, multispecific antibodies (e.g., bispecific antibodies), provided that they exhibit the desired antigen binding activity. Antibodies can be a complete antibody (e.g., having two full-length light chains and two full-length heavy chains) of any type and subtype (e.g.,IgM, IgD, IgG1, IgG2, IgG3, IgG4, IgE, IgA1 and IgA2).

The terms "all-antibody," " full-length antibody, " "full antibody," and "complete antibody" are used interchangeably herein to refer to an antibody which has a structure substantially similar to that of a natural antibody.

The term "antibody heavy chain" refers to a greater of two types of polypeptide chains that are present in the antibody molecule in its native presence, which in normal circumstances determine the class to which the antibody belongs.

The term "antibody light chain" refers to a smaller of two types of polypeptide chains present in an antibody molecule in its native presence. κ light chain and λ light chain refers to two major antibody light chain types.

"Bispecific antibody" is an artificial heterozygous antibody having two different heavy chain/light chain pairs and having two different binding sites. Bispecific antibodies can be prepared by a variety of methods, including the attachment of hybridoma fusion or Fab' fragments.

The term "antigen-binding fragment" of an antibody is a part or a segment of an antibody or antibody chain that has less amino acid residue than a complete or complete antibody or antibody chain, which can bind to an antigen or compete with full antibody (i.e., with a complete antibody derived from an antigen-binding fragment) for binding to an antigen. Antigen binding fragments can be prepared by recombinant DNA techniques, or by enzyme or chemically cleavage of complete antibodies. Antigen binding fragments include, but are not limited to, Fab ', Fab ', F (ab ') ₂, Fv ', single chain Fv '. The Fab fragment is a monovalent fragment consisting of VL, VH, Cl and CHI domains, for example, a Fab fragment can be obtained by digestion of a complete antibody by papain. In addition, F (ab ') ₂, which is a dimer of Fab ', is a bivalent fragment by digestion of the complete antibody under the disulfide bond of the hinge region by pepsin. F (ab ')₂ may be reduced under neutral conditions by breaking disulfide bonds in the hinge region, thus converting the F (ab ')₂ dimer to a Fab ' monomer. The Fab ' monomer is essentially a Fab fragments with a hinge region (more detailed description of other antibody fragments, please refer to: Fundamental Immunology, edited by W.E. Paul, Raven Press, N.Y. (1993)). The Fv fragment consists of VL and VH domains of the antibody single arm. In addition, although the two domains VL and VH of the Fv fragment are encoded by independent genes, they can be connected through a synthetic linker that enable these two domains to be produced as a single protein chain using recombination methods, and the VL region and VH region in the protein chain are paired to form a single-chain Fv. The antibody fragments can be obtained by chemical methods, recombinant DNA methods or protease digestion methods.

The term "immunoglobulin" refers to a protein having a structure of a naturally occurring antibody. For example, IgG immunoglobulin is a heterotetrameric glycoprotein of about 150,000 Daltons consisting of two light chains and two heavy chains bound by disulfide bonds. From the N-terminal to the C-terminal, each immunoglobulin heavy chain has a variable region (VH), also referred to as a variable heavy chain domain or a heavy chain variable domain, followed by three constant domains (CH1, CH2 and CH3), also referred to as heavy chain constant regions. Similarly, from N-terminal to the C-terminal, each immunoglobulin light chain has a variable region (VL), also referred to as a variable light chain domain or a light chain variable domain, followed by a constant light chain (CL) domain, also referred to as a light chain constant region. The heavy chains of immunoglobulins can belong to one of five categories, called α (IgA), δ (IgD), ε (IgE), γ (IgG) or µ (IgM), some of which can be further divided into sub-classes, such as γ₁ (IgG1), γ₂ (IgG2), γ₃ (IgG₃), γ₄ (IgG₄), α₁ (IgA₁, and α₂ (IgA₂). The light chains of immunoglobulins can be divided into one of two types based on the amino acid sequence of their constant domains, called κ and λ An immunoglobulin basically consists of two Fab molecules and an Fc domain connected by the hinge region of an immunoglobulin.

The term "Fc domain" or "Fc region" is used herein to define a C-terminal region of an immunoglobulin heavy chain containing at least a portion of the constant region. The term includes a natural sequence Fc region and a variant Fc region. A natural immunoglobulin "Fc domain" includes two or three constant domains, i.e., CH2 domains, CH3 domains, and optional CH4 domains. For example, in a natural antibody, the immunoglobulin Fc domain comprises second and third constant domains (CH2 domains and CH3 domains) derived from two heavy chains of IgG, IgA and IgD antibodies; or second, third, and fourth constant domains (CH2 domains, CH3 domains and CH4 domains) comprising two heavy chains derived from IgM and IgE antibodies. Unless otherwise indicated herein, the number of the amino acid residue in the Fc region or the heavy chain constant region are numbered according to the EU numbering system (also referred to as the EU index) described in Kabat et al. Sequences of Proteins of Immunological Interest, 5th Ed. Public Health Care Service, National Institutes of Health, Bethesda et al. MD. 1991.

"Human immunoglobulin" is an immunoglobulin that possesses an amino acid sequence corresponding to an immunoglobulin produced by a human or human cell or derived from a non-human source utilizing a human immunoglobulin library or other sequences encoding human immunoglobulin.

"Identity Percent (%)" of the amino acid sequence refers to the percentage of amino acid residues in the candidate sequence that are identical to the amino acid residues of the specific amino acid sequence shown in this specification, and without regard to any conservative replacement as part of the sequence identity, after the candidate sequence is compared to the specific amino acid sequence shown in this specification and is introduced in gaps, and if necessary, to reach the maximum sequence identity percent.

The term "effectively linked" means that the specified components are in a relationship that allows them to function in an intended manner.

The term "N-terminal" refers to the last amino acid of the N-terminus, and the term "C-terminal" refers to the last amino acid of the C-terminus.

The term "fusion" refers to two or more components are effectively linked directly by a peptide bond or by one or more peptide linkers.

The term "host cell" refers to a cell into which exogenous polynucleotides have been introduced, including progeny of such cells. Host cells include "transformants" and "transformed cells", which include primary transformed cells and progeny derived therefrom. Host cells are any type of cell system that can be used to produce the multi-targeting fusion protein of the present invention. Host cells include cultured cells, also including transgenic animals, transgenic plants, or cultured plant tissues or cells inside animal tissues.

The terms "individual" or "subject" are used interchangeably to refer to a mammal. Mammals include, but are not limited to, domesticated animals (e.g., cows, sheep, cats, dogs, and horses), primate (e.g., human and non-human primate such as monkeys), rabbits, and rodents (e.g., mice and rats). In particular, an individuals is human.

The term "treatment" refers to a clinical intervention intended to alter the natural process of a disease in individuals undergoing treatment. The desired therapeutic effect includes, but is not limited to, preventing the occurrence or recurrence of a disease, alleviating symptoms, reducing any direct or indirect pathologies of a disease, preventing metastasis, reducing disease progression rate, ameliorating or alleviating disease states, and alleviating or improving prognosis. In some embodiments, the multi-targeting fusion protein or pharmaceutical composition of the present invention is used to delay disease progression or to slow the progression of disease.

The term "anti-tumor action" refers to a biological effect that can be demonstrated by a variety of means, including but not limited to, for example, reduction in tumor volume, reduced number of tumor cells, reduced tumor cell proliferation, or reduced tumor cell survival. The terms "tumor", " cancer"and"cancerous disease" are used interchangeably herein to encompass solid tumors and liquid tumors.

### II. Multi-Targeting Fusion Protein

The present invention provides a novel multi-targeting fusion protein comprising (i) a vascular endothelial growth inhibitor domain; (ii) an immunoglobulin Fc domain; and (iii) a CD80 extracellular domain (ECD).

In some embodiments, the (i), (ii) and/or (iii) are optionally effectively linked by a peptide linker.

In some embodiments, the multi-targeting fusion protein of the present invention is a heterotetrameric glycoprotein composed of two fusion protein first subunits and two fusion protein second subunits that are bonded by disulfide bonds.

In some embodiments, the multi-targeting fusion protein of the present invention is homodimer or heterodimer glycoprotein bonded by disulfide bonds.

Multi-targeting fusion proteins of the present invention block vascular endothelial cell growth and activate T lymphocytes. This multi-targeting fusion protein is capable of blocking vascular endothelial cell growth by blocking VEGF/VEGFR pathways, on the other hand, inhibiting PD-1/PD-L1 inhibitory signal pathways by activating CD28/B7 co-stimulatory pathways, inhibiting regulatory T-cell (Treg) functions by theB7/CTLA -4signaling pathway to activate T lymphocytes, thereby improving the tumor microenvironment and the tumor immunotherapy effect.

In some embodiments, the multi-targeting fusion protein of the present invention binds to VEGF or VEGFR at a dissociation constant (KD) of 10⁻⁸ M or less, e.g., at a dissociation constant (KD) of 10⁻⁹ M to 10⁻¹² M; and specifically binds to CD28, PD-L1, andCTLA -4.

Various components of multi-targeting fusion proteins of the present invention are described below.

### Vascular endothelial growth inhibitor domain

"Vascular endothelial growth inhibitor domains" in multi-targeting fusion proteins of the present invention are capable of specifically binding VEGF and/or VEGFR, including, but not limited to, antigen-binding fragments and/or VEGFR extracellular receptor functional domains derived from anti-VEGF antibodies and/or anti-VEGFR antibodies.

In one embodiment, the multi-targeting fusion protein of the present invention comprises antigen-binding fragments derived from anti-VEGF antibodies and/or anti-VEGFR antibodies so that the multi-targeting fusion protein of the present invention can specifically bind to VEGF and/or VEGFR with high affinity, e.g., at 10⁻⁸ M or less, preferably 10⁻⁹ M to 10⁻¹² M.

Examples of paired heavy chain variable regions (VH) and light chain variable regions (VL) in antigen-binding fragments of anti-VEGF antibodies or anti-VEGFR antibodies included in the multi-targeting fusion proteins of the present invention are provided herein below in Table 1. In some embodiments, the antigen-binding fragment of the multi-targeting fusion protein of the present invention comprise a sequence which is substantially identical to the amino acid sequence of the heavy chain variable region (VH) and/or light chain variable region (VL) of the anti-VEGF antibody and/or anti-VEGFR antibody in Table 1, for example, a sequence having at least 90%,91%, 92%, 93%, 94%, 95%, 96%, 97%, 98%, 99% or moresequence identity to the paired heavy chain variable region sequence/light chain variable region shown in Table 1.

**Table 1. Examples of heavy chain variable regions and light chain variable region sequences in antigen binding fragments of anti-VEGF antibodies or anti-VEGFR antibodies.**

| Variable region | Amino acid sequence | Sequence number (SEQ ID NO:) |
|---|---|---|
| VH | EVQLVESGGGLVQPGGSLRLSCAASGYTFTNYGMNWVRQAPGK GLEWVGWINTYTGEPTYAADFKRRFTFSLDTSKSTAYLQMNSLR AEDTAVYYCAKYPHYYGSSHWYFDVWGQG | 1 |
| VL | DIQMTQSPSSLSASVGDRVTITCSASQDISNYLNWYQQKPGKAPK VLIYFTSSLHSGVPSRFSGSGSGTDFTLTISSLQPEDFATYYCQQYS TVPWTFGQG | 2 |
| VH | EVQLVESGGGLVQPGGSLRLSCAASGYDFTHYGMNWVRQAPGK GLEWVGWINTYTGEPTYAADFKRRFTFSLDTSKSTAYLQMNSLR AEDTAVYYCAKYPYYYGTSHWYFDVWGQG | 3 |
| VL | DIQLTQSPSSLSASVGDRVTITCSASQDISNYLNWYQQKPGKAPK | 4 |
| | VLIYFTSSLHSGVPSRFSGSGSGTDFTLTISSLQPEDFATYYCQQYS TVPWTFGQG | |
| VH | EVQLVQSGGGLVKPGGSLRLSCAASGFTFSSYSMNWVRQAPGK GLEWVSSISSSSSYIYYADSVKGRFTISRDNAKNSLYLQMNSLRA EDTAVYYCARVTDAFDIWGQG | 5 |
| VL | DIQMTQSPSSVSASIGDRVTITCRASQGIDNWLGWYQQKPGKAP KLLIYDASNLDTGVPSRFSGSGSGTYFTLTISSLQAEDFAVYFCQQ AKAFPPTFGGG | 6 |

In one embodiment, the antigen binding fragment of the multi-targeting fusion protein of the present invention comprises all six heavy chain complementarity determining regions (CDRs) and light chain CDRs contained in paired heavy chain variable region sequences/light chain variable region sequences selected from SEQ ID NO: 1/2, 3/4, and 5/6. Methods and techniques for identifying CDRs in an amino acid sequence of a heavy chain variable region and a light chain variable region are known in the art and may be used to identify CDRs in the amino acid sequence of a particular heavy chain variable region and/or light chain variable region disclosed herein. Exemplary well-known techniques that may be used to identify CDR boundary include, for example, Kabat Definition, Chothia Definition, and ABM Definition. See, for example, Kabat, Sequences of Proteins of Immunological Interest, National Institutes of Health, Bethesda, Md. (1991); Al-Lazikano et al. Standard Modifications for the Canopy Structures of Immunoglobalins. J Mol. Biol.273: 927-948 (1997); and Martin Ac etal. Modeling of Antibody Hypervariable Loops: a Combined Algorithm, Proc. Natl. Acad. Sci. USA 86: 9268-9272 (1989).

Anti-VEGF antibodies or anti-VEGFR antibodies, which are sources of antigen binding fragments in a multi-targeting fusion protein of the present invention, can be divided into κ type or λ type, based on the amino acid sequence of its light chain constant region, preferably κ type.

Examples of amino acid sequences of light chain constant regions of anti-VEGF antibodies or anti-VEGFR antibody are provided herein below.

**Table 2. Examples of light chain constant region sequences of antibodies.**

| Light chain type | amino acid sequences of constant region |
|---|---|
| κ type | TKVEIKRTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGEC （SEQ ID NO：15） |
| λ type | TKLTVLGQPKAAPSVTLFPPSSEELQANKATLVCLISDFYPGAVTVAWKADSSPVKAG VETTTPSKQSNNKYAASSYLSLTPEQWKSHRSYSCQVTHEGSTVEKTVAPTECS（SEQ ID NO：16） |

The anti-VEGF antibody or anti-VEGFR antibody that is source of the antigen-binding fragment of the multi-targeting fusion protein in the present invention is preferably an IgG antibody, particularly IgG₁ subclass, IgG₂ subclass, IgG₄ subclass, more particularly IgG₄ subclass based on the amino acid sequence of the constant region of the anti-VEGF antibody or anti-VEGFR antibody. Preferably, theIgG4 subclass antibody contains amino acid replacement that prevent arm-exchange at position S228 in the Fc region, particularly amino acid replacement S228P.

Examples of amino acid sequences of antibody heavy chain constant regions are provided herein in Table 3 below.

**Table 3. Examples of heavy chain constant region sequences of antibodies.**

| Heavy chain type | amino acid sequences of constant region |
|---|---|
| IgG1 | TLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCL VKGFYPSDIAVEWESNGQPENNYKTTPPVLDSDGSFFLYSKLTVDKSRWQQ GNVFSCSVMHEALHNHYTQKSLSLSPGK（SEQ ID NO：17） |
| IgG2 | TTVTVSTASTKGPSVFPLAPCSRSTSESTAALGCLVKDYFPEPVTVSWNSGA LTSGVHTFPAVLQSSGLYSLSSVVTVPSSNFGTQTYTCNVDHKPSNTKVDKT VERKCCVECPPCPAPPVAGPSVFLFPPKPKDTLMISRTPEVTCVVVDVSHEDP EVQFNWYVDGVEVHNAKTKPREEQFNSTFRVVSVLTVVHQDWLNGKEYK CKVSNKGLPAPIEKTISKTKGQPREPQVYTLPPSREEMTKNQVSLTCLVKGF YPSDIAVEWESNGQPENNYKTTPPMLDSDGSFFLYSKLTVDKSRWQQGNVF |
| | SCSVMHEALHNHYTQKSLSLSPGK（SEQ ID NO：18） |
| IgG4 | TLVTVSSASTKGPSVFPLAPCSRSTSESTAALGCLVKDYFPEPVTVSWNSGAL TSGVHTFPAVLQSSGLYSLSSVVTVPSSSLGTKTYTCNVDHKPSNTKVDKRV ESKYGPPCPPCPAPEFLGGPSVFLFPPKPKDTLMISRTPEVTCVVVDVSQEDP EVQFNWYVDGVEVHNAKTKPREEQFNSTYRVVSVLTVLHQDWLNGKEYK CKVSNKGLPSSIEKTISKAKGQPREPQVYTLPPSQEEMTKNQVSLTCLVKGF YPSDIAVEWESNGQPENNYKTTPPVLDSDGSFFLYSRLTVDKSRWQEGNVFS CSVMHEALHNHYTQKSLSLSLGK（SEQ ID NO：19） |

In one embodiment, the VEGFR extracellular receptor functional region contained in the multi-targeting fusion protein of the present invention is part of or a combination of the extracellular domain of the VEGFR. VEGFR receptor is a tyrosine kinase receptor located on the surface of a cell consisting of 7 immunoglobulin (Ig)-like domains. For example, human VEGFR1 contains 7 Ig-like domains numbered 1,2,3, 4,5,6, and 7, the Ig-like domain 1 is at the N-terminus of the extracellular domain, and the Ig-like domain 7 is at the C-terminus of the extracellular domain. Unless otherwise indicated herein, the Ig-like domains are numbered sequentially from the N-terminal to the C-terminal of the VEGFR protein. In some embodiments, an VEGFR extracellular receptor functional domain comprises at least one IG-like domain selected from one or more VEGFR of VEGFR1, VEGFR2, and VEGFR3. In some aspects, an VEGFR extracellular receptor functional domain comprises at least 1,2,3, 4,5,6, but not exceed 7 Ig-like domains of VEGFR. In another aspect, the VEGFR extracellular receptor functional domain comprises 1-7, 1-6, 1-5, 1-4, 1-3, or 1-2 Ig-like domains of VEGFR.

The VEGFR extracellular receptor functional domain that contains at least one Ig-like domain of two or more VEGFRs is also considered herein. In some embodiments, the VEGFR extracellular receptor functional domain comprises at least one Ig-like domain from two or more VEGFR selected from VEGFR1, VEGFR2, and VEGFR3. A VEGFR extracellular receptor functional domain comprises any combination of 7 Ig-like domains of each VEGFR is considered herein. For example, the VEGFR extracellular receptor functional region may comprise an Ig-like domain 2 of VEGFR1 (e.g., human VEGFR1) and an Ig-like domain 3 of VEGFR2 (e.g., human VEGFR2). In another embodiment, the VEGFR extracellular receptor function domain may comprise lg-like domains 1-3 of VEGFR1 (e.g., human VEGFR1), lg-like domains 2-3 of VEGFR1 (e.g., human VEGFR1), lg-like domains 1-3 of VEGFR2 (e.g., human VEGFR2), lg-like domains 2 of VEGFR1 (e.g., human VEGFR1) and lg-like domains 3-4 of VEGFR2 (e.g., human VEGFR2), or lg-like domains 2 of VEGFR1 (e.g., human VEGFR1) and lg-like domains 3 of VEGFR3 (e.g., human VEGFR3). These Ig-like domains and other Ig-like domains that can be used as part of the VEGFR extracellular receptor functional domain are described more in detail in U.S. Patent No. 7,531,173; Yu DC et al., Soluble vascular endothelial growth factor decision receptor FP3 exerts potent antiangiogenic effects , Mol.Ther., 2012, 20(3): 938-947 and Holash, J. et al., VEGF-Trap: a VEGF blocker with potent antitumor effects, PNAS, 2002, 99(17): 11393-11398, all documents are hereby incorporated by reference in their entirety. In some embodiments, the VEGFR extracellular receptor functional region has any amino acid sequence selected from the group consisting of amino acid sequences shown inSEQ ID NO: 7-9 in Table 4 or amino acid sequences having at least 90%, 91%, 92%, 93%, 94%, 95%, 96%, 97%, 98%, 99% or more identity to the amino acid sequence shown in SEQ ID NO: 7-9.

**Table 4. Examples of amino acid sequences of VEGFR extracellular receptor functional domain in Multi-Targeting Fusion Protein**

| The VEGFR extracell ular receptor functional domain | Amino acid sequence |
|---|---|
| VEGFR1-D2/V EGFR2-D3 | SDTGRPFVEMYSEIPEIIHMTEGRELVIPCRVTSPNITVTLKKFPLDTLIPDGKRIIW DSRKGFIISNATYKEIGLLTCEATVNGHLYKTNYLTHRQTNTIIDVVLSPSHGIELS VGEKLVLNCTARTELNVGIDFNWEYPSSKHQHKKLVNRDLKTQSGSEMKKFLST LTIDGVTRSDQGLYTCAASSGLMTKKNSTFVRVHEK（SEQ ID NO：7） |
| VEGFR1-D2/V EGFR2-D3-D4 | SDTGRPFVEMYSEIPEIIHMTEGRELVIPCRVTSPNITVTLKKFPLDTLIPDGKRIIW DSRKGFIISNATYKEIGLLTCEATVNGHLYKTNYLTHRQTNTIIDVVLSPSHGIELS VGEKLVLNCTARTELNVGIDFNWEYPSSKHQHKKLVNRDLKTQSGSEMKKFLST LTIDGVTRSDQGLYTCAASSGLMTKKNSTFVRVHEKPFVAFGSGMESLVEATVG |
| | ERVRIPAKYLGYPPPEIKWYKNGIPLESNHTIKAGHVLTIMEVSERDTGNYTVILT NPISKEKQSHVVSLVVYVPPGPGD（SEQ ID NO：8） |
| VEGFR1-D2 | SDTGRPFVEMYSEIPEIIHMTEGRELVIPCRVTSPNITVTLKKFPLDTLIPDGKRIIW DSRKGFIISNATYKEIGLLTCEATVNGHLYKTNYLTHRQTNT （SEQ ID NO：9） |

The VEGFR extracellular receptor functional domain of the multi-targeting fusion protein of the present invention can specifically bind to VEGF family with high affinity, e.g., at 10⁻⁸ M or less, preferably 10⁻⁹ M to 10⁻¹² M, and thereby inhibit binding of VEGF family to cell surface VEGFR and subsequent signaling.

### Immunoglobulin Fc domain

The "immunoglobulin Fc domain" in the multi-targeting fusion protein of the present invention includes all the amino acid residues of the naturally-occurring immunoglobulin Fc domain or a part of the amino acid residues of the naturally-occurring immunoglobulin Fc domain. The immunoglobulin Fc domain provides favorable pharmacokinetics properties for the multi-targeting fusion protein of the present invention, including but not limited to long serum half-life. In addition, an immunoglobulin Fc domain also makes it possible to purify the multi-targeting fusion protein of the present invention by, for example, protein A affinity chromatography.

An immunoglobulin Fc domain is usually a dimeric molecule. The immunoglobulin Fc domain can be produced by papain digestion or trypsin digestion of intact (full-length) immunoglobulins or can be recombinantly produced, which comprise a CH2 domain, a CH3 domain and an optional CH4 domain.

In one embodiment, an IgG Fc domain includes an IgG CH2 domain and an IgG CH3 domain. Preferably, the immunoglobulin Fc domain has the amino acid sequence shown inSEQ ID NO: 10-12 in Table 5 or having an amino acid sequence at least 90%, 91%, 92%, 93%, 94%, 95%, 96%, 97%, 98%, 99% or more identical to the amino acid sequence shown in SEQ ID NO: 10-12.

**Table 5. Examples of amino acid sequence of immunoglobulin Fc domain in multi-targeting fusion protein**

| Name | Amino acid sequence |
|---|---|
| IgG1 Fc | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAK |
| | GQPREPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSPGK （SEQ ID NO：10） |
| IgG2 Fc | DKTVERKCCVECPPCPAPPVAGPSVFLFPPKPKDTLMISRTPEVTCVVVDVSHEDPEVQFN WYVDGVEVHNAKTKPREEQFNSTFRVVSVLTVVHQDWLNGKEYKCKVSNKGLPAPIEKT ISKTKGQPREPQVYTLPPSREEMTKNQVSLTCLVKGFYPSDIAVEWESNGQPENNYKTTPP MLDSDGSFFLYSKLTVDKSRWQQGNVFSCSVMHEALHNHYTQKSLSLSPGK （ SEQ ID NO：11） |
| IgG4 Fc | DKRVESKYGPPCPPCPAPEFLGGPSVFLFPPKPKDTLMISRTPEVTCVVVDVSQEDPEVQFN WYVDGVEVHNAKTKPREEQFNSTYRVVSVLTVLHQDWLNGKEYKCKVSNKGLPSSIEKT ISKAKGQPREPQVYTLPPSQEEMTKNQVSLTCLVKGFYPSDIAVEWESNGQPENNYKTTPP VLDSDGSFFLYSRLTVDKSRWQEGNVFSCSVMHEALHNHYTQKSLSLSLGK（SEQ ID NO： 12） |

In addition to the sequence defined in SEQ ID NO: 10-12, the IgG Fc domain may also include a peptide sequence obtained after additional sequence modification of SEQ ID NO: 10-12, for example, a peptide sequence obtained by substituting, deleting or deriving one or more amino acid residues of the amino acid residues in SEQ ID NO: 10-12. In one embodiment, the S228 position in the IgG Fc domain includes amino acid replacement that prevents arm-exchange, especially amino acid replacement S228P. In one embodiment, the IgG4 Fc region contains amino acid replacement S228P.

### Extracellular domain (ECD) of CD 80

The "extracellular domain (ECD) of CD80" in the multi-targeting fusion protein of the present invention comprises all amino acid residues of naturally occurring CD80 ECD or a portion of the amino acid residues comprising naturally occurring CD80 ECD. In some embodiments, the CD80 ECD comprises a CD80 IgV, preferably the CD80 ECD comprises a human CD80 IgV, and more preferably the CD80 ECD has an amino acid sequence as shown inSEQ ID NO: 13 or 14 in Table 6.

**Table 6 Examples of CD80 ECD amino acid sequence in multi-targeting fusion protein**

| Name | Amino acid sequence |
|---|---|
| CD80-IgV | VIHVTKEVKEVATLSCGHNVSVEELAQTRIYWQKEKKMVLTMMSGDMNIWPEY KNRTIFDITNNLSIVILALRPSDEGTYECVVLKYEKDAFKREHLAEVTLSVKADFP |
| | TPS（SEQ ID NO：13） |
| CD80-IgVIgC | （SEQ ID NO：14） |

In addition to the sequences as defined by SEQ ID NOS: 13 and 14, the CD80 ECD may also contain peptide sequences obtained after additional sequence modifications to SEQ ID NO: 13 and 14, such as peptide sequences obtained after one or more conservative replacements, deletions, or derivatization of amino acid residues in SEQ ID NO: 13 and 14, as long as they have substantially the same activity or function as unmodified peptides. The modified peptide will retain the activity or function associated with the unmodified peptide. The modified peptide typically has an amino acid sequence that is substantially homologous to the amino acid sequence of the unmodified sequence, e.g., an amino acid sequence having at least 90%, 91%, 92%, 93%, 94%, 95%, 96%, 97%, 98%, 99% or more identity to the amino acid sequence shown in SEQ ID NO: 13 or 14.

### Peptide linker

The multi-targeting fusion protein of the present invention will comprise (i) a vascular endothelial growth inhibitor domain; (ii) an immunoglobulin Fc domain; and (iii) a "peptide linker" optionally effectively linked to the CD80 ECD is a peptide of one or more amino acids, typically about 2-20 amino acids. Peptide linkers are known in the art or described herein.

In some embodiments, the peptide linker comprises at least 5 amino acids, preferably comprising the peptide linker selected from AKTTPKLEEGEFSEAR (SEQ ID NO: 20); AKTTPKLEEGEFSEARV(SEQ ID NO: 21); AKTTPKLGG(SEQ ID NO: 22) ; SAKTTPKLGG(SEQ ID NO: 23); SAKTTP(SEQ ID NO: 24); RADAAP(SEQ ID NO: 25); RADAAPTVS(SEQ ID NO: 26); RADAAAAGGPGS(SEQ ID NO: 27); RADAAAA(SEQ ID NO: 28); SAKTTPKLEEGEFSEARV(SEQ ID NO: 29); ADAAP(SEQ ID NO: 30) ; DAAPTVSIFPP(SEQ ID NO: 31); TVAAP(SEQ ID NO: 32); TVAAPSVFIFPP(SEQ ID NO: 33); QPKAAP(SEQ ID NO: 34); QPKAAPSVTLFPP(SEQ ID NO: 35); AKTTPP(SEQ ID NO: 36); AKTTPPSVTPLAP(SEQ ID NO: 37); AKTTAP(SEQ ID NO: 38); AKTTAPSVYPLAP(SEQ ID NO: 39); ASTKGP(SEQ ID NO: 40); ASTKGPSVFPLAP(SEQ ID NO: 41) ; GGGGSGGGGSGGGGS(SEQ ID NO: 42); GENKVEYAPALMALS(SEQ ID NO: 43); GPAKELTPLKEAKVS(SEQ ID NO: 44); GHEAAAVMQVQYPAS(SEQ ID NO: 45); GGGGSGGGGSGGGGSA(SEQ ID NO: 46).

### Multi-targeting fusion protein

Provided herein are multi-targeting fusion protein comprising (i) a vascular endothelial growth inhibitor domain; (ii) an immunoglobulin Fc domain; and (iii) a CD80 ECD multi-targeting fusion protein in any order, including, but not limited to, the multi-targeting fusion protein is effectively linked in the order of (I), (II) and (III); the order of (III), (II) and (II); or the order of (III), (II), and (I) from N-terminal to C-terminal.

In one embodiment, the multi-targeting fusion protein comprises a full-length anti-VEGF antibody, a full-length anti-VEGFR antibody, or a full-length anti-VEGF and VEGFR bispecific antibody from the N-terminal to the C-terminal; and one CD80 ECD effectively linked to the C-terminal of each of the two heavy chains of the antibody.

In another embodiment, the multi-targeting fusion protein comprises a full-length anti-VEGF antibody, a full-length anti-VEGFR antibody, or a full-length anti-VEGF and VEGFR bispecific antibody; a CD80 ECD that is operatively linked to the N-terminal of each heavy chain of the two heavy chains of the antibody; and one CD80 ECD that is effectively connected to the N-terminal of each of the two light chains of the antibody.

In yet another embodiment, the multi-targeting fusion protein comprises CD80 ECD from N-terminal to C-terminal; an immunoglobulin Fc domain in dimer form effectively linked at the C-terminal of CD80 ECD; and an antigen-binding fragment derived from an anti-VEGF antibody and/or an anti-VEGFR antibody is effectively linked to the C-terminal of the immunoglobulin Fc domain in dimer form.

In yet another embodiment, the multi-targeting fusion protein comprises CD80 ECD from N-terminal to C-terminal; a dimer-form immunoglobulin Fcdomain is effectively linked at C-terminal of CD80 ECD; and a VEGFR extracellular receptor functional domain effectively connected to the C-terminal of the immunoglobulin Fc domain in dimer form.

III. Production and purification of the multi-targeting fusion protein of the present invention The multi-targeting fusion protein of the present invention can be obtained, for example, by solid phase peptide synthesis (for example, Merrifield solid phase synthesis) or recombinant production. The polynucleotides encoding each subunit of the multi-targeting fusion protein are separated and inserted into one or more vectors for further cloning and/or expression in host cells for recombinant production. The polynucleotides can be easily separated and sequenced using conventional methods. In one embodiment, a vector comprising one or more polynucleotides of the present invention is provided, preferably an expression vector.

The expression vector can be constructed using methods well known to those skilled in the art. Expression vectors include but are not limited to viruses, plasmids, cosmids, λ phage or yeast artificial chromosomes (YAC). In a preferred embodiment, a glutamine synthetase high-efficiency expression vector with double expression cassettes is used.

Once an expression vector containing one or more polynucleotides of the present invention has been prepared for expression, the expression vector can be transfected or introduced into a suitable host cell. Various techniques can be used to achieve this purpose, for example, protoplast fusion, calcium phosphate precipitation, electroporation, retroviral transduction, viral transfection, gene gun, liposome-based transfection or other conventional techniques.

In one embodiment, a host cell comprising one or more polynucleotides of the invention is provided. In some embodiments, a host cell comprising an expression vector of the invention is provided. As used herein, the term "host cell" refers to any kind of cellular system that can be engineered to produce the multi-targeting fusion protein of the present invention. Host cells suitable for replicating and supporting the expression of the multi-targeting fusion protein of the present invention are well known in the art. Depending on the needs, such cells can be transfected or transduced with a specific expression vector, and a large number of cells containing the vector can be cultivated for inoculating a large-scale fermenter to obtain a sufficient amount of the multi-targeting fusion protein of the present invention for clinical applications. Suitable host cells include prokaryotic microorganisms, such as Escherichia coli, eukaryotic microorganisms such as filamentous fungi or yeast, or various eukaryotic cells, such as Chinese hamster ovary cells (CHO), insect cells and the like. A mammalian cell line suitable for suspension culture can be used. Examples of useful mammalian host cell lines include SV40 transformed monkey kidney CV1 line (COS-7); human embryonic kidney line (HEK 293 or 293F cells), baby hamster kidney cells (BHK), monkey kidney cells (CV1), African green monkey kidney cells (VERO-76), human cervical cancer cells (HELA), canine kidney cells (MDCK), Buffalo rat liver cells (BRL3A), human lung cells (W138), human liver cells (Hep G2)), CHO cells, NSO cells, myeloma cell lines such as YO, NS0, P3X63 and Sp2/0. For a review of mammalian host cell lines suitable for protein production, see, for example, Yazaki and Wu, Methods in Molecular Biology, Volume 248 (Edited by B.K.C.Lo, Humana Press, Totowa, NJ), pages 255-268 (2003). In a preferred embodiment, the host cell is a CHO, HEK293 or NSO cell.

Standard techniques for expressing foreign genes in these host cell systems are known in the art. In one embodiment, a method is provided for producing the multi-targeting fusion protein of the present invention, wherein the method comprises culturing the host cell as provided herein under conditions suitable for expressing the multi-targeting fusion protein, the host cell contains a polynucleotide encoding the multi-targeting fusion protein, and the multi-targeting fusion protein is recovered from the host cell (or host cell culture medium).

The multi-targeting fusion protein prepared as described herein can be purified by known existing techniques such as high performance liquid chromatography, ion exchange chromatography, gel electrophoresis, affinity chromatography, size exclusion chromatography and the like. The actual conditions used to purify a particular protein also depend on factors such as net charge, hydrophobicity, hydrophilicity, etc., and these will be obvious to those skilled in the art.

The purity of the multi-targeting fusion protein of the present invention can be determined by any one of a variety of well-known analytical methods, including gel electrophoresis, high-performance liquid chromatography, and the like. The physical/chemical properties and/or biological activity of the multi-targeting fusion protein provided herein can be identified, screened or characterized by various assays known in the art.

### IV. Combination therapy

PD-1 is an immunosuppressive protein with two ligands, PD-L1 and PD-L2. It is known that the interaction between PD-1 and PD-L1 leads to, for example, a decrease in tumor infiltrating lymphocytes and/or immune evasion of cancer cells. Immunosuppression can be reversed by inhibiting the local interaction of PD-1 with PD-L1 or PD-L2; when the interaction of PD-1 with PD-L2 is also blocked, the effect is additive (Iwai Y. et al., Involvement of PD-L1 on tumor cells in the escape from host immune system and tumor immunotherapy by PD-L1 blockade, Proc.Nat'l. Acad. Sci. USA, 2002, 99: 12293-7). In view of the importance of the signal transduction of the immune checkpoint PD-1 in regulating the immune response, the present invention has developed a pharmaceutical composition for combination therapy, which comprises the multi-targeting fusion protein of the present invention and an anti-PD-1 antibody.

Compared with monotherapy using the multi-targeting fusion protein of the present invention or anti-PD-1 antibody monotherapy, the pharmaceutical composition for combination therapy described herein can provide superior beneficial effects, such as enhanced anti-cancer effects, reduced toxicity and/or reduced side effects. For example, the multi-targeting fusion protein and/or anti-PD-1 antibody of the present invention in the pharmaceutical composition can be administered at a lower dose or shorter administration time required to achieve the same therapeutic effect than monotherapy administration. Therefore, the present invention also discloses the use of a pharmaceutical composition for combination therapy to treat cancer. The effectiveness of the aforementioned pharmaceutical composition can be tested in cell models and animal models known in the art.

The anti-PD-1 antibody contained in the combination therapy can be any anti-PD-1 antibody, as long as it can inhibit or reduce the binding of PD-1 to its ligand, including anti-PD-1 antibodies known in the prior art and anti-PD-1 antibody developed in the future. The anti-PD-1 antibody can specifically bind to PD-1 with high affinity, for example, with a KD of 10⁻⁸M or less, preferably with a KD of 10⁻⁹M to 10⁻¹²M, and thereby block the signal transduction pathway mediated by binding PD-1 to the ligand PD-L1 and/or PD-L2.

Table 7 herein below provides examples of paired heavy chain variable region (VH) and light chain variable region (VL) of the anti-PD-1 antibody included in the combination therapy of the present invention. In some embodiments, the anti-PD-1 antibody in the combination therapy of the present invention comprises a sequence substantially identical to the amino acid sequence shown in Table 7, for example, a sequence having at least 90%, 91%, 92%, 93%, 94%, 95%, 96%, 97%, 98%, 99% or moresequence identity to the paired heavy chain variable region sequence shown in Table 7.

**Table 7. Examples of sequences of heavy chain variable region and light chain variable region of anti-PD-1 antibody in combination therapy**

| Variable region | Amino acid sequence | Sequ ence number (SEQ ID NO:) |
|---|---|---|
| VH | QVQLVESGGGVVQPGRSLRLDCKASGITFSNSGMHWVRQAPG KGLEWVAVIWYDGSKRYYADSVKGRFTISRDNSKNTLFLQMN SLRAEDTAVYYCATNDDYWGQG | 47 |
| VL | EIVLTQSPATLSLSPGERATLSCRASQSVSSYLAWYQQKPGQAP | 48 |
| | RLLIYDASNRATGIPARFSGSGSGTDFTLTISSLEPEDFAVYYCQ QSSNWPRTFGQG | |
| VH | QVQLVQSGVEVKKPGASVKVSCKASGYTFTNYYMYWVRQAP GQGLEWMGGINPSNGGTNFNEKFKNRVTLTTDSSTTTAYMEL KSLQFDDTAVYYCARRDYRFDMGFDYWGQG | 49 |
| VL | EIVLTQSPATLSLSPGERATLSCRASKGVSTSGYSYLHWYQQKP GQAPRLLIYLASYLESGVPARFSGSGSGTDFTLTISSLEPEDFAV YYCQHSRDLPLTFGGG | 50 |
| VH | QVQLVQSGSELKKPGASVKISCKASGYTFTNYGMNWVRQAPG QGLQWMGWINTDSGESTYAEEFKGRFVFSLDTSVNTAYLQITS LTAEDTGMYFCVRVGYDALDYWGQG | 51 |
| VL | EIVLTQSPSSLSASVGDRVTITCSARSSVSYMHWFQQKPGK APKLWIYRTSNLASGVPSRFSGSGSGTSYCLTINSLQPEDFATYY CQQRSSFPLTFGGG | 52 |
| VH | EVQLVESGGGLVQPGGSLRLSCAASGFTFSSYMMSWVRQAPG KGLEWVATISGGGANTYY | 53 |
| | PDSVKGRFTISRDNAKNSLYLQMNSLRAEDTAVYYCARQLYYF DYWGQG | |
| VL | DIQMTQSPSSLSASVGDRVTITCLASQTIGTWLTWYQQKPGKAP KLLIYTATSLADGVPS | 54 |
| VH | RFSGSGSGTDFTLTISSLQPEDFATYYCQQVYSIPWTFGGG QVTLKESGPALVKPTQTLTLTCTFSGFSLSTSGTCVSWIRQPPGK ALEWLATICWEDSKGYNPSLKSRLTISKDTSKNQAVLTMTNMD | 55 |
| VL | PVDTATYYCARREDSGYFWFPYWGQG NIQMTQSPSSLSASVGDRVTITCKAGQNVNNYLAWYQQKPGK APKVLIFNANSLQTGVPSRFSGSGSGTDFTLTISSLQPEDFATYY CQQYNSWTTFGGG | 56 |
| VH | QVQLQESGPGLVKPSETLSLTCTVSGFSLTSYGVHWIRQPPGKG LEWLGVIWAGGSTNYNPSLKSRLTISKDNSKSQVSLKMSSVTA | 57 |
| | ADTAVYYCARAYGNYWYIDVWGQG | |
| VL | DIVMTQSPDSLAVSLGERATINCKASQSVSNDVAWYQQKPGQP PKLLINYAFHRFTGVPDRFSGSGYGTDFTLTISSLQAEDVAVYY CHQAYSSPYTFGGG | 58 |
| VH | VQLVESGGGVVQPGRSLRLDCKASGITFSNYGMHWVRQAPGK GLEWVAVIWYDSSRKYYADSVKGRFTISRDNSKNTLFLQMNSL RAEDTAVYYCATNNDYWGQG | 59 |
| VL | DIQMTQSPSSLSASVGDRVTITCRASQSVSNYLDWYQQKPGKA PKLLIYDASTRATGVPSRFSGSGSGTDFTLTISSLQPEDFATYYC QQNMQLPLTFGQG | 60 |
| VH | DLVQSGAEVKKPGASVKVSCKASGYTFTSYGISWVRQAPGQG LEWMGWISAYNGNTNYAQKLQGRVTMTTDTSTSTAYMELRSL RSDDTAVYYCARGRGYSYGIDAFDIWGQG | 61 |
| VL | LSYVLTQPPSVSVSPGQTARITCSGDALPKQYAYWYQQKPGQA PVLVIYKDSERPSGIPERFSGSSSGTTVTLTISGVQAEDEADYYC QSADSSGTYVVFGGG | 62 |
| VH | QGQLVQSGAEVKKPGASVKVSCKASGYTFTDYEMHWVRQAP GQGLEWMGVIESETGGTAYNQKFQGRVTLTADKSSSTAYMEL SSLRSEDTAVYYCTREGITTVATTYYWYFDVWGQG | 63 |
| VL | DVVMTQSPLSLPVTLGQPASISCRSSQSIVHSNGNTYLEWYLQK PGQSPQLLIYKVSNRFSGVPDRFSGSGSGTDFTLKISRVEAEDVG VYYCFQGSHVPLTFGQG | 64 |
| VH | EVQLVESGGGLVQPGGSLRLSCAASGFTFSSYGMSWVRQAPGK GLEWVATISGGGSDTYYADSVKGRFTISRDNSKNTLYLQMNSL RAEDTAVYYCARQLNYAWFAYWGQG | 65 |
| VL | DIVLTQSPASLAVSPGQRATITCRASESVDNYGISFMNWYQQKP GQPPKLLIYTSSNKDTGVPARFSGSGSGTDFTLTINPMEAEDTA VYYCQQSKEVPWTFGGG | 66 |
| VH | QVQLVQSGAEVKKPGSSVKVSCKASGGTFSSYAISWVRQAPGQ | 67 |
| | GLEWMGLIIPMFDTAGYAQKFQGRVAITVDESTSTAYMELSSL | |
| VL | RSEDTAVYYCARAEHSSTGTFDYWGQG DIQMTQSPSSVSASVGDRVTITCRASQGISSWLAWYQQKPGKA PKLLISAASSLQSGVPSRFSGSGSGTDFTLTISSLQPEDFATYYCQ QANHLPFTFGGG | 68 |
| VH | QLQLQESGPGLVKPSETLTLTCTVSADSISSTTYYWVWIRQPPG KGLEWIGSISYSGSTYYNPSLKSRVTVSVDTSKNQFSLKLNSVA ATDTALYYCARHLGYNGRYLPFDYWGQG | 69 |
| VL | QSALTQPASVSGSPGQSITISCTGTSSDVGFYNYVSWYQQHPGK APELMIYDVSNRPSGVSDRFSGSKSGNTASLTISGLQAEDEADY YCSSYTNISTWVFGGG | 70 |
| VH | QVQLVESGGGVVQPGRSLRLTCKASGLTFSSSGMHWVRQAPG KGLEWVAVIWYDGSKRYYADSVKGRFTISRDNSKNTLFLQMN SLRAEDTAVYYCATNNDYWGQG | 71 |
| VL | EIVLTQSPATLSLSPGERATLSCRASQSVSSYLAWYQQKPGQAP RLLIYTASNRATGIPARFSGSGSGTDFTLTISSLEPEDFAVYYCQ QYSNWPRTFGQG | 72 |

In one embodiment, the anti-PD-1 antibody in the combination therapy of the present invention comprises all heavy chain CDRs and light chain CDRs contained in paired heavy chain variable region sequence/light chain variable region sequence selected from SEQ ID NO: 47/48, 49/50, 51/52, 53/54, 55/56, 57/58, 59/60, 61/62, 63/64, 65/66, 67/68, 69/70, and 71/72. Methods and techniques for identifying the CDRs in the amino acid sequence of the heavy chain variable region and the light chain variable region are known in the art, and can be used to identify CDR of the amino acid sequence in the specific heavy chain variable region and/or light chain the variable region disclosed herein. Exemplary well-known techniques that can be used to identify CDR boundary include, for example, the Kabat definition method, the Chothia definition method, and the AbM definition method. See, for example, Kabat, Sequences of Proteins of Immunological Interest, National Institutes of Health, Bethesda, Md. (1991); Al-Lazikano et al. Standard Modifications for the Canopy Structures of Immunoglobalins. J Mol. Biol.273: 927-948 (1997); and Martin Ac et al. Modeling of Antibody Hypervariable Loops: A combined Algorithm, Proc. Natl. Acad. Sci. USA 86: 9268-9272 (1989).

In one embodiment, the anti-PD-1 antibody in the combination therapy of the present invention is selected from Nivolumab, Pidilizumab and Pembrolizumab.

The pharmaceutical composition of the present invention may contain a "therapeutically effective amount" or a "prophylactically effective amount" of the multi-targeting fusion protein of the present invention and an anti-PD-1 antibody. "Therapeutically effective amount" refers to the amount that is effective to achieve the desired therapeutic result at the required dose and for the required period of time. The therapeutically effective amount can be varied according to various factors such as disease state, age, sex and weight of the individual. A therapeutically effective amount is any amount whose toxic or harmful effect is not as good as the therapeutically beneficial effect. Relative to an untreated subject, a "therapeutically effective amount" preferably inhibits a measurable parameter (such as tumor growth rate) by at least about 20%, more preferably at least about 40%, even more preferably at least about 60%, and still more preferably at least about 80%. The ability of the pharmaceutical composition of the present invention to inhibit a measurable parameter (e.g., tumor volume) can be evaluated in an animal model system that predicts efficacy in human tumors.

"Prophylactically effective amount" refers to an amount that effectively achieves the desired preventive result at the required dose and for the required period of time. Generally, since the prophylactic dose is used in the subject before or in the early stage of the disease, the prophylactically effective amount is less than the therapeutically effective amount.

The multi-targeting fusion protein and anti-PD-1 antibody treatment regimens in the combination therapy of the present invention can be synergistic with each other as demonstrated in the examples in tumor immunity. Therefore, the pharmaceutical composition of the present invention is advantageous for preventing immune escape of tumors. Co-administration of the pharmaceutical composition comprising the multi-targeting fusion protein and anti-PD-1 antibody of the present invention can be implemented by independently administering the multi-targeting fusion protein or anti-PD-1 antibody, or combined preparation of the multi-targeting fusion protein and anti-PD-1 antibody can be administered in a single time. The administration of the multi-targeting fusion protein and anti-PD-1 antibody in the combination therapy of the present invention allows flexibility in dosage and time course.

### V. Use of multi-targeting fusion protein and pharmaceutical composition

The multi-targeting fusion protein and pharmaceutical composition disclosed herein have therapeutic and preventive uses for cancer. For example, the multi-targeting fusion protein and its composition with an anti-PD-1 antibody can be administered to cultured cells in vitro or ex vivo or to a subject, for example, a human subject, to treat and/or prevent multiple kind of cancerous disease.

In one aspect, the present invention relates to the use of a multi-targeting fusion protein or the pharmaceutical composition of the present invention to treat or prevent diseases that require blocking the growth of vascular endothelial cells and activating T cells in a subject, thereby inhibiting or reducing related diseases such as the growth or emergence, metastasis or recurrence of cancerous tumors. Multi-targeting fusion proteins can be used alone to inhibit the growth of cancerous tumors or prevent their appearance. Alternatively, the multi-targeting fusion protein can be administered in combination with other cancer therapeutic/prophylactic agents (e.g., anti-PD-1 antibodies). When the multi-targeting fusion protein of the present invention is administered with an anti-PD-1 antibody, this combination can be administered in any order or simultaneously.

Therefore, in one embodiment, the present invention provides a method of inhibiting the growth of tumor cells in a subject, and the method comprises administering to the subject a therapeutically effective amount of the multi-targeting fusion protein or pharmaceutical composition described herein. In another embodiment, the present invention provides a method for preventing the appearance or metastasis or recurrence of tumor cells in a subject, and the method comprises administering to the subject a prophylactically effective amount of the multi-targeting fusion protein described herein or pharmaceutical composition.

In some embodiments, cancers treated and/or prevented with the multi-targeting fusion protein or pharmaceutical composition include, but are not limited to, solid tumors, hematological cancers (e.g., leukemia, lymphoma, myeloma, e.g., multiple myeloma) and metastatic lesions. In one embodiment, the cancer is a solid tumor. Examples of solid tumors include malignant tumors, for example, sarcomas and cancers of multiple organ systems, such as those that invade the lung, breast, ovary, lymphoid, gastrointestinal (e.g., colon), anus, genitals, and genitourinary tract (e.g., kidney, bladder epithelium, bladder cells, prostate), pharynx, CNS (e.g., brain, neural or glial cells), head and neck, skin (e.g., melanoma), nasopharyngeal (e.g., differentiated or undifferentiated metastatic or locally recurrent nasopharyngeal carcinoma) and those of the pancreas, as well as adenocarcinomas, including malignant tumors such as colon cancer, rectal cancer, renal cell carcinoma, liver cancer, non-small cell lung cancer, small intestine cancer and esophageal cancer. The cancer can be early, middle or late or metastatic cancer.

In some embodiments, the cancer is selected from melanoma, breast cancer, colon cancer, esophageal cancer, gastrointestinal stromal tumor (GIST), kidney cancer (e.g., renal cell carcinoma), liver cancer, non-small cell lung cancer (NSCLC), ovarian cancer, pancreatic cancer, prostate cancer, head and neck tumors, gastric cancer, hematological malignancies (e.g., lymphoma).

The following examples are described to assist the understanding of the present invention. It is not intended and should not be construed in any way to limit the scope of protection of the present invention.

### EXAMPLES

Example 1. Construction of high-efficiency glutamine synthetase expression vectors containing the target gene

### (1) Synthesis of nucleotides encoding anti-PD1 Antibody BY18.1 and construction of expression vectors

According to the amino acid sequence data of nivolumab numbered 9623 in the International Nonproprietary Name (INN) database, it was optimized to the following nucleotide sequences suitable for expression in Chinese hamster ovarian cancer cells (CHO), and the nucleotide sequences were entrusted to synthesize by Shanghai Jierui Biotechnology Engineering Co., Ltd. The anti-PD1 antibody produced after the expression of the nucleotide sequence is referred to herein as the antibody BY18.1.

The nucleotide sequence (SEQ ID NO: 73) of the light chain (BY18.1L) of the anti-PD1 antibody BY18.1:

CTCGAGGCCACCATGGAGACCGACACACTCCTCCTGTGGGTGCTGCTGCTGTGGGT
GCCTGGCTCCACTGGCGAGATTGTGCTGACACAGTCCCCCGCTACTCTGAGCCTGAGCC
CTGGCGAGAGGGCTACACTGTCTTGCAGAGCTTCTCAGTCCGTGTCTTCTTACCTCGCT
TGGTATCAGCAGAAGCCCGGCCAGGCTCCAAGACTGCTGATCTATGACGCTTCTAACC
GCGCTACAGGCATTCCTGCTAGGTTCAGCGGCAGCGGCTCTGGCACCGACTTCACACTC
ACAATTAGCTCTCTTGAACCTGAGGACTTCGCCGTGTACTACTGCCAGCAGTCTAGCAA
CTGGCCTAGAACATTCGGCCAGGGCACTAAGGTGGAGATTAAGAGAACCGTGGCCGCC

CCCAGCGTGTTCATCTTCCCTCCCAGCGACGAGCAGCTGAAGTCTGGCACCGCCAGCGT
GGTGTGCCTGCTGAACAACTTCTACCCCCGCGAGGCCAAGGTGCAGTGGAAGGTGGAC
AACGCCCTGCAGAGCGGCAACAGCCAGGAGAGCGTGACCGAGCAGGACTCCAAGGAC
AGCACCTACAGCCTGAGCAGCACCCTGACCCTGAGCAAGGCCGACTACGAGAAGCAC
AAGGTGTACGCCTGCGAGGTGACCCACCAGGGACTGTCTAGCCCCGTGACCAAGAGCT
TCAACCGGGGCGAGTGCTAAGAATTC

The amino acid sequence (SEQ ID NO: 74) of the light chain (BY18.1L) of the anti-PD1 antibody BY18.1:

METDTLLLWVLLLWVPGSTGEIVLTQSPATLSLSPGERATLSCRASQSVSSYLAWYQQ
KPGQAPRLLIYDASNRATGIPARFSGSGSGTDFTLTISSLEPEDFAVYYCQQSSNWPRTFGQ
GTKVEIKRTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

The nucleotide sequence (SEQ ID NO: 75) of the heavy chain (BY18.1H) of the anti-PD1 antibody BY18.1:

TCTAGAGCCACCATGGAGACCGACACCCTGCTGCTGTGGGTGCTGCTCCTGTGGGT
GCCTGGCTCCACAGGCCAGGTGCAGCTCGTGGAGTCCGGCGGCGTGGTGCAGCCC
GGCAGATCCCTCAGACTGGACTGCAAGGCATCCGGCATTACATTCTCTAACTCTGGAAT
GCACTGGGTGAGACAGGCTCCTGGCAAGGGCCTGGAATGGGTGGCCGTGATTTGGTAC
GACGGCTCTAAGAGATACTACGCTGACTCCGTGAAGGGCCGGTTCACAATTAGCAGAG
ACAACTCCAAGAACACTCTGTTCCTCCAGATGAACAGCCTGAGAGCCGAGGACACCGC
TGTGTACTACTGCGCCACCAACGACGACTACTGGGGCCAGGGCACCCTCGTGACAGTG
TCTTCCGCCTCCACCAAGGGCCCTTCCGTGTTCCCTCTGGCCCCTTGCTCCCGCTCCACC
TCCGAGTCCACCGCCGCCCTGGGCTGCCTGGTGAAGGACTACTTCCCTGAGCCTGTGAC
CGTGTCCTGGAACTCCGGCGCCCTGACCTCCGGCGTGCACACCTTCCCTGCCGTGCTGC
AGTCCTCCGGCCTGTACTCCCTGTCCTCCGTGGTGACCGTGCCTTCCTCCTCCCTGGGCA
CCAAGACCTACACCTGCAACGTGGACCACAAGCCTTCCAACACCAAGGTGGACAAGCG
CGTGGAGTCCAAGTACGGCCCTCCTTGCCCTCCTTGCCCTGCCCCTGAGTTCCTGGGCG
GCCCTTCCGTGTTCCTGTTCCCTCCTAAGCCTAAGGACACCCTGATGATCTCCCGCACC
CCTGAGGTGACCTGCGTGGTGGTGGACGTGTCCCAGGAGGACCCTGAGGTGCAGTTCA
ACTGGTACGTGGACGGCGTGGAGGTGCACAACGCCAAGACCAAGCCTCGCGAGGAGC

AGTTCAACTCCACCTACCGCGTGGTGTCCGTGCTGACCGTGCTGCACCAGGACTGGCTG
AACGGCAAGGAGTACAAGTGCAAGGTGTCCAACAAGGGCCTGCCTTCCTCCATCGAGA
AGACCATCTCCAAGGCCAAGGGCCAGCCTCGCGAGCCTCAGGTGTACACCCTGCCTCC
TTCCCAGGAGGAGATGACCAAGAACCAGGTGTCCCTGACCTGCCTGGTGAAGGGCTTC
TACCCTTCCGACATCGCCGTGGAGTGGGAGTCCAACGGCCAGCCTGAGAACAACTACA
AGACCACCCCTCCTGTGCTGGACTCCGACGGCTCCTTCTTCCTGTACTCCCGCCTGACC
GTGGACAAGTCCCGCTGGCAGGAGGGCAACGTGTTCTCCTGCTCCGTGATGCACGAGG
CCCTGCACAACCACTACACCCAGAAGTCCCTGTCCCTGTCCCTGGGCAAGTAA
GTCGAC

The heavy chain (BY18.1H) amino acid sequence (SEQ ID NO: 76) of the anti-PD1 antibody BY18 1:METDTLLLWVLLLWVPGSTGQVQLVESGGGVVQPGRSLRLDCKASGITFSNSGM HWVRQAPGKGLEWVAVIWYDGSKRYYADSVKGRFTISRDNSKNTLFLQMNSLRAEDTAV YYCATNDDYWGQGTLVTVSSASTKGPSVFPLAPCSRSTSESTAALGCLVKDYFPEPVTVS WNSGALTSGVHTFPAVLQSSGLYSLSSVVTVPSSSLGTKTYTCNVDHKPSNTKVDKRVESK YGPPCPPCPAPEFLGGPSVFLFPPKPKDTLMISRTPEVTCVVVDVSQEDPEVQFNWYVDGV EVHNAKTKPREEQFNSTYRVVSVLTVLHQDWLNGKEYKCKVSNKGLPSSIEKTISKAKGQ PREPQVYTLPPSQEEMTKNQVSLTCLVKGFYPSDIAVEWESNGQPENNYKTTPPVLDSDGS FFLYSRLTVDKSRWQEGNVFSCSVMHEALHNHYTQKSLSLSLGK

Wherein the underlined part "METDTLLLWVLLLWVPGSTG" is a signal peptide sequence.

The above nucleotide sequences encoding BY18.1L and nucleotide sequences encoding BY18.1H were synthesized by Shanghai Jierui Bioengineering Co., Ltd. The nucleotides encoding BY18.1L were respectively digested with XhoI-EcoRI double enzymes, and the high-efficiency glutamine synthetase expression vectors (Patent No.: CN104195173B, obtained from Beijing Biyang Biotechnology Co., Ltd.) were digested with XhoI -EcoRI double enzymes, and then nucleotides encoding BY18.1L digested by XhoI-EcoRI double enzymes were linked by ligase to glutamine synthetase high-efficiency expression vectors having double expression cassettes digested by XhoI-EcoRI double enzymes, and the high-efficiency glutamine synthetase expression vectors having double expression cassettes which have been introduced into nucleotide encoding BY18.1L were obtained; and then, the nucleotides encoding BY18.1H were respectively digested with Xbal-Sall double enzymes, and the high-efficiency glutamine synthetase expression vectors were digested with Xbal-Sall double enzymes, and then nucleotides encoding BY18.1H digested by Xbal-Sall double enzymes were linked by ligase to glutamine synthetase high-efficiency expression vectors having double expression cassettes digested by Xbal-Sall double enzymes, thereby the high-efficiency glutamine synthetase expression vectors having double expression cassettes which have been introduced into nucleotides encoding BY18.1H were obtained, after the correct expression verified by sequencing the anti-PD1 antibodies BY18.1 were obtained.

Alternatively, nucleotides encoding BY18.1L can also be ligated into high-efficiency glutamine synthetase high-efficiency expression vectors with double expression cassette which have been introduced into nucleotides encoding BY18.1H, and the antibodies BY18.1 were expressed and obtained.

### (2)Synthesis of nucleotides encoding fusion protein containing CD80 extracellular domain and immunoglobulin Fc region and construction of expression vectors

According to the sequence of the CD80 extracellular domain in Table 1, and the IgG4Fc sequence in Table 6 were optimized to nucleotide sequences suitable for expression in Chinese Hamster Ovarian Cancer Cells (CHO), and polynucleotide sequences SEQ ID NO: 77 as follows were entrusted to synthesize by Shanghai Jierui Bioengineering Co., Ltd. The CD80-Fc fusion protein produced after the expression of the nucleotide sequence is also referred to herein as the fusion protein BY31.19.

Nucleotide sequence of the fusion protein BY31.19 (CD80-Fc, IgG4) (SEQ ID NO: 77)

CTCGAGGCCACCATGGAGACCGACACACTCCTCCTGTGGGTGCTGCTGCTGTGGGT
GCCTGGCTCCACTGGCGTCATCCATGTGACCAAAGAGGTCAAAGAAGTCGCAACTCTG
TCTTGCGGACACAACGTCTCCGTCGAAGAACTCGCCCAGACTAGAATATATTGGCAGA
AGGAAAAGAAGATGGTGCTCACCATGATGTCCGGCGATATGAACATTTGGCCTGAGTA
CAAGAACCGGACAATCTTCGATATTACTAATAACCTGAGTATCGTGATTCTCGCTCTGC
GCCCTAGCGACGAGGGCACATACGAGTGTGTGGTGCTGAAGTACGAGAAGGATGCATT
CAAGCGGGAGCACCTCGCAGAGGTGACACTCTCCGTGAAGGCCGACTTCCCAACCCCA
TCTGACAAGCGCGTGGAGTCCAAGTACGGCCCTCCTTGCCCTCCTTGCCCTGCCCCTGA
GTTCCTGGGCGGCCCTTCCGTGTTCCTGTTCCCTCCTAAGCCTAAGGACACCCTGATGA
TCTCCCGCACCCCTGAGGTGACCTGCGTGGTGGTGGACGTGTCCCAGGAGGACCCTGA
GGTGCAGTTCAACTGGTACGTGGACGGCGTGGAGGTGCACAACGCCAAGACCAAGCCT
CGCGAGGAGCAGTTCAACTCCACCTACCGCGTGGTGTCCGTGCTGACCGTGCTGCACC
AGGACTGGCTGAACGGCAAGGAGTACAAGTGCAAGGTGTCCAACAAGGGCCTGCCTTC
CTCCATCGAGAAGACCATCTCCAAGGCCAAGGGCCAGCCTCGCGAGCCTCAGGTGTAC

ACCCTGCCTCCTTCCCAGGAGGAGATGACCAAGAACCAGGTGTCCCTGACCTGCCTGG
TGAAGGGCTTCTACCCTTCCGACATCGCCGTGGAGTGGAGTCCAACGGCCAGCCTGA
GAACAACTACAAGACCACCCCTCCTGTGCTGGACTCCGACGGCTCCTTCTTCCTGTACT
CCCGCCTGACCGTGGACAAGTCCCGCTGGCAGGAGGGCAACGTGTTCTCCTGCTCCGT
GATGCACGAGGCCCTGCACAACCACTACACCCAGAAGTCCCTGTCCCTGTCCCTGGGC
TAAGAATTC

The amino acid sequence of the fusion protein BY31.19 (CD80-Fc, IgG4) (SEQ ID NO: 78)

METDTLLLWVLLLWVPGSTGVIHVTKEVKEVATLSCGHNVSVEELAQTRIYWQKEK
KMVLTMMSGDMNIWPEYKNRTIFDITNNLSIVILALRPSDEGTYECVVLKYEKDAFKREHL
AEVTLSVKADFPTPSDKRVESKYGPPCPPCPAPEFLGGPSVFLFPPKPKDTLMISRTPEVTCV
VVDVSQEDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNHYTQKSLS
LSLG

Wherein the amino acid sequence "METDTLLLWVLLLWVPGSTG" is a signal peptide.

Using the same method as in Example 1(1) above, the nucleotides encoding BY31.19 digested by XhoI-EcoRI double enzymes were linked to glutamine synthetase high-efficiency expression vectors with double expression cassettes (Patent No.: CN104195173B, obtained From Beijing Biyang Biotechnology Co., Ltd.). The recombinant vectors were sequenced to verify that it was correct and used for CD80-Fc fusion protein expression. The expressed CD80-Fc fusion protein was named fusion protein BY31.19.

### (3)Synthesis of nucleotides encoding fusion protein BY24.4 containing anti-VEGF antibody and CD80 extracellular domain and construction of expression vectors

According to the sequence of the CD80 extracellular domain in Table 1, the heavy chain variable region and light chain variable region sequence of the anti-VEGF antibody in Table 2, the heavy chain constant region sequence of the antibody in Table 3, and the constant region sequence of the light chain of the antibody in Table 4, and the peptide linker sequence of SEQ ID NO: 20-46 were optimized to nucleotide sequences suitable for expression in Chinese hamster ovarian cancer cells (CHO), and Shanghai Jierui Bioengineering Co., Ltd. was entrusted to synthesize the following polynucleotide sequence shown in SEQ ID NO: 79 and 81. The anti-VEGF antibody-CD80 fusion protein produced after the expression of the nucleotide sequence is referred to herein as the fusion protein BY24.4.

The nucleotide sequence of the first subunit (BY24.4L) of the fusion protein BY24.4 (κ, IgG4) (SEQ ID NO: 79)

CTCGAGGCCACCATGGAGACAGATACTCTGCTGCTCTGGGTCCTGCTGCTGTGGGT
GCCAGGCAGCACAGGCGACATCCAGATGACCCAGTCCCCTTCCTCCCTGTCTGCCTCCG
TGGGCGACAGGGTGACCATCACCTGTTCCGCATCCCAGGATATCTCCAACTACCTGAA
CTGGTATCAGCAGAAGCCAGGAAAGGCCCCCAAGGTGCTGATCTACTTTACTTCTTCCC
TGCATTCCGGCGTGCCCTCCAGGTTCTCTGGCTCCGGGTCTGGAACCGACTTTACACTG
ACAATCTCCTCTCTGCAGCCAGAAGACTTTGCCACCTATTACTGCCAGCAGTACTCCAC
TGTCCCCTGGACCTTCGGGCAAGGAACCAAAGTGGAAATCAAGAGGACCGTGGCCGCC
CCCAGCGTGTTCATCTTCCCTCCCAGCGACGAGCAGCTGAAGTCTGGCACCGCCAgCGT
GGTGTGCCTGCTGAACAACTTCTACCCCCGCGAGGCCAAGGTGCAGTGGAAGGTGGAC
AACGCCCTGCAgAGCGGCAACAGCCAGGAGAGCGTGACCGAGCAGGACTCCAAGGAC
AGCACCTACAGCCTGAGCAGCACCCTGACCCTGAGCAAGGCCGACTACGAGAAGCAC
AAGGTGTACGCCTGCGAGGTGACCCACCAGGGACTGTCTAGCCCCGTGACCAAGAGCT
TCAACCGGGGCGAGTGCTAAGAATTC

The amino acid sequence of the first subunit (BY24.4L) of the fusion protein BY24.4 (κ, IgG4) (SEQ ID NO: 80)

METDTLLLWVLLLWVPGSTGDIQMTQSPSSLSASVGDRVTITCSASQDISNYLNWYQQ
KPGKAPKVLIYFTSSLHSGVPSRFSGSGSGTDFTLTISSLQPEDFATYYCQQYSTVPWTFGQ
GTKVEIKRTVAAPSVFIFPPSDEQLKSGTASVVCLLNNFYPREAKVQWKVDNALQSGNSQE
SVTEQDSKDSTYSLSSTLT LSKADYEKHKVYACEVTHQGLSSPVTKSFNRGEC

The nucleotide sequence of the second subunit (BY24.4H) of the fusion protein BY24.4 (κ, IgG4) (SEQ ID NO: 81)

TCTAGAGCCACCATGGAAACCGACACACTGCTGCTGTGGGTCCTGCTGCTGTGGGT
GCCAGGCTCTACAGGCGAAGTGCAGCTGGTCGAATCTGGCGGCGGACTGGTGCAACCA
GGGGGCAGCCTGAGGCTGTCCTGCGCCGCTTCTGGATACACCTTTACCAATTACGGCAT
GAATTGGGTGAGGCAGGCTCCTGGCAAGGGCCTGGAGTGGGTGGGATGGATCAACAC
CTACACCGGCGAGCCCACCTACGCCGCTGACTTTAAGAGGAGGTTCACCTTCTCTCTGG

ACACCTCCAAGTCCACCGCTTACCTGCAAATGAACTCTCTGAGGGCTGAAGACACCGC
CGTGTACTACTGCGCCAAGTACCCCCATTACTACGGGTCCTCCCACTGGTACTTCGACG
TGTGGGGCCAGGGCACCCTCGTGACAGTGTCTTCCGCCTCCACCAAGGGCCCTTCCGTG
TTCCCTCTGGCCCCTTGCTCCCGCTCCACCTCCGAGTCCACCGCCGCCCTGGGCTGCCT
GGTGAAGGACTACTTCCCTGAGCCTGTGACCGTGTCCTGGAACTCCGGCGCCCTGACCT
CCGGCGTGCACACCTTCCCTGCCGTGCTGCAGTCCTCCGGCCTGTACTCCCTGTCCTCC
GTGGTGACCGTGCCTTCCTCCTCCCTGGGCACCAAGACCTACACCTGCAACGTGGACCA
CAAGCCTTCCAACACCAAGGTGGACAAGCGCGTGGAGTCCAAGTACGGCCCTCCTTGC
CCTCCTTGCCCTGCCCCTGAGTTCCTGGGCGGCCCTTCCGTGTTCCTGTTCCCTCCTAAG
CCTAAGGACACCCTGATGATCTCCCGCACCCCTGAGGTGACCTGCGTGGTGGTGGACG
TGTCCCAGGAGGACCCTGAGGTGCAGTTCAACTGGTACGTGGACGGCGTGGAGGTGCA
CAACGCCAAGACCAAGCCTCGCGAGGAGCAGTTCAACTCCACCTACCGCGTGGTGTCC
GTGCTGACCGTGCTGCACCAGGACTGGCTGAACGGCAAGGAGTACAAGTGCAAGGTGT
CCAACAAGGGCCTGCCTTCCTCCATCGAGAAGACCATCTCCAAGGCCAAGGGCCAGCC
TCGCGAGCCTCAGGTGTACACCCTGCCTCCTTCCCAGGAGGAGATGACCAAGAACCAG
GTGTCCCTGACCTGCCTGGTGAAGGGCTTCTACCCTTCCGACATCGCCGTGGAGTGGA
GTCCAACGGCCAGCCTGAGAACAACTACAAGACCACCCCTCCTGTGCTGGACTCCGAC
GGCTCCTTCTTCCTGTACTCCCGCCTGACCGTGGACAAGTCCCGCTGGCAGGAGGGCAA
CGTGTTCTCCTGCTCCGTGATGCACGAGGCCCTGCACAACCACTACACCCAGAAGTCCC
TGTCCCTGTCCCTGGGCGGCGGAGGATCTGGCGGCGGAGGCAGTGGAGGCGGCGGAA
GTGTGATCCATGTAACAAAAGAAGTGAAGGAAGTGGCTACACTCTCTTGCGGCCACAA
CGTGTCCGTGGAGGAACTAGCTCAGACCCGGATCTATTGGCAGAAAGAAAAGAAGAT
GGTGCTGACCATGATGTCCGGCGACATGAACATTTGGCCAGAGTACAAGAACCGCACA
ATTTTCGACATTACAAACAACCTCTCTATTGTGATTCTGGCTCTCAGGCCTAGCGACGA
GGGCACATACGAGTGCGTGGTGCTCAAGTACGAGAAGGACGCTTTCAAGCGGGAGCA
CCTCGCTGAGGTGACCCTGTCCGTGAAGGCCGACTTCCCTACTCCATCTTAAGTCGAC

The amino acid sequence of the second subunit (BY24.4H) of the fusion protein BY24.4 (κ, IgG4) (SEQ ID NO: 82)

METDTLLLWVLLLWVPGSTGEVQLVESGGGLVQPGGSLRLSCAASGYTFTNYGMNW
VRQAPGKGLEWVGWINTYTGEPTYAADFKRRFTFSLDTSKSTAYLQMNSLRAEDTAVYY

CAKYPHYYGSSHWYFDVWGQGTLVTVSSASTKGPSVFPLAPCSRSTSESTAALGCLVKDY
FPEPVTVSWNSGALTSGVHTFPAVLQSSGLYSLSSVVTVPSSSLGTKTYTCNVDHKPSNTK
VDKRVESKYGPPCPPCPAPEFLGGPSVFLFPPKPKDTLMISRTPEVTCVVVDVSQEDPEVQF
NWYVDGVEVHNAKTKPREEQFNSTYRVVSVLTVLHQDWLNGKEYKCKVSNKGLPSSIEK
TISKAKGQPREPQVYTLPPSQEEMTKNQVSLTCLVKGFYPSDIAVEWESNGQPENNYKTTP
PVLDSDGSFFLYSRLTVDKSRWQEGNVFSCSVMHEALHNHYTQKSLSLSLGGGGSGGGGS
GGGGSVIHVTKEVKEVATLSCGHNVSVEELAQTRIYWQKEKKMVLTMMSGDMNIWPEY
KNRTIFDITNNLSIVILALRPSDEGTYECVVLKYEKDAFKREHLAEVTLSVKADFPTPS

Wherein the amino acid sequence "METDTLLLWVLLLWVPGSTG" is a signal peptide.

Using the same method as in Example 1 (1) above, the nucleotides encoding BY24.4L digested by XhoI-EcoRI double enzymes were linked to glutamine synthetase high-efficiency expression vectors with double expression cassette (patent No.: CN104195173B, obtained From Beijing Biyang Biotechnology Co., Ltd.); And then, the nucleotides encoding BY24.4H digested by Xbal-Sall double enzymes were linked to glutamine synthetase high-efficiency expression vectors connected to nucleotides encoding BY24.4L with a double expression cassette; or vice versa. The recombinant vectors were sequenced and verified to be correct then used for expression. The expressed anti-VEGF antibody-CD80 fusion protein was named fusion protein BY24.4.

### (4)Synthesis of nucleotides encoding fusion Protein BY24.5 containing anti-VEGFR antibody and CD80 extracellular domain and construction of expression vectors

According to the sequence of the CD80 extracellular domain in Table 1, the heavy chain variable region and light chain variable region sequence of the anti-VEGFR antibody in Table 2, the heavy chain constant region sequence of the antibody in Table 3, the constant region sequence of the light chain of the antibody in Table 4, and the peptide linker sequence of SEQ ID NO: 20-46 were optimized to nucleotide sequences suitable for expression in Chinese hamster ovarian cancer cells (CHO), and Shanghai Jierui Bioengineering Co., Ltd. was entrusted to synthesize the following polynucleotide sequences shown in SEQ ID NO: 83 and 85. The anti-VEGFR antibody-CD80 fusion protein produced after the expression of the nucleotide sequence is referred to herein as the fusion protein BY24.5.

The nucleotide sequence of the first subunit (BY24.5L) of the fusion protein BY24.5 (κ, IgG4) (SEQ ID NO: 83)

CTCGAGGCCACCATGGAGACAGATACTCTGCTGCTCTGGGTCCTGCTGCTGTGGGT
GCCAGGCAGCACAGGCGACATCCAGATGACCCAGTCTCCTAGCTCCGTGAGCGCCTCT
ATCGGCGACAGAGTGACCATCACATGCAGAGCTTCTCAGGGCATCGACAACTGGCTCG
GCTGGTACCAGCAGAAGCCTGGCAAGGCCCCAAAGCTGCTGATCTACGACGCCTCTAA
CCTGGACACCGGCGTGCCATCCCGGTTCAGCGGCTCTGGCTCCGGCACATACTTCACAC
TCACAATCTCTTCCCTGCAGGCCGAGGACTTCGCTGTGTACTTCTGCCAGCAGGCTAAG
GCTTTCCCACCTACATTCGGCGGCGGAACCAAAGTGGAAATCAAGAGGACCGTGGCCG
CCCCCAGCGTGTTCATCTTCCCTCCCAGCGACGAGCAGCTGAAGTCTGGCACCGCCAgC
GTGGTGTGCCTGCTGAACAACTTCTACCCCCGCGAGGCCAAGGTGCAGTGGAAGGTGG
ACAACGCCCTGCAgAGCGGCAACAGCCAGGAGAGCGTGACCGAGCAGGACTCCAAGG
ACAGCACCTACAGCCTGAGCAGCACCCTGACCCTGAGCAAGGCCGACTACGAGAAGC
ACAAGGTGTACGCCTGCGAGGTGACCCACCAGGGACTGTCTAGCCCCGTGACCAAGAG
CTTCAACCGGGGCGAGTGCTAAGAATTC

The amino acid sequence of the first subunit (BY24.5L) of the fusion protein BY24.5 (κ, IgG4) (SEQ ID NO: 84)

METDTLLLWVLLLWVPGSTGDIQMTQSPSSVSASIGDRVTITCRASQGIDNWLGWYQ
QKPGKAPKLLIYDASNLDTGVPSRFSGSGSGTYFTLTISSLQAEDFAVYFCQQAKAFPPTFG
GGTKVEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC

The nucleotide sequence of the second subunit (BY24.5H) of the fusion protein BY24.5 (κ, IgG4) (SEQ ID NO: 85)

TCTAGAGCCACCATGGAAACCGACACACTGCTGCTGTGGGTCCTGCTGCTGTGGGT
GCCAGGCTCTACAGGCGAGGTGCAGCTCGTGCAGTCTGGCGGCGGCCTCGTGAAGCCA
GGCGGCTCCCTCAGACTGTCTTGCGCTGCTAGCGGCTTCACATTCTCTTCCTACTCTATG
AACTGGGTGAGACAGGCTCCCGGCAAGGGCCTGGAGTGGGTGTCCAGCATCTCTTCCT
CTAGCTCTTACATCTACTACGCCGACTCCGTGAAGGGCAGATTCACAATCAGCCGGGA
CAACGCCAAGAACTCCCTGTACCTGCAGATGAACAGCCTGAGAGCCGAGGACACCGCT
GTGTACTACTGCGCTAGAGTGACCGACGCTTTCGACATCTGGGGCCAGGGCACTCTCGT
GACAGTGTCTTCCGCCTCCACCAAGGGCCCTTCCGTGTTCCCTCTGGCCCCTTGCTCCC
GCTCCACCTCCGAGTCCACCGCCGCCCTGGGCTGCCTGGTGAAGGACTACTTCCCTGAG

CCTGTGACCGTGTCCTGGAACTCCGGCGCCCTGACCTCCGGCGTGCACACCTTCCCTGC
CGTGCTGCAGTCCTCCGGCCTGTACTCCCTGTCCTCCGTGGTGACCGTGCCTTCCTCCTC
CCTGGGCACCAAGACCTACACCTGCAACGTGGACCACAAGCCTTCCAACACCAAGGTG
GACAAGCGCGTGGAGTCCAAGTACGGCCCTCCTTGCCCTCCTTGCCCTGCCCCTGAGTT
CCTGGGCGGCCCTTCCGTGTTCCTGTTCCCTCCTAAGCCTAAGGACACCCTGATGATCT
CCCGCACCCCTGAGGTGACCTGCGTGGTGGTGGACGTGTCCCAGGAGGACCCTGAGGT
GCAGTTCAACTGGTACGTGGACGGCGTGGAGGTGCACAACGCCAAGACCAAGCCTCGC
GAGGAGCAGTTCAACTCCACCTACCGCGTGGTGTCCGTGCTGACCGTGCTGCACCAGG
ACTGGCTGAACGGCAAGGAGTACAAGTGCAAGGTGTCCAACAAGGGCCTGCCTTCCTC
CATCGAGAAGACCATCTCCAAGGCCAAGGGCCAGCCTCGCGAGCCTCAGGTGTACACC
CTGCCTCCTTCCCAGGAGGAGATGACCAAGAACCAGGTGTCCCTGACCTGCCTGGTGA
AGGGCTTCTACCCTTCCGACATCGCCGTGGAGTGGGAGTCCAACGGCCAGCCTGAGAA
CAACTACAAGACCACCCCTCCTGTGCTGGACTCCGACGGCTCCTTCTTCCTGTACTCCC
GCCTGACCGTGGACAAGTCCCGCTGGCAGGAGGGCAACGTGTTCTCCTGCTCCGTGAT
GCACGAGGCCCTGCACAACCACTACACCCAGAAGTCCCTGTCCCTGTCCCTGGGCGGC
GGAGGATCTGGCGGCGGAGGCAGTGGAGGCGGCGGAAGTGTGATCCATGTAACAAAA
GAAGTGAAGGAAGTGGCTACACTCTCTTGCGGCCACAACGTGTCCGTGGAGGAACTAG
CTCAGACCCGGATCTATTGGCAGAAAGAAAAGAAGATGGTGCTGACCATGATGTCCGG
CGACATGAACATTTGGCCAGAGTACAAGAACCGCACAATTTTCGACATTACAAACAAC
CTCTCTATTGTGATTCTGGCTCTCAGGCCTAGCGACGAGGGCACATACGAGTGCGTGGT
GCTCAAGTACGAGAAGGACGCTTTCAAGCGGGAGCACCTCGCTGAGGTGACCCTGTCC
GTGAAGGCCGACTTCCCTACTCCATCTTAAGTCGAC

The amino acid sequence of the second subunit (BY24.5H) of the fusion protein BY24.5 (κ, IgG4) (SEQ ID NO: 86)

METDTLLLWVLLLWVPGSTGEVQLVQSGGGLVKPGGSLRLSCAASGFTFSSYSMNW
VRQAPGKGLEWVSSISSSSSYIYYADSVKGRFTISRDNAKNSLYLQMNSLRAEDTAVYYCA
RVTDAFDIWGQGTLVTVSSASTKGPSVFPLAPCSRSTSESTAALGCLVKDYFPEPVTVSWN
SGALTSGVHTFPAVLQSSGLYSLSSVVTVPSSSLGTKTYTCNVDHKPSNTKVDKRVESKYG
PPCPPCPAPEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISKAKGQPR

EPQVYTLPPSQEEMTKNQVSLTCLVKGFYPSDIAVEWESNGQPENNYKTTPPVLDSDGSFF
LYSRLTVDKSRWQEGNVFSCSVMHEALHNHYTQKSLSLSLGGGGSGGGGSGGGGSVIHVT
KEVKEVATLSCGHNVSVEELAQTRIYWQKEKKMVLTMMSGDMNIWPEYKNRTIFDITNN
LSIVILALRPSDEGTYECVVLKYEKDAFKREHLAEVTLSVKADFPTPS

Wherein the amino acid sequence "METDTLLLWVLLLWVPGSTG" is a signal peptide.

Using the same method as in Example 1 (1) above, the nucleotides encoding BY24.5L digested by XhoI-EcoRI double enzymes were linked to glutamine synthetase high-efficiency expression vectors with double expression cassette (patent No.: CN104195173B, obtained From Beijing Biyang Biotechnology Co., Ltd.); And then, the nucleotides encoding BY24.5H digested by Xbal-Sall double enzymes were linked to glutamine synthetase high-efficiency expression vectors connected to nucleotides encoding BY24.5L with a double expression cassette; or vice versa. The recombinant vectors were sequenced and verified to be correct then used for expression. The expressed anti-VEGFR antibody-CD80 fusion protein was named fusion protein BY24.5.

### (5)Synthesis of nucleotides encoding fusion protein containing CD80 extracellular domain, immunoglobulin Fc region and VEGFR extracellular receptor functional domain and construction of expression vectors

According to the sequence of the CD80 extracellular domain in Table 1, the IgG4Fc sequence in Table 6, and the VEGFR functional region sequence in Table 5 (VEGFR1-D2/VEGFR2-D3) were optimized to be suitable for expression in Chinese hamster ovarian cancer cells (CHO). And Shanghai Jierui Bioengineering Co., Ltd. was entrusted to synthesize the following polynucleotide sequence shown in SEQ ID NO: 87. The CD80-Fc-VEGFR fusion protein produced after the expression of the nucleotide sequence is also referred to herein as the fusion protein BY24.12.

Nucleotide sequence of fusion protein BY24.12 (CD80-Fc-VEGFR) (SEQ ID NO: 87)

CTCGAGGCCACCGCCACCATGGAGACCGACACACTCCTCCTGTGGGTGCTGCTGCT
GTGGGTGCCTGGCTCCACTGGCGTCATCCATGTGACCAAAGAGGTCAAAGAAGTCGCA
ACTCTGTCTTGCGGACACAACGTCTCCGTCGAAGAACTCGCCCAGACTAGAATATATTG
GCAGAAGGAAAAGAAGATGGTGCTCACCATGATGTCCGGCGATATGAACATTTGGCCT
GAGTACAAGAACCGGACAATCTTCGATATTACTAATAACCTGAGTATCGTGATTCTCGC
TCTGCGCCCTAGCGACGAGGGCACATACGAGTGTGTGGTGCTGAAGTACGAGAAGGAT
GCATTCAAGCGGGAGCACCTCGCAGAGGTGACACTCTCCGTGAAGGCCGACTTCCCAA

CCCCATCTGACAAGCGCGTGGAGTCCAAGTACGGCCCTCCTTGCCCTCCTTGCCCTGCC
CCTGAGTTCCTGGGCGGCCCTTCCGTGTTCCTGTTCCCTCCTAAGCCTAAGGACACCCT
GATGATCTCCCGCACCCCTGAGGTGACCTGCGTGGTGGACGTGTCCCAGGAGGAC
CCTGAGGTGCAGTTCAACTGGTACGTGGACGGCGTGGAGGTGCACAACGCCAAGACCA
AGCCTCGCGAGGAGCAGTTCAACTCCACCTACCGCGTGGTGTCCGTGCTGACCGTGCT
GCACCAGGACTGGCTGAACGGCAAGGAGTACAAGTGCAAGGTGTCCAACAAGGGCCT
GCCTTCCTCCATCGAGAAGACCATCTCCAAGGCCAAGGGCCAGCCTCGCGAGCCTCAG
GTGTACACCCTGCCTCCTTCCCAGGAGGAGATGACCAAGAACCAGGTGTCCCTGACCT
GCCTGGTGAAGGGCTTCTACCCTTCCGACATCGCCGTGGAGTGGGAGTCCAACGGCCA
GCCTGAGAACAACTACAAGACCACCCCTCCTGTGCTGGACTCCGACGGCTCCTTCTTCC
TGTACTCCCGCCTGACCGTGGACAAGTCCCGCTGGCAGGAGGGCAACGTGTTCTCCTG
CTCCGTGATGCACGAGGCCCTGCACAACCACTACACCCAGAAGTCCCTGTCCCTGTCCC
TGGGCGGCGGAGGATCTGGCGGCGGAGGCAGTGGAGGCGGCGGAAGTGCTTCCGACA
CCGGCCGCCCTTTCGTGGAGATGTACTCCGAGATCCCTGAGATCATCCACATGACCGA
GGGCCGCGAGCTGGTGATCCCTTGCCGCGTGACCTCCCCTAACATCACCGTGACCCTGA
AGAAGTTCCCTCTGGACACCCTGATCCCTGACGGCAAGCGCATCATCTGGGACTCCCG
CAAGGGCTTCATCATCTCCAACGCCACCTACAAGGAGATCGGCCTGCTGACCTGCGAG
GCCACCGTGAACGGCCACCTGTACAAGACCAACTACCTGACCCACCGCCAGACCAACA
CCATCATCGACGTGGTGCTGTCCCCTTCCCACGGCATCGAGCTGTCCGTGGGCGAGAA
GCTGGTGCTGAACTGCACCGCCCGCACCGAGCTGAACGTGGGCATCGACTTCAACTGG
GAGTACCCTTCCTCCAAGCACCAGCACAAGAAGCTGGTGAACCGCGACCTGAAGACCC
AGTCCGGCTCCGAGATGAAGAAGTTCCTGTCCACCCTGACCATCGACGGCGTGACCCG
CTCCGACCAGGGCCTGTACACCTGCGCCGCCTCCTCCGGCCTGATGACCAAGAAGAAC
TCCACCTTCGTGCGCGTGCACGAGTAAGAATTC

The amino acid sequence of the fusion protein BY24.12 (CD80-Fc-VEGFR) (SEQ ID NO: 88)

METDTLLLWVLLLWVPGSTGVIHVTKEVKEVATLSCGHNVSVEELAQTRIYWQKEK
KMVLTMMSGDMNIWPEYKNRTIFDITNNLSIVILALRPSDEGTYECVVLKYEKDAFKREHL
AEVTLSVKADFPTPSDKRVESKYGPPCPPCPAPEFLGGPSVFLFPPKPKDTLMISRTPEVTCV
VVDVSQEDPEVQFNWYVDGVEVHNAKTKPREEQFNSTYRVVSVLTVLHQDWLNGKEYK
CKVSNKGLPSSIEKTISKAKGQPREPQVYTLPPSQEEMTKNQVSLTCLVKGFYPSDIAVEWE

SNGQPENNYKTTPPVLDSDGSFFLYSRLTVDKSRWQEGNVFSCSVMHEALHNHYTQKSLS
LSLGGGGSGGGGSGGGGSASDTGRPFVEMYSEIPEIIHMTEGRELVIPCRVTSPNITVTLKKF
PLDTLIPDGKRIIWDSRKGFIISNATYKEIGLLTCEATVNGHLYKTNYLTHRQTNTIIDVVLSP
SHGIELSVGEKLVLNCTARTELNVGIDFNWEYPSSKHQHKKLVNRDLKTQSGSEMKKFLST
LTIDGVTRSDQGLYTCAASSGLMTKKNSTFVRVHE

Using the same method as in Example 1(1) above, the nucleotides encoding BY24.12 digested by XhoI-EcoRI double enzymes were linked to glutamine synthetase high-efficiency expression vectors with double expression cassettes (Patent No.: CN104195173B, obtained from Beijing Biyang Biotechnology Co., Ltd.). The recombinant vectors were sequenced and verified to be correct then used for expression. The expressed fusion protein was named fusion protein BY24.12.

### Example 2. Expression and purification of target protein

### (1) Transient expression of target protein

293 F (available from Invitrogen Inc., catalog No.: 11625-019) was suspended in serum-free CD293 broth (available from Invitrogen Inc. catalog No.: 11913-019). The cell culture was centrifuged prior to transfection, cell precipitation was obtained, cells were suspended with fresh serum-free CD293 broth, and the cell concentration was adjusted to 1 × 10⁶ cells/mL. The cell suspension was placed in a flask. Taking 100ml cell suspension as an example, 250ug of each recombinant expression vector plasmid DNA containing the target gene prepared in Example 1 and 500ug of polyethylenimine (PEI) (Sigma, catalog number: 408727) were added separately and mixed uniformly in 1ml serum-free CD 293 culture medium, after standing at room temperature for 8 minutes, the PEI/DNA suspension was added dropwise to a shake flask containing 100 mL of the cell suspension. Gently mixed uniformly, and placed in a 5% CO₂, 37°C shaker (120 revolutions per minute). The culture supernatant was collected after 5 days.

### (2) Purification of expressed protein

The protein of interest present in the culture supernatant collected by Example 2 (1) was purified by HiTrap MabSelect SuRe 1ml column (GE Healthcare Life Sciences Product, Catalog No. 11-0034-93) equilibrated with a pH 7.4 PBS solution. Briefly, a PBS solution of pH 7.4 was used to equilibrate HiTrap MabSelect SuRe 1ml column at 10 column bed volumes, and the flow rate was 0.5 mL/min. After the culture supernatant collected in Example 2 (1) was filtered with a 0.45 µm filter membrane, the sample was loaded to the HiTrap MabSelect SuRe 1ml column equilibrated a PBS solution of pH 7.4;

After supernatant was loaded, the column was first washed with PBS solution of pH 7.4 at a flow rate of 0.5 mL/min for 5-10 column bed volumes, and then eluted with 100 mM citric acid buffer (pH 4.0) at a flow rate of 0.5 mL/min. The elution peak was collected, and the proteins of interest BY18.1, BY31.19, BY24.4, BY24.5, BY24.12, respectively, were present in the elution peaks.

The purity and molecular weight of the proteins of interest BY18.1, BY31.19, BY24.4, BY24.5, BY24.5, BY24.12 respectively, were analyzed by SDS-PAGE electrophoresis and stained with Coomassie blue in the presence of a reducing agent (5 mM 1,4-dithiothreitol). The results are shown in Table 8 below, where theoretical predicted values and the actual measured values of the molecular weight are given. Since there is a glycosylation effect on the protein in the eukaryotic expression system, the actual measured value of the molecular weight is slightly higher than the theoretical predicted value.

**Table 8 The molecular weight of the purified expressed protein**

| Name of protein | Subunit | The size of theoretical predicted molecular weight(kDa) | The size of actual molecular weight(kDa) |
|---|---|---|---|
| Fusion protein BY24.4 | First subunit | 23 | 27 |
| | Second subunit | 62 | 70 |
| Fusion protein BY24.5 | First subunit | 23 | 27 |
| | Second subunit | 74 | 70 |
| Fusion protein BY24.12 | Any subunit | 63 | 72 |
| Fusion protein BY31.19 | Any subunit | 40 | 45 |
| Antibody BY18.1 | Light chain | 23 | 27 |
| | Heavy chain | 48 | 50 |

### Example 3. Detection of specific binding effects using ELISA methods

Recombinant human CD28 (the product of Beijing Yiqiao Shenzhou Biotechnology Co., Ltd., catalog No.: 50103-M08H), recombinant human PD-L1 (Beijing Biosciences Biotechnology Co., Ltd., catalog No. PD1-H5229) and recombinant human CTLA-4 (the product of Beijing Yiqiao Shenzhou Biotechnology Co., Ltd., catalog No.: 11159-H08H) was diluted to 0.5µg/ml, 0.25µg/ml and 1.0µg/ml and coated with 96-well ELISA plate (Corning company, catalog No.: 42592). The fusion proteins BY24.4, BY24.5, BY24.12, and BY31.19 purified in the above-mentioned Example 2(2) were diluted to 2000µg/ml, and then a 3-fold serial dilution was carried out and was diluted to 16 gradients totally, each concentration gradient is tested by multiple holes. 50 µl of the diluted sample was added to the 96-well plate coated with recombinant human CD28, recombinant human CTLA-4 or recombinant human PD-L1, and incubated at 37° C for 2 hours. After washing 3 times, goat anti-human secondary antibody labeled with horseradish peroxidase (product of Beijing Zhongshan Jinqiao Company, product No.: ZDR-5301) was added, and incubated at 37°C for 1 hour. After washing 3 times, 3,3',5,5'-tetramethylbenzidine (TMB) substrate color developing solution (Beijing Kangwei Century Biotechnology Co., Ltd., product No.: CW0050) was added 50 µl/well. 10 minutes later, 2N H₂SO₄ was added to stop the color development. The absorbance OD value of each well at 450nm was measured using an ELISA reader.

The protein concentration of fusion proteins BY24.4, BY24.5, BY24.12, and BY31.19 against the absorbance OD value was plotted using GraphPadrism5 software, and the data was fitted to produce a half maximum effective concentration EC50 value of the specific binding effect mediated by fusion protein. The results are shown in Table 9 below.

**Table 9 The binding effect of each fusion protein on human PD-L1, human CD28 and human CTLA-4**

| Name of protein | Binding of hPD-L1 EC50 (µg/ml) | Binding of hCD28 EC50 (µg/ml) | Binding of hCTLA-4 EC50 (µg/ml) |
|---|---|---|---|
| Fusion protein BY24.4 | 6.352 | 23.05 | 2.270 |
| Fusion protein BY24.5 | 5.167 | 35.07 | 3.751 |
| Fusion protein BY24.12 | 3.748 | 13.16 | 2.890 |
| Fusion protein BY31.19 | 2.20 | 24.33 | 1.924 |

The ELISA results showed that the multi-targeting fusion protein BY24.4, BY24.5, BY24.12, and BY 31.19 as a positive control can bind to recombinant human PD-L1, recombinant human CD28 and CTLA -4. Each fusion protein had the strongest binding ability to CTLA-4, followed by PD-L1, and the weakest binding to CD28.

Example 4 the affinity of the protein of interest to the target in the present invention was determined by Biacore T100.

The surface plasmon resonance measurement was performed on T 100 Instruments (GE Healthcare Biosciences, AB, Sweden) at 25° C.

First, the anti-IgG antibody (GE Healthcare Life Sciences, catalog No.: BR-1008-39) was covalently immobilized on the CM5 chip by amide coupling. The CM5 chip was activated using 60 µl of N-ethyl-N '-(3-dimethylaminopropyl) carbodiimide hydrochloride (EDC) and 60 µl of N-hydroxysuccinimide (NHS), and 95µl dilution buffer HBST (0.1M HEPES, 1.5M NaCl, pH7.4, and 0.005% Tween 20) was added to 5µl anti- IgG antibody and filtered through 0.2um filter membrane, then anti-IgG antibody was covalently immobilized on the CM5 chip by amide coupling, yielding a capture system of about 9000 to 14,000 resonant units (RU). 120 µl ethanolamine was used to block the CM5 chip.

Then, each protein of interest in the present invention prepared in Example 2 was diluted to 5 µg/ml, and the diluent was injected at a flow rate of 10 µL/min for 2 minutes, each protein of interest in the present invention prepared in Example 2 was covalently captured on the surface of the CM5 chip by their respective Fc domains. The resulting composite was stabilized by cross-linking with EDC/NHS to avoid baseline drift during measurement and regeneration.

The binding antigen PD-1 (the product of Beijing Yiqiao Shenzhou Biotechnology Co., Ltd., catalog No.: 10377-H08H), VEGF165 (the product of Beijing Yiqiao Shenzhou Biotechnology Co., Ltd., catalog No.: 11066-HNAH), and mouse VEGF164 (the product of Beijing Yiqiao Shenzhou Biotechnology Co., Ltd., catalog number: 50159-MNAB) and VEGFR2 (the product of Beijing Yiqiao Shenzhou Biotechnology Co., Ltd., catalog No.: 10012-H02H1) are formulated into the following concentration gradients: 7nM, 22nm, 66nM, 200nM, 600nM. The binding was measured by injecting each concentration at a flow rate of 30 µl/min for 180 seconds and a dissociation time of 600 seconds. The surface was regenerated by washing with a 3M MgCl2 solution at a flow rate of 10 µL/min for 30 seconds. Data analysis BIA was performed by evaluation software (BIA evaluation 4.1 software, from GE Healthcare Biosciences AB, Sweden), and the affinity data shown in Table 10 below were obtained.

**Table 10 The binding of each protein of interest to the target**

| Name of protein of interest | Target | ka(l/Ms) | kd(l/s) | KD(M) |
|---|---|---|---|---|
| Fusion protein BY24.4 | VEGF-A | 2.221E+6 | 3 .407E-5 | 1.534E-11 |
| Fusion protein BY24.5 | VEGFR2 | 6.82 E+5 | 5.57 E-5 | 8.17 E-11 |
| Fusion protein BY24.12 | human VEG F-A | 3.15 E+6 | 1.25 E-4 | 3.98 E-11 |
| Fusion protein BY24.12 | mouse VEGF-A | 9.18 E+5 | 3.13 E-5 | 3.41E-11 |
| Antibody BY18.1 | PD-1 | 6.66 E+5 | 1.58 E-3 | 2.37E-9 |

The KD(M) of the fusion proteins BY24.4, BY24.5, BY24.12 and their respective targets are all lower than 10⁻⁹M, indicating that they have high affinity with their respective targets, especially the fusion protein BY24.12 can bind to both mice and human VEGF-A with high affinity and the difference is not very big.

Example 5. The effect of the protein of interest in the present invention and its composition on the secretion of IFN-γ in the mixed lymphocyte reaction (MLR)

CD4+ T lymphocytes and dendritic cells (DC) were available from Beijing Shihe Biotechnology Co., Ltd., and the CD4+ T lymphocytes and dendritic cells (DC) were derived from different healthy people. CD4+ T lymphocytes and dendritic cells (DC) were seeded in 96-well cell culture plates at 1 × 10⁵ cells/well and 1 × 10⁴ cells/well, respectively. The experiments were divided into 7 groups, namely BY18.1 group (1.0µg/ml), BY24.4 group (1.14µg/ml), BY24.4 (1.14µg/ml) + BY18.1 (1.0µg/ml) group, BY24.5 (1.14µg/ml) group, BY24.5 (1.14µg/ml) + BY18.1 (1.0µg/ml) group, BY24.12 (0.84µg/ml) group and BY24.12 (0.84µg/ml)+BY18.1 (1.0ug/ml) group. 3 replicate wells in each group, antibody BY18.1, fusion protein BY24.4, BY24.5, BY24.12, or antibody BY18.1 and each combination of BY24.4, BY24.5, BY24.12 were added as required to the final concentration shown. Finally, 1640 medium containing 10% fetal bovine serum was added and the final volume was 200µl. Cultured at 37 C, 5% CO₂.

After 5 days of culture, there were many clump cells in all the seven experimental groups, and there was a significant portion of the presence of shuttle and adherent cells. The IFN-γ expression level of each group was detected by an IFN-γ kit (Cargo No.: EH 008-96 ELISA).

The detection results were as follows: Compared with the antibody BY18.1 group (2221.8±364.5pg/ml), the secretion of IFN-γ in the fusion protein BY24.4 group alone (924.1±221.9pg/ml), BY24.5 group (760.1±286.8pg/ml) and BY24.12 group (793.4±139.2pg/ml) was all significantly lower than that of the antibody BY18.1 group (P<0.01); but after the fusion proteins BY24.4, BY24.5 and BY24.12 were added to BY18.1 respectively, the secretion of IFN-γ all increased significantly. The secretion of IFN-γ in each group was respectively as follows: BY24.4+BY18.1 group (3494.2±364.5pg/ml), BY24.5+BY18.1 group (3523.8 ±465.1pg/ml) and BY24.12+BY18.1 group (3801.8±702.2pg/ml). It can be seen that the combination of fusion proteins BY24.4, BY24.5 and BY24.12 respectively with BY18.1 can lead to a significant increase in the secretion of IFN-γ (P<0.01), and it indicated that the antibody BY18.1 (PD-1 antibody) in combination with the multi-targeting fusion protein BY24.4, BY24.5 and BY24.12 of the present invention has a significant synergistic effect on the secretion of IFN-γ.

Example 6. The synergistic effect of the multi-targeting fusion protein of the present invention andPD-1 antibody

The main purpose of this example is to explore the synergistic effect of the multi-targeting fusion protein and the PD-1 antibody in anti-tumor in vivo in the present invention, therefore, the doses of the PD-1 antibody and each fusion protein are all low doses because high dose may have a better tumor suppressive effect and no synergy between the PD-1 antibody and each fusion protein can be observed.

Mouse-derived colon cancer cells CT26 (ATCC) in RPMI -1640 medium were seeded in rib subcutaneous at right front of about 18 g, 6 weeks old female BALB/c mouse, 100 µl/mouse, the amount of inoculation is 1 × 10⁶ cells/mouse. The time of CT26 inoculation of mouse-derived colon cancer cells was set to day 0. When the average tumor volume reached about 93 mm³, the tumor-bearing mice were randomly grouped, 6 mice per group, and 5 groups in total. The molar amounts were calculated according to the size of the molecular weight, the fusion protein BY 31.19, BY 24.12, and the anti-MPD-1 antibody were administered in equivalent molar amounts. Specifically, the grouping and dosage were as follows: vehicle (PBS) control group; fusion protein BY31.19 (1.6 mg/kg) group; fusion protein BY24.12 (2.5 mg/kg) group; anti-mPD-1 (3.0 mg/kg, available from BioXcell, clone No.: RMP1-14, product No.: BE0146) group; fusion protein BY24.12 (2.5 mg/kg) + anti-mPD-1 (3 mg/kg) group. Dosing twice a week. The tumor volume was measured three times a week. While the tumor volume was being measured, the mice were weighed, and the relationship between the changes in the mouse body weight and tumor volume and the administration time was recorded. At the end of the experiment, the mice were euthanized, serum and tumors were collected, and the serum was cryopreserved at -80°C. After the tumors were weighed and photographed, they were formalin-fixed and paraffin-embedded (FFPE) to prepare tissue samples for later use. The data was analyzed statistically and the relative tumor volume ratio (T/C) % (That is: (the mean value of tumor volume change in the drug-administered group/the mean value of tumor volume change in the PBS vehicle control group) x 100%) and tumor growth inhibition rate (Tumor Growth Inhibition%) of each treatment group and vehicle control group were calculated, which was (1-T/C) %.

At the end of the experiment, the tumor growth inhibition rates (TGI%) of BY31.19 group, BY24.12 group, PD-1 group, BY24.12+anti-mPD-1 group were 15%, 17%, 24%, and 47% (calculated by tumor volume), all have different degrees of inhibition. The inhibitory effect of the immune fusion protein BY24.12 and PD-1 antibody in combination was significantly better than that of BY24.12 and PD-1 alone, so there is a synergistic effect between the two.

The animals in each group continued to increase their body weight after the administration, and the overall condition was good, no obvious abnormality, and there was no significant drug withdrawal or death in each group.

## Claims

1. A multi-targeting fusion protein that blocks the growth of vascular endothelial cells and activates T cells, which comprises (i) a vascular endothelial growth inhibitor domain; (ii) an immunoglobulin Fc domain; and (iii) CD80 extracellular domain (ECD).

2. The multi-targeting fusion protein according to claim 1, wherein the (i) comprises an antigen-binding fragment derived from an anti-VEGF antibody and/or an anti-VEGFR antibody and/or a VEGFR extracellular receptor functional domain;
preferably, the antigen-binding fragment of the anti-VEGF antibody and/or the anti-VEGFR antibody is the Fab, Fab', F(ab')₂, Fv, single-chain Fv of the anti-VEGF antibody and/or the anti-VEGFR antibody; more preferably, the antigen-binding fragment of the anti-VEGF antibody and/or the anti-VEGFR antibody comprises all six heavy chain CDRs and light chain CDRs that are contained in paired heavy chain variable region sequence/light chain variable region sequence selected from the group consisting of SEQ ID NO: 1/2, 3/4, and 5/6, or a sequence having one, two, three, four, or five amino acid changes (e.g., amino acid replacement or deletion) from one or more CDR(s) in all the six heavy chain CDRs and light chain CDRs; further preferably, the antigen-binding fragment of the anti-VEGF antibody and/or the anti-VEGFR antibody comprises the paired heavy chain variable region sequence/light chain variable region sequence selected from the group consisting of SEQ ID NO: 1/2, 3/4 and 5/6, or a sequence having at least 90%,91%, 92%, 93%, 94%, 95%, 96%, 97%, 98%, 99% or moresequence identity to the paired heavy chain variable region sequence/light chain variable region sequence; most preferably, the antigen-binding fragment of the anti-VEGF antibody and/or the anti-VEGFR antibody is the Fab of Bevacizumab, Ranibizumab or Ramucirumab;
preferably, the VEGFR extracellular receptor functional domain comprises the immunoglobulin-like domain 2 of VEGFR1 and the immunoglobulin-like domain 3 of VEGFR2; or theVEGFR extracellular receptor functional domain comprises the immunoglobulin-like domain 2 of VEGFR1 and the immunoglobulin-like domain 3 of VEGFR2 and the immunoglobulin-like domain 4 of VEGFR2; or theVEGFR extracellular receptor functional domain comprises the immunoglobulin-like domain 2 of VEGFR1; more preferably,the VEGFR extracellular receptor functional domain has any amino acid sequence selected fromSEQ ID NO: 7-9 or an amino acid sequence having at least 90%, 91%, 92%, 93%, 94%, 95%, 96%, 97%, 98%, 99% or more sequence identity to the amino acid sequence shown in SEQ ID NO: 7-9.

3. The multi-targeting fusion protein according to claim 1 or 2, wherein the (ii) is a human immunoglobulin Fc domain; preferably, the (ii) is a human IgG1, IgG2, IgG3 or IgG4 Fc domain; more preferably, the (ii) comprises the Fc domain of the amino acid sequence shown in SEQ ID NO: 10, 11 or 12, or comprises the Fc domain having at least 90%,91%, 92%, 93%, 94%, 95%, 96%, 97%, 98%, 99% or moresequence identity to the amino acid sequence shown in SEQ ID NO: 10, 11 or 12.

4. The multi-targeting fusion protein according to any one of claims 1 to 3, wherein the (iii) comprises human CD80 ECD; preferably, the (iii) comprises human CD80 IgV or human CD80 IgVIgC; preferably, the (iii) has the amino acid sequence shown in SEQ ID NO: 13 or 14 or an amino acid sequence having at least 90%, 91%, 92%, 93%, 94%, 95%, 96%, 97%, 98%, 99% or more sequence identity to the amino acid sequence shown in SEQ ID NO: 13 or 14.

5. The multi-targeting fusion protein according to any one of claims 1 to 4, which further comprises a peptide linker between (i), (ii) and/or (iii); preferably, the peptide linker comprises one or more amino acids, more preferably at least 5 amino acids, and most preferably a peptide linker selected from SEQ ID NO: 20-46.

6. The multi-targeting fusion protein according to any one of claims 1 to 5, wherein the fusion protein is effectively connected in the order of (i), (ii) and (iii); the order of (iii), (i) and (ii); or the order of (iii), (ii) and (i) from N-terminal to C-terminal.

7. The multi-targeting fusion protein according to claim 6, which comprises
(a) A full-length anti-VEGF antibody, a full-length anti-VEGFR antibody, or a full-length anti-VEGF and VEGFR bispecific antibody; and one CD80 ECD effectively connected to the C-terminal of each of the two heavy chains of the antibody;
(b) A full-length anti-VEGF antibody, a full-length anti-VEGFR antibody, or a full-length anti-VEGF and VEGFR bispecific antibody; and one CD80 ECD effectively connected to the N-terminal of each of the two heavy chains of the antibody; and one CD80 ECD effectively connected to the N-terminal of each of the two light chains of the antibody;
(c) A CD80 ECD; an immunoglobulin Fc domain in dimer form effectively connected at the C-terminal of a CD80 ECD; and an antigen-binding fragment derived from an anti-VEGF antibody and/or an anti-VEGFR antibody effectively connected at the C-terminal of an immunoglobulin Fc domain in dimer form;
or
(d) A CD80 ECD;an immunoglobulin Fc domain in dimer form effectively connected at the C-terminal of a CD80 ECD; and a VEGFR extracellular receptor functional domain effectively connected to the C-terminal of the immunoglobulin Fc domain in dimer form;
Preferably, the antibody is an IgG class antibody, particularly an IgG₁ subclass, an IgG₂ subclass, an IgG₄ subclass antibody; and more particularly an IgG₄ subclass antibody; also preferably, the IgG4 subclass antibody contains amino acid replacement at the S228 position of the Fc domain, more preferably amino acid replacement S228P; further preferably, the light chain type of the antibody is κ type or λ type, preferably κ type;
Preferably, the full-length anti-VEGF antibody is Bevacizumab, and the full-length anti-VEGFR antibody is Ramucirumab.

8. The multi-targeting fusion protein according to any one of claims 1-7, which is selected from
(1) A fusion protein comprising the first subunit of the fusion protein of SEQ ID NO: 80 and the second subunit of the fusion protein of SEQ ID NO: 82;
(2) A fusion protein comprising the first subunit of the fusion protein of SEQ ID NO: 84 and the second subunit of the fusion protein of SEQ ID NO: 86;
(3) A fusion protein comprising the fusion protein subunit of SEQ ID NO:88.

9. A polynucleotide encoding the multi-targeting fusion protein of any one of claims 1-8.

10. A vector, preferably an expression vector, most preferably a glutamine synthetase expression vector with double expression cassettes, the vector comprises the polynucleotide of claim 9.

11. A host cell, which comprises the polynucleotide of claim 9 or the vector of claim 10, preferably the host cell is a CHO, HEK293 or NSO cell.

12. A method for producing the multi-targeting fusion protein of any one of claims 1 to 8, which comprises step (i) culturing the host cell in claim 11 under conditions suitable for expressing the multi-targeting fusion protein , and step (ii) recovering the fusion protein.

13. A pharmaceutical composition, which comprises the multi-targeting fusion protein according to any one of claims 1 to 8 and an anti-PD-1 antibody, preferably, the anti-PD-1 antibody comprises all six heavy chain CDRs and light chain CDRs contained in the paired heavy chain variable region sequence/light chain variable region sequence selected from the group consisting of SEQ ID NO: 47 /48, 49/50, 51/52, 53/54, 55/56, 57/58, 59/60, 61/62, 63/64, 65/66, 67/68, 69/70 and 71/72, or a sequence having one, two, three, four, or five amino acid changes (e.g., amino acid replacement or deletion) from one or more CDR(s) in all six heavy chain CDRs and light chain CDRs; further preferably, the anti-PD-1 antibody comprises paired heavy chain variable region sequence/light chain variable region sequence selected from SEQ ID NO: 47/ 48, 49/50, 51/52, 53/54, 55/56, 57/58, 59/60, 61/62, 63/64, 65/66, 67/68, 69/70 and 71/72 , or a sequence having at least 90%,91%, 92%, 93%, 94%, 95%, 96%, 97%, 98%, 99% or moresequence identity to the paired heavy chain variable region sequence/light chain variable region sequence; most preferably, the anti-PD-1 antibody is selected from Nivolumab, Pidilizumab and Pembrolizumab.

14. Use of the multi-targeting fusion protein according to any one of claims 1 to 8, or use of the pharmaceutical composition according to claim 13, for manufacturing a medicament for the treatment or prevention of a cancerous disease (e.g., a solid tumor or a soft tissue tumor) in an individual, preferably the cancerous disease is melanoma, breast cancer, colon cancer, esophageal cancer, gastrointestinal stromal tumor (GIST), kidney cancer (e.g., renal cell carcinoma), liver cancer, non-small cell lung cancer (NSCLC), ovarian cancer, pancreatic cancer, prostate cancer, head and neck tumors, gastric cancer, or a hematological malignancy (e.g., lymphoma); in particular, the disease is colon cancer or triple negative breast cancer; preferably, wherein the individual is a mammal, more preferably a human.
